# FASCICULE DE BREVET EUROPEEN

(11) **EP 0 876 356 B1**
(45) Date de publication et mention de la délivrance du brevet: **31.10.2001**
(21) Numéro de dépôt: 96941734.4
(22) Date de dépôt: 10.12.1996
(51) Int. Cl.: C07D 235/02, C07D 263/60, C07D 413/04, C07D 277/84, C07D 417/04, A61K 31/41, C07C 50/00, C07C 225/28

(54) **UTILISATION DE DERIVES TRICYCLIQUES DU 1,4-DIHYDRO-1,4-DIOXO-1H-NAPHTALENE, NOUVEAUX COMPOSES OBTENUS ET LEUR APPLICATION EN THERAPEUTIQUE**
VERWENDUNG VON TRICYCLISCHEN 1,4-DIHYDRO-1,4-DIOXO-1H-NAPHALEN DERIVATEN, NEUE VERBINDUNGEN UND IHRE THERAPEUTISCHE VERWENDUNG
USE OF TRICYCLIC 1,4-DIHYDRO-1,4-DIOXO-1H-NAPHTHALENE DERIVATIVES, RESULTING NOVEL COMPOUNDS AND THERAPEUTICAL USE THEREOF

(30) Priorité: 12.12.1995 FR 9514683
(43) Date de publication de la demande: 11.11.1998
(73) Titulaire: LABORATOIRE INNOTHERA Société Anonyme, 94111 Arcueil (FR)
(72) Inventeur: BOUTHERIN-FALSON, Odile, F-91120 Palaiseau (FR); DESQUAND-BILLIALD, Stéphanie, F-75015 Paris (FR); FAVROU, Anita, F-94230 Cachan (FR); FINET, Michel, F-92290 Châtenay-Malabry (FR); TEMBO, Olivier, F-95540 Méry-sur-Oise (FR); TORREGROSA, Jean-Luc, F-94230 Cachan (FR); YANNIC-ARNOULT Sylvie, F-91360 Epinay-sur-Orge (FR); DOMAGALA-LE MARQUER, Florence, F-92160 Antony (FR)
(74) Mandataire: Dupuis-Latour, Dominique
(86) Numéro de dépôt international: FR9601973
(87) Numéro de publication internationale: WO9721684

(56) Documents cités:
- WO-A-92/19211

## Description

La présente invention concerne l'utilisation de dérivés tricycliques et de leurs sels acceptables du point de vue pharmaceutique pour l'obtention d'un médicament destiné au traitement de maladies liées à une altération de la fonction veineuse et/ou à l'oedème inflammatoire et les nouveaux composés obtenus. Elle se rapporte plus particulièrement aux dérivés tricycliques du 1,4-dihydro-1,4-dioxo-1H-naphtalène. L'invention concerne l'application thérapeutique de tous ces composés.

Il est décrit dans J. Heterocycl. Chem., 6(6), 909-916, 1969, par Carroll F.I., Blackwell J.T., la synthèse de 1H-napht[2,3-d]imidazole-4,9-diones substituées en position 2. De plus le document Zh. Org. Khim. , 3(1), 162-168, 1967, par Efimova G.A., Efros L.S., concerne l'obtention de la 1,2-diméthyl-1H-napht[2,3-d]imidazole-4,9-dione. Enfin le document J. Am. Chem. Soc. ,76, 4148-4152, 1954, par Hoover J. R. E., Day A. R., montre la préparation de dérivés de la 1H-naphtimidazole-4,9-dione à partir du 2,3-dichloro-1,4-dihydro-1,4-dioxo-naphtalène.

L'article J. Prakt. Chem. 319(2), 254-258, 1977, par Hammam Ahmed S., Osman Abdel-Magid, décrit la synthèse de 2-amido-3-chloro-1,4-dihydro-1,4-dioxo-naphtalènes à partir du 2,3-dichloro-1,4-dihydro-1,4-dioxo-naphtalène, composé qui peut servir d'intermédiaire pour la synthèse ultérieure de napht[2,3-d] oxazole-4,9-diones substituées ou non en position 2. Le brevet U.S. 3,039,925, du 19 juin 1962 et d'une demande de brevet DE du 24 avril 1967 par Gerhard Domagk, Karl W. Schellhammer, Siegfried Petersen, Hans B. Koenig concernent la synthèse de la 2-méthyl-napht[2,3-d]oxazole-4,9-dione réalisée par Fries K. et Ochwat P. (Berichte 56, 1926 [1923]).

Le brevet japonais JP 61,251,675 de S. Hiroyuki, ainsi que l'article Collect. Czech. Chem. Commun., 50(1), 71-79, 1985. par Hammam A.S., Bayoumy B.E. et le document J. Heterocyclic Chem. 25, 901-906, 1988, par Katritzky A.R., Fan W.Q., décrivent la préparation des naphto[2,3-d] thiazole-4,9-diones.

Les dérivés tricycliques et leurs sels acceptables du point de vue pharmaceutique selon la présente invention répondent à la formule générale exposée dans la revendication 1.

L'invention concerne également les produits nouveaux énoncés par les revendications 2 à 70.

L'invention se rapporte aussi à l'utilisation des dérivés tricycliques et de leurs sels acceptables du point de vue pharmaceutique répondant à la formule générale de la revendication 1, pour l'obtention d'un médicament destiné :
- au traitement de l'insuffisance veineuse fonctionnelle et organique ;
- au traitement des pathologies hémorroidaires ;
- au traitement de la migraine ;
- au traitement des inflammations ostéoarticulaires dermatologiques et cardiovasculaires ;
- au traitement des états de chocs constitués par une chute importante de la pression artérielle plus particulièrement dans les états de chocs septiques.

La présente invention concerne également les sels des composés salifiables de la formule de la revendication 1. Ces sels comprennent les sels d'addition d'acides minéraux tels que l'acide chlorhydrique, bromhydrique, sulfurique, phosphorique ou nitrique, et les sels d'addition d'acides organiques tels que l'acide acétique, propionique, oxalique, citrique, maléique, fumarique, succinique, tartrique.

L'invention est illustrée par les exemples non limitatifs ci-après.

Les exemples indiqués par un chiffre correspondent à de nouveaux composés, tandis que les exemples comprenant une lettre correspondent à des composés connus.

Dans tous les exemples, les analyses sont réalisées comme indiqué ci-après :
- Points de fusion : Ils ont été réalisés sur un appareil de type «Banc de Kofler» LEICA - REICHERT modèle WME et ne sont pas corrigés.
- Chromatographies sur couche mince : Elles ont été obtenues sur des plaques de gel de silice avec indicateur de fluorescence UV₂₅₄ de 0,25 mm d'épaisseur de type MACHEREY-NAGEL (Référence 805 023). Les solvants d'élution sont indiqués pour chaque composé.
- Spectres de masse : Ils ont été effectués soit sur un spectromètre de type AEI MS-50 soit sur un spectromètre de type FISONS VG PLATFORM. Le mode d'ionisation est indiqué pour chaque exemple.
- Spectres RMN : Les spectres de RMN du ¹H et du ¹³C ont été réalisés soit sur un spectromètre de type JEOL respectivement à 270 MHz et à 68 MHz, soit sur un spectromètre de type BRUCKER respectivement à 400 MHz et à 100 MHz. Les solvants deutérés utilisés sont indiqués pour chaque analyse.
- Spectres Infra-Rouge : Ils ont été obtenus sur un spectromètre de type NICOLET 205 FT-IR. Ils sont effectués à 1 % (m/m) en dispersion dans le KBr.

### Exemple 1

### Sulfate de 4.9-Dihydro-4.9-dioxo-1,2-diméthyl-1H-napht[2,3-d]imidazole

A une solution de 2 g (8,84 mmoles) de 4,9-dihydro-4,9-dioxo-1,2-diméthyl-lH-napht[2,3-d] imidazole dans 300 ml d'un mélange (2/1) méthanol/dichlorométhane porté à 70°C, on ajoute 1 ml d'acide sulfurique concentré. Le mélange réactionnel est agité à 70°C pendant deux heures, concentré sous pression réduite et le précipité jaune pâle qui apparait est filtré, lavé au dichlorométhane puis à l'éther éthylique pour fournir 2 g de sulfate de 4,9-dihydro-4,9-dioxo-1,2-diméthyl-1H-napht [2,3-d]-imidazole, sous forme de cristaux jaunes.
**Rdt :** 70 %
**F : >** 260°C
**Rf :** 0,50 (CH₂Cl₂/Méthanol, 97,5/2,5)
**RMN du** ^{**1**}**H (DMSO d**_{**6**}**) : δ (ppm)**
8,05 (dd, 2H, H-5, H-8, J_{H5-H6} = J_{H7-H8} = 8,85 Hz; J_{H5-H7} = J_{H6-H8} = 1,73 Hz)
7,85 (m, 2H, H-6, H-7)
5,44 (s, 1H, NH+)
3,98 (S, 3H, CH₃)
2,53 (S, 3H, CH₃)
**RMN du** ^{**13**}**C (DMSO,d**_{**6**}**) :** δ **(ppm)**
177,22, 175,44 (2C, C-4, C-9)
153,46 (1C, C-2)
138,96 (1C, C-3a)
134,10, 134,01 (2C, C-6, C-7)
132,30, 131,93 (3C, C-8a, C-9a, C-4a)
126,24, 126,13 (2C, C-5, C-8)
32,33 (1C, CH₃)
12,30 (1C, CH₃)
**IR (KBr) : ν (cm**^{**-1**}**)**
3414 à 2400 (bande large NH+) ; 1674 (C=O)

### Exemple 2

### 4,9-Dihydro-4,9-dioxo-2-(2-fluorophényl)-1H-napht-[2,3-d]imidazole

A une suspension de 1,37 g (7,29 mmoles) de 2,3-diamino-1,4-dihydro-1,4 -dioxo-naphtalène dans 50 ml d'eau, on ajoute une solution de 0,77 ml (7,29 mmoles) de 2-fluorobenzaldéhyde dans 5 ml d'acide acétique glacial. Après 5 heures de reflux le solide noir obtenu est filtré, puis lavé trois fois avec 30 ml d'eau. Le solide est ensuite repris par 2 litres de dichlorométhane et la phase organique est lavée trois fois avec l'eau, séchée sur du chlorure de calcium puis évaporée à sec pour fournir 1,50 g de cristaux marrons. Le produit est purifié sur colonne flash (support : silice, conditionnement : heptane, éluants : dichlorométhane/heptane, 95/5 puis dichlorométhane/méthanol, 99/1), on obtient ainsi après évaporation des solvants sous pression réduite 1,00 g de 4,9-dihydro-4,9-dioxo-2-(2-fluorophényl)-1H-napht[2,3-d]-imidazole sous forme de cristaux jaunes.
**Rdt :** 47 %
**F : >** 260°C
**Rf :** 0,56 (CH₂Cl₂/Acétate d'éthyle, 92/8)
**SM (I.E.) :** m/z 292 (M+.)
**RMN du** ^{**1**}**H (DMSO d**_{**6**}**) : δ (ppm)**
14,28 (s, 1H, NH)
8,12 (m, 2H, H-5, H-8)
8,00 (m, 1H, H-6')
7,86 (m, 2H, H-6, H-7)
7,60 (m, 1H, H-3')
7,44 (m, 2H, H-4', H-5')
**RMN du** ^{**13**}**C (DMSO d**_{**6**}**) : δ (ppm)**
179,13, 175,03 (2C, C-4, C-9)
157,41 (1C, C-2')
147,88 (1C, C-2)
133,85 (2C, C-6, C-7)
132,67 (3C, C-6', C-9a, C-3a)
130,62 (2C, C-4a, C-8a)
126,24, 124,82 (3C, C-5, C-8, C-5')
116,62, 116,31 (2C, C-1', C-3')
**IR (KBr) : ν (cm**^{**-1**}**)**
3339 (NH) ; 1683, 1665 (C=O)

### Exemple 3

### 4,9-Dihydro-4,9-dioxo-2-(2-fluorophényl)-napht[2,3-d]oxazole

A une solution de 6,0 g (28,8 mmoles) de 2-amino-3-chloro-1,4-dihydro-1,4-dioxo-naphtalène dans 120 ml de nitrobenzène on ajoute à l'abri de la lumière 17,10 ml (144,0 mmoles) du chlorure de l'acide 2-fluorobenzoïque. Après 10 minutes d'agitation à 80°C, on ajoute 0,20 ml d'acide sulfurique concentré. Le mélange réactionnel est portéà reflux pendant 18 heures. Après complet refroidissement, on ajoute de l'éther pour obtenir un précipité jaune qui est filtré sur verre fritté et lavé à l'éther. Ce solide est ensuite purifié sur colonne flash (support : silice; conditionnement : heptane ; éluant : dichlorométhane/heptane, 60/40) pour fournir 2,5 g de 4,9-dihydro-4,9-dioxo-2-(2-fluorophényl)-napht[2,3-d]-oxazole sous forme de cristaux jaunes.
**Rdt :** 30 %
**F :** > 260°C
**Rf :** 0,60 (CH₂Cl₂)
**SM (I.E.) :** m/z 293 (M+.)
**RMN du** ^{**1**}**H (DMSO d**_{**6**}**) : δ (ppm)**
8,25 (m, 2H, H-5, H-8)
8,19 (m, 1H, H-6')
7,95 (m, 2H, H-6, H-7)
7,76 (m, 1H, H-4')
7,52 (m, 2H, H-3', H-5')
**RMN du** ^{**13**}**C (DMSO d**_{**6**}**) : δ (ppm)**
178,22, 173,06 (2C, C-4, C-9)
161,88 (1C, C-2)
158,08 (1C, C-2')
150,67, 142,83 (2C, C-3a, C-9a)
135,37 (1C, C-6')
134,64 (2C, C-6, C-7)
132,31, 131,94 (2C, C-4a, C-8a)
130,65 (1C, C-4')
126,79, 126,47 (2C, C-5, C-8)
125,62 (1C, C-5')
117,32 (1C, C-3')
113,21 (1C, C-1')
**IR (KBr) : ν (cm**^{**-1**}**)**
1693, 1680 (C=O)

### Exemple 4

### 4,9-Dihydro-4,9-dioxo-2-(3-fluorophényl)-napht[2,3-d]oxazole

A une solution de 5,00 g (24 mmoles) de 2-amino-3-chloro-1,4-dihydro-1,4-dioxo-naphtalène dans 50 ml de nitrobenzène, on ajoute à l'abri de la lumière 14,6 ml (120 mmoles) de chlorure d'acide 3-fluorobenzoïque puis après 5 minutes d'agitation 0,50 ml d'acide sulfurique concentré. Après 24 heures de reflux et complet refroidissement, on ajoute au mélange réactionnel 200 ml d'éther. Le précipité formé est filtré puis repris par 200 ml de dichlorométhane auquel on ajoute 100 ml d'une solution glaciale d'hydroxyde de sodium. Après 6 heures sous agitation à cette température, la phase organique est extraite, lavée plusieurs fois à l'eau et séchée sur du chlorure de calcium. Le solide obtenu après évaporation du solvant est purifié sur colonne moyenne pression (support : silice ; conditionnement : heptane ; éluant : dichlorométhane/heptane, 50/50). Les cristaux jaunes ainsi obtenus sont décolorés, recristallisés dans un mélange (v/v) dichlorométhane/heptane pour fournir 0,50 g de 4,9-dihydro-4,9-dioxo-2-(3-fluorophényl)-napht[2,3-d]oxazole sous forme de cristaux jaunes.
**Rdt :** 7 %
**F :** 230°C
**Rf :** 0,50 (CH₂Cl₂/Heptane, 95/5)
**SM (I.E.) :** m/z 293 (M+.)
**RMN du** ^{**1**}**H (CDCl**_{**3**}**) : δ (ppm)**
8,31 (m, 1H, H-6')
8,27 (m, 2H, H-5, H-8)
8,02 (s, 1H, H-2')
7,83 (m, 2H, H-6, H-7)
7,63 (m, 1H, H-5')
7,31 (m, 1H, H-4')
**RMN du** ^{**13**}**C (DMSO d**_{**6**}**) : δ (ppm)**
178, 173 (2C, C-4, C-9)
162,53 (1C, C-3')
149,37(1C, C-9a)
143,47 (1C, C-3a)
134,85, 134,46 (2C, C-6, C-7)
132,45, 132,08 (2C, C-4a, C-8a)
131,06 (1C, C-5')
127,52, 127,11 (2C, C-5, C-8)
124,74 (1C, C-6')
117,46, 117,15 (2C, C-2', C-4').
**IR (KBr) : ν (cm**^{**-1**}**)**
1695, 1680 (C=O)

### Exemple 5

### 4,9-Dihydro-4,9-dioxo-2-(4-fluorophényl)-napht[2,3-d]oxazole

A une solution de 6,22 g (30 mmoles) de 2-amino-3-chloro-1,4-dihydro-1,4-dioxo-naphtalène dans 60 ml de nitrobenzene on ajoute à l'abri de la lumière 18,00 ml (150 mmoles) du chlorure de l'acide 4-fluorobenzoïque. Après 10 minutes d'agitation à reflux, on ajoute 0,20 ml d'acide sulfurique concentré. Après 12 heures et complet refroidissement, on ajoute de l'éther pour obtenir un précipité jaune qui est filtré sur verre fritté, lavé à l'éther et purifié sur colonne flash (support : silice; conditionnement : heptane ; éluant : dichlorométhane/ heptane, 60/40). La poudre jaune obtenue après évaporation du solvant est décolorée, recristallisée dans du dichlorométhane pour fournir 2,90 g de 4,9-dihydro-4,9-dioxo-2-(4-fluorophényl)-napht[2,3-d]oxazole sous forme de cristaux jaunes.
**Rdt :** 33 %
**F :** > 260°C
**Rf :** 0,60 (CH₂Cl₂/Heptane, 80/20)
**SM (I.E.) :** m/z 293 (M+.)
**RMN du** ^{**1**}**H (CDCl**_{**3**}**) : δ (ppm)**
8,33 (m, 2H, H-2', H-6')
8,28 (m, 2H, H-5, H-8)
7,82 (m, 2H, H-6, H-7)
7.26 (m, 2H, H-3', H-5')
**RMN du** ^{**13**}**C (CDCl**_{**3**}**) : δ (ppm)**
166,89 (1C, C-4')
143,45 (1C, C-3a)
134,42 (2C, C-6, C-7)
132,86 132,55 (2C, C-4a, C-8a)
130,78 (2C, C-2', C-6')
127,52, 127,07 (2C, C-5, C-8)
121,50 (1C, C-1')
116,82, 116,50 (2C, C-3', C-5')
**IR (KBr) : ν (cm**^{**-1**}**)**
1689, 1669 (C=O)

### Exemple 6

### 4,9-Dihydro-4,9-dioxo-2-(2-méthylphénol)-napht[2,3-d]oxazole

A une solution de 5,00 g (24 mmoles) de 2-amino-3-chloro-1,4-dihydro-1,4-dioxo-naphtalène dans 37 ml (280 mmoles) de chlorure d'acide-2-méthylbenzoïque, on ajoute à l'abri de la lumière 8 gouttes d'acide sulfurique concentré. Après 7 heures de reflux et complet refroidissement, le précipité ocre qui se forme est filtré sur verre fritté, lavé à l'éther et purifié sur colonne flash (support : silice ; conditionnement : heptane ; éluant : dichlorométhane/heptane, 50/50). La poudre jaune obtenue après évaporation du solvant est décolorée, recristallisée dans du dichlorométhane pour fournir 2,.34 g de 4,9-dihydro-4,9-dioxo-2-(2-méthylphényl)-napht [2,3-d]oxazole sous forme de cristaux jaunes.
**Rdt :** 34 %
**F :** 212°C
**Rf :** 0,50 (CH₂Cl₂/Heptane, 80/20)
**SM (I.E.)** : m/z 289 (M+.)
**RMN du** ^{**1**}**H (CDCl**_{**3**}**) : δ (ppm)**
8,30 (m, 1H, H-6')
8,27 (m, 2H, H-5, H-8)
7,81 (m, 2H, H-6, H-7)
7,47 (m, 1H, H-4')
7,37 (m, 2H, H-3', H-5')
2,84 (s, 3H, CH₃)
**RMN du** ^{**13**}**C (CDCl**_{**3**}**) :** δ **(ppm)**
179,05, 173,50 (2C, C-4, C-9)
140,81 (1C, C-2')
134,77 (2C, C-6, C-7)
133,50 (1C, C-3')
132,50 (1C, C-4')
130,80 (1C, C-6')
127,91 127,49 (2C, C-5, C-8)
125,60 (1C, C-5')
123,45 (1C, C-1')
22,50 (1C, CH₃)
**IR (KBr) :** ν **(cm**^{**-1**}**)**
1668, 1678 (C=O)

### Exemple 7

### 4,9-Dihydro-4,9-dioxo-2-(3-méthylphényl)-napht[2,3-d]oxazole

A une solution de 5 g (24 mmoles) de 2-amino-3-chloro-1,4-dihydro-1,4-dioxo-naphtalène dans 70 ml de dioxane, on ajoute à l'abri de la lumière 32,00 ml (240 mmoles) de chlorure d'acide 3-méthylbenzoïque puis, après 5 minutes d'agitation, 0,50 ml d'acide sulfurique concentré. Après 45 minutes de reflux et complet refroidissement, on ajoute 200 ml d'éther et le précipité qui se forme est éliminé par filtration. Le filtrat rouge est évaporé à sec, repris dans du dichlorométhane, lavé plusieurs fois avec l'eau, séché et purifié sur colonne flash (support : silice ; conditionnement : heptane ; éluant : dichlorométhane/heptane, 50/50). Les cristaux jaunes formés après évaporation du solvant sont décolorés, recristallisés dans du dichlorométhane pour fournir 3 g de 4,9-dihydro-4,9-dioxo-2-(3-méthylphényl)-napht[2,3-d]-oxazole sous forme de cristaux jaunes.
**Rdt :** 43 %
**F :** 255°C
**Rf :** 0,43 (CH₂Cl₂/Heptane, 80/20)
**SM (I.E.) :** m/z 289 (M+.)
**RMN du** ^{**1**}**H (CDCl**_{**3**}**) : δ (ppm)**
8,26 (m, 2H, H-5, H-8)
8,17 (s, 1H, H-2')
8,13 (m, 1H, H-5')
7,82 (m, 2H, H-6, H-7)
7,43 (m, 2H, H-4', H-6')
2,48 (s, 3H, CH₃)
**RMN du** ^{**13**}**C (CDCl**_{**3**}**) : δ (ppm)**
179,13, 173,72 (2C, C-4, C-9)
166,95 (1C, C-2)
150,65, 144,17 (2C, C-3a, C-9a)
139,62 (1C, C-3')
134,81, 134,77 ; 134,32 (3C, C2', C-6, C-7)
132,86, 132,55 (2C, C-4a, C-8a)
129,54, 129,26 (2C, C-4', C-5')
127,91, 127,49 (2C, C-5, C-8)
125,86 (1C, C-6')
125,44 (1C, C-1')
21,71 (1C, CH₃)
**IR (KBr) : ν (cm**^{**-1**}**)**
1693, 1678 (C=O)

### Exemple 8

### 4,9-Dihydro-4,9-dioxo-2-(4-méthoxyphényl)-napht[2,3-d]oxazole

A une solution de 5,0 g (24 mmoles) de 2-amino-3-chloro-1,4-dihydro-1,4-dioxo-naphtalène dans 80 ml de dichlorométhane, on ajoute à l'abri de la lumière et à température ambiante 16 ml (120 mmoles) de chlorure de l'acide 4-méthoxybenzoïque puis 0,003 ml d'acide sulfurique concentré. Après 3 heures de reflux le mélange réactionnel est évaporé à sec, et le résidu huileux noir obtenu est repris par 300 ml de dichlorométhane, lavé avec une solution d'hydroxyde de sodium 10 N, puis avec de l'eau et enfin séché sur du chlorure de calcium. Le produit est ensuite purifié sur colonne flash (silice 6-35 mm; conditionnement : heptane; éluant : dichlorométhane/heptane, 60/40), pour fournir 4,1 g de 4,9-dihydro-4,9-dioxo-2-(4-méthoxyphényl)-napht[2,3-d]oxazole sous forme de cristaux jaune-orangé après recristallisation et décoloration au noir animal.
**Rdt :** 56 %
**F : >** 260°C
**Rf :** 0,44 (CH₂Cl₂/Ethanol, 99/1)
**SM (I.E.) :** m/z 305 (M+.)
**RMN du** ^{**1**}**H (CDCl**_{**3**}**) : δ (ppm)**
8,28 (dd, 2H, H-5, H-8, J_{H5-H6} = J_{H7-H8} = 8,85 Hz, J_{H5-H7} = J_{H6-H8} = 1,73 Hz)
8,24 (d, 2H, H-2', H-6', J_{H2'-H3'} = J_{H5'-H6'} = 8,60 Hz)
7,80 (m, 2H, H-6, H-7)
7,05 (d, 2H, H-3', H-5', J_{H2'-H3'} = J_{H5'-H6'} = 8,60 Hz)
3,92 (s, 3H, CH₃)
**IR (KBr) : ν (cm**^{**-1**}**)**
1750, 1680 (C=O)

### Exemple 9

### 2-(2-chlorophényl)-4,9-dihydro-4,9-dioxo-napht[2,3-d]oxazole

A une solution de 5 g (24 mmoles) de 2-amino-3-chloro-1,4-dihydro-1,4-dioxo-nahpthalene dans 60 ml de dioxane, on ajoute 15,3 ml (120 mmoles) de chlorure d'acide 2-chlorobenzoïque puis, après 5 minutes d'agitation, 0,5 ml d'acide sulfurique concentré. Le mélange réactionnel est porté à reflux pendant 4 heures et le précipité obtenu après évaporation du dioxane sous pression réduite est dissous dans 200 ml de dichlorométhane. A cette solution, on ajoute à froid 100 ml d'une solution d'hydroxyde de sodium 10 N. On laisse agiter pendant 2 heures. La phase organique est alors extraite, lavée plusieurs fois avec l'eau et séchée sur du chlorure de calcium. Le résidu solide obtenu après évaporation du solvant est purifié sur colonne moyenne pression (support : silice ; éluant : dichlorométhane/heptane, 40/60). Le produit obtenu est recristallisé après décoloration dans un mélange (1/1) heptane/dichlorométhane pour fournir 5 g de 2-(2-chlorophényl)-4,9-dihydro-4,9-dioxo-napht[2,3-d]-oxazole sous forme de cristaux jaune-clair.
**Rdt :** 67 %
**F** : 216°C
**Rf :** 0,42 (CH₂Cl₂/Heptane, 80/20)
**SM** (I.E.) : m/z 309, 311(M+.)
**RMN du** ^{**1**}**H (CDCl**_{**3**}**) : δ (ppm)**
8,26 (m, 3H, H-5, H-8, H-6')
7,82 (m, 2H, H-6, H-7)
7,53 (m, 3H, H-3', H-4', H-5')
**RMN du** ^{**13**}**C (CDCl**_{**3**}**) : δ (ppm)**
134,99, 134,87 (3C, C-2', C-6, C-7)
133,78, 132,84, 132,18 (4C, C-3', C-4', C-5', C-6')
132,38, 132,04 (2C, C-4a, C-8a)
128,04, 127,63 (2C, C-5, C-8)
124,80 (1C, C-1')
**IR (KBr) : ν (cm**^{**-1**}**)**
1691, 1674 (C=O)

### Exemple 10

### 2-(4-Chlorophényl)-4,9-dihydro-4,9-dioxo-napht[2,3-d]oxazole

A une solution de 5,0 g (24 mmoles) de 2-amino-3-chloro-1,4-dihydro-1,4-dioxo-naphtalène dans 60 ml de dioxane on ajoute à température ambiante 15,3 ml (120 mmoles) de chlorure de l'acide 4-chlorobenzoïque et 0,5 ml d'acide sulfurique concentré. Le mélange réactionnel est porté à reflux pendant 4 heures puis est évaporé à sec, repris par 200 ml de dichlorométhane et neutralisé à froid par 100 ml d'hydroxyde de sodium 10 N. La phase organique est ensuite lavée trois fois à l'eau et séchée sur du chlorure de calcium. La poudre rouge ainsi obtenue est purifiée sur colonne flash (support : silice ; conditionnement : heptane ; éluant : dichlorométhane/heptane, 70/30). Les cristaux jaunes obtenus après évaporation du solvant sont décolorés, recristallisés dans du dichlorométhane pour fournir 2,5 g de 2-(4-chlorophényl)-4,9-dihydro-4,9-dioxo-1H-napht[2,3-d]oxazole sous forme de cristaux jaunes.
**Rdt :** 34 %
**F :** > 260°C
**Rf :** 0,40 (CH₂Cl₂/Heptane, 80/20)
**SM (I.E.) :** m/z 309, 311 (MH+)
**RMN du** ^{**1**}**H (CDCl**_{**3**}**) : δ (ppm)**
8,28 (m, 4H, H-5, H-8, H-2', H-6')
7,82 (m, 2H, H-6, H-7)
7,55 (d, 2H, H-3', H-5', J_{H2'-H3'} = J_{H5'-H6'} = 8,34 Hz).
**RMN du** ^{**13**}**C (CDCl**_{**3**}**) : δ (ppm)**
178,55, 173,21 (2C, C-4, C-9)
165,31 (1C, C-2)
150,35, 144,03 (2C, C-3a, C-9a)
139,55 (1C, C-4')
134,48, 134,43 (2C, C-6, C-7)
132,38, 132,04 (2C, C-4a, C-8a)
129,65, 129,49 (4C, C-2', C-3', C-5', C-6')
127,55, 127,09 (2C, C-5, C-8)
123,63 (1C, C-1')
**IR (KBr) : ν (cm**^{**-1**}**)**
1695, 1675 (C=O)

### Exemple 11

### 4,9-Dihydro-4,9-dioxo-2-(2-thiényl)-napht[2,3-d]-oxazole

A une solution de 5,0 g (24 mmoles) de 2-amino-3-chloro-1,4-dihydro-1,4-dioxo-naphtalène dans 50 ml de dichlorométhane, on ajoute à l'abri de la lumière et à température ambiante 12,9 ml (120 mmoles) de chlorure de l'acide 2-thénoyle puis 3 µl d'acide sulfurique concentré. Après 21 heures de reflux, le mélange réactionnel est évaporé à sec et le résidu huileux noir obtenu est repris par 100 ml de dichlorométhane et lavé avec une solution d'hydroxyde de sodium 10 N, puis à l'eau et finalement séché sur du chlorure de calcium. Le produit est ensuite purifié sur colonne flash (silice 6-35 µm ; conditionnement : heptane ; éluant : dichlorométhane/ heptane, 60/40), pour fournir 5,3 g de 4,9-dihydro-4,9-dioxo-2-(2-thiényl)-napht[2,3-d]oxazole sous forme de cristaux jaune-orangé après recristallisation et décoloration au noir animal.
**Rdt : 78 %**
**F : >** 260°C
**Rf :** 0,41 (CH₂Cl₂)
**SM (I.E.)** : m/z 281(M+.)
**RMN du** ^{**1**}**H (CDCl**_{**3**}**) : δ (ppm)**
8,22 (dd, 2H, H-5, H-8, J_{H5-H6} = 8,85 Hz, J_{H5-H7} = 1,73 Hz)
8,06 (dd, 1H, H-5', J_{H5'-H4'} = 3,49 Hz, J_{H5'-H3'} = 1,00 Hz)
7,83 (m, 2H, H-6, H-7)
7,73 (dd, 1H, H-3', J_{H3'-H4'} = 4,88 Hz, J_{H3'-H5'} = 1,00 Hz)
7,27 (dd, 1H, H-4', J_{H4'-H3'} = 4,88 Hz, J_{H4'-H5'} = 3,49 Hz)
**IR (KBr) : ν (cm**^{**-1**}**)**
1687, 1667 (C=O)

### Exemple 12

### 4,9-Dihydro-4,9-dioxo-2-(2-fluorophényl)-naphto[2,3-d]thiazole

A une suspension de 5,0 g (24 mmoles) de 2-amino-3-chloro-1,4-dihydro-1,4-dioxo-naphtalène dans 80 ml d'eau, on ajoute 8,4 g (35 mmoles) de sulfure de sodium nonahydraté. Le mélange réactionnel est porté au reflux pendant environ 20 minutes, puis on ajoute 20 ml d'une solution aqueuse contenant 2,0 g de sulfure de sodium. Quand le milieu vire complètement à la coloration bleue on additionne successivement 1,95 ml (24 mmoles) de 2-fluorobenzaldéhyde et 6,36 ml d'acide acétique glacial. Après une heure de reflux, le produit semi-solide noir obtenu est filtré, séché puis purifié sur colonne flash (support : silice ; conditionnement : heptane ; éluant : dichlorométhane/heptane, 50/50). Les cristaux verdâtres formés après évaporation du solvant sont lavés plusieurs fois avec l'éthanol et l'éther puis décolorés et recristallisés dans du dichlorométhane pour fournir 2,0 g de 4,9-dihydro-4,9-dioxo-2-(2-fluorophényl)-naphto[2,3-d]-thiazole sous forme de cristaux jaunes.
**Rdt :** 27 %
**F :** > 260°C
**Rf :** 0,60 (CH₂Cl₂)
**SM (I.E.) :** m/z 309 (M+.)
**RMN du** ^{**1**}**H (CDCl**_{**3**}**) :** δ **(ppm)**
8,62 (m, 1H, H-6')
8,30, 8,21 (2dd, 2H, H-5, H-8, J_{H5-H6} = J_{H7-H8} = 8,85 Hz, J_{H6-H8} = J_{H5-H7} = 1,73 Hz)
7,81 (m, 2H, H-6, H-7)
7,56 (m, 1H, H-4')
7,29 (m, 2H, H-3', H-5')
**RMN du** ^{**13**}**C (CDCl**_{**3**}**) : δ (ppm)**
179,17 (2C, C-4, C-9)
169,20 (1C, C-2')
153,47 (1C, C-2)
134,43, 134,09 (2C, C-6, C-7)
133,70, 133,56 (2C, C-4a, C-8a)
130,02 (1C, C-6')
127,86, 126,97 (2C, C-5, C-8)
124,99 (1C, C-4')
120,00 (1C, C-5')
116,48, 115,90 (2C, C-1', C-3')
**IR (KBr) : ν (cm**^{**-1**}**)**
1683, 1661 (C=O)

### Exemple 13

### 4,9-Dihydro-4,9-dioxo-2-(3-fluorophényl)-naphto[2,3-d]thiazole

A une suspension de 5,0 g (24 mmoles) de 2-amino-3-chloro-1,4-dihydro-1,4-dioxo-naphtalène dans 50 ml d'eau, on ajoute une solution contenant 8,4 g (35 mmoles) de sulfure de sodium nonahydraté dans 50 ml d'eau. Le mélange réactionnel est porté à reflux pendant environ 20 minutes. Lorsque le milieu réactionnel devient bleu on additionne successivement 2,50 ml (24 mmoles) de 3-fluorobenzaldéhyde et 6,36 ml d'acide acétique glacial. Après deux heures de reflux, le produit noir obtenu est filtré, dissous dans du dichlorométhane, lavé avec de l'eau distillée, séché sur du chlorure de calcium, filtré et évaporé sous pression réduite. Le solide noir-vert obtenu est ensuite purifié sur colonne flash (support : silice ; conditionnement : heptane ; éluant : dichlorométhane/heptane, 50/50, puis dichlorométhane, puis dichlorométhane/méthanol, 99/1). Le solide obtenu après évaporation du solvant est lavé plusieurs fois avec du méthanol, de l'éthanol et de l'éther puis décoloré et recristallisé dans du dichlorométhane pour fournir 4,0 g de 4,9-dihydro-4,9-dioxo-2-(3-fluorophényl)-naphto[2,3-d]-thiazole sous forme de cristaux jaunes.
**Rdt :** 54 %
**F :** > 260°C
**Rf :** 0,56 (CH₂Cl₂)
**SM (I.E.) :** m/z 309 (M+.)
**RMN du** ^{**1**}**H (CDCl**_{**3**}**) : δ (ppm)**
8,37, 8,25 (2dd, 2H, H-5, H-8, J_{H5-H6} = J_{H7-H8} = 8,85 Hz, J_{H6-H8} = J_{H5-H7} = 1,73 Hz)
7,95 (m, 2H, H-2', H-6')
7,84 (m, 2H, H-6, H-7)
7,55 (m, 1H, H-5')
7,25 (m, 1H, H-4')
**IR (KBr) : ν (cm**^{**-1**}**)**
1676, 1661 (C=O)

### Exemple 14

### 4,9-Dihydro-4,9-dioxo-2-(4-fluorophényl)-naphto[2,3-d]thiazole

A une suspension de 5,0 g (24 mmoles) de 2-amino-3-chloro-1,4-dihydro-1,4-dioxo-naphtalène dans 130 ml d'eau, on ajoute 8,4 g (35 mmoles) de sulfure de sodium nonahydraté. Le mélange réactionnel est porté à reflux pendant 20 minutes, puis on ajoute 50 ml d'une solution aqueuse contenant 1,0 g de sulfure de sodium. Le milieu vire complètement à la coloration bleue. On additionne successivement 2,60 ml (24 mmoles) de 4-fluorobenzaldéhyde et 6,36 ml d'acide acétique glacial. Après une heure de reflux, le précipité verdâtre obtenu est filtré, séché puis purifié sur colonne flash (support : silice ; conditionnement : heptane ; éluant : dichlorométhane/heptane, 60/40). Les cristaux jaunes formés après évaporation du solvant sont lavés successivement avec l'isopropanol et l'éther puis décolorés et recristallisés dans du dichlorométhane pour fournir 2,0 g de 4,9-dihydro-4,9-dioxo-2-(4-fluorophényl)-naphto[2,3-d] -thiazole sous forme de cristaux jaunes.
**Rdt :** 27 %
**F : >** 260°C
**Rf :** 0,51 (CH₂Cl₂/Heptane, 90/10)
**SM (I.E.) :** m/z 309 (M+.)
**RMN du** ^{**1**}**H (CDCl**_{**3**}**) : δ (ppm)**
8,30, 8,21 (2dd, 2H, H-5, H-8, J_{H5-H6} = J_{H8-H7} = 8,85 Hz, J_{H5-H7} = J_{H8-H6} = 1,73 Hz)
8,12 (m, 2H, H-2', H-6')
7,83 (m, 2H, H-6, H-7)
7,22 (m, 2H, H-3', H-5')
**RMN du** ^{**13**}**C (CDCl**_{**3**}**) : δ (ppm)**
179,17 (2C, C-4, C-9)
165,21 (1C, C-4')
134,46, 134, 04 (2C, C-6, C-7)
132,78, 132,43 (2C, C-4a, C-8a)
130,08, 129,96 (2C, C-2', C-6')
127,88, 126,98 (2C, C-5, C-8)
116,74, 116,42 (2C, C-3', C-5')
**IR (KBr) : ν (cm**^{**-1**}**)**
1678, 1659 (C=O)

### Exemple 15

### 2-(2,4-Difluorophényl)-4,9-dihydro-4,9-dioxo-naphto-[2,3-d]thiazole

A 5,0 g (24,15 mmoles) de 2-amino-3-chloro-1,4-dihydro-1,4-dioxo-naphtalène dans 150 ml d'eau, on ajoute 150 ml d'une solution aqueuse contenant 29,0 g (120,00 mmoles) de sulfure de sodium nonahydraté. Le mélange réactionnel est porté à reflux pendant 20 minutes. Le milieu vire complètement à la coloration bleue. On additionne successivement 2,0 ml (18,00 mmoles) de 2,4-difluorobenzaldéhyde et 6,3 ml d'acide acétique glacial. Après 3 heures de reflux, le solide formé est filtré, lavé à l'eau distillée, séché puis purifié sur cake (support : silice ; éluant : dichlorométhane). Les cristaux jaunes obtenus après évaporation des solvants sont recristallisés dans du dichlorométhane, décolorés au noir animal pour fournir 3,9 g de 2-(2,4-difluorophényl)-4,9-dihydro-4,9-dioxo-naphto[2,3-d]thiazole.
**Rdt :** 97 %
**F : >** 260°C
**Rf :** 0,40 (CH₂Cl₂)
**SM (I.E.)** : m/z 327 (M+.)
**RMN du** ^{**1**}**H (CDCl**_{**3**}**) : δ (ppm)**
8,55 (m, 1H, H-3')
8,28, 8,24 (2dd, 2H, H-5, H-8, J_{H5-H6} = J_{H7-H8} = 8,85 Hz, J_{H5-H7} = J_{H6-H8} = 1,73 Hz)
7,84 (m, 2H, H-6, H-7)
7,10 (m, 2H, H-5', H-6')
**RMN du** ^{**13**}**C (CDCl**_{**3**}**) : δ (ppm)**
178,88, 177,93 (2C, C-4, C-9)
163,45 (1C, C-2)
134,87, 134,52 (2C, C-6, C-7)
133,44, 133,10, 131,61 (4C, C-3a, C-4a, C-8a, C-9a)
127,95, 127,18 (2C, C-5, C-8)
123,45 (1C, C-1')
113,53, 113,21 (2C, C-2', C-4')
105,56, 105,18, 104,79 (3C, C-3', C-5', C-6')
**IR (KBr) : ν (cm**^{**-1**}**)**
1681, 1658 (C=O)

### Exemple 16

### 4,9-Dihydro-4,9-dioxo-2-(3-pyridyl)-naphto[2,3-d]-thiazole

A une suspension de 10,00 g (48,2 mmoles) de 2-amino-3-chloro-1,4-dihydro-1,4-dioxo-naphtalène dans 100 ml d'eau, on ajoute 13,88 g (74,5 mmoles) de sulfure de sodium nonahydraté. Le mélange réactionnel, porté progressivement à reflux, vire à la coloration bleue dès 60°C. L'ajout de 4,00 g (16,7 mmoles) de sulfure de sodium concentré dans de l'eau est nécessaire pour compléter le changement de coloration. On ajoute alors successivement 6 ml (60,6 mmoles) de 3-pyridine carboxaldéhyde et 10 ml d'acide acétique glacial. Après deux heures de reflux, et complet refroidissement, on additionne 300 ml d'éthanol. Le précipité formé est éliminé par filtration. Le filtrat est évaporé à sec, repris dans du dichlorométhane, lavé plusieurs fois avec de l'éther puis avec de l'eau, séché et purifié sur colonne moyenne pression (support : silice ; conditionnement : heptane ; éluant : dichlorométhane/méthanol, 100/0 à 99/1). Les cristaux jaunes formés après évaporation du solvant sont lavés avec l'éthanol puis décolorés, recristallisés dans du dichlorométhane pour fournir 1,00 g de 4,9-dihydro-4,9-dioxo-2-(3-pyridyl)-naphto[2,3-d]thiazole sous forme de cristaux jaunes.
**Rdt :** 7 %
**F :** 256°C
**Rf :** 0,50 (CH₂Cl₂/Méthanol, 98/2)
**SM (I.E.) :** m/z 292 (M+.)
**RMN du** ^{**1**}**H (CDCl**_{**3**}**) : δ (ppm)**
9,33 (d, 1H, H-2', J = 1,83 Hz)
8,80 (d, 1H, H-6', J_{H5'-H6'} = 3,36 Hz)
8,49 (d, 1H, H-4', J_{H4'-H5}, = 7,93 Hz)
8,36, 8,26 (2dd, 2H, H-5, H-8, J_{H5-H6} = J_{H7-H8} = 8,85 Hz, J_{H5-H7} = J_{H6-H8} = 1,73 Hz)
7,84 (m, 2H, H-6, H-7)
7,50 (dd, 1H, H-5', J_{H4'-H5'} = 7,93 Hz, J_{H5'-H6'} = 3,36 Hz)
**RMN du** ^{**13**}**C (CDCl**_{**3**}**) : δ (ppm)**
178,13, 177,78 (2C, C-4, C-9)
177,67 (1C, C-2)
155,18 (1C, C-3a)
152,87, 148,58 (2C, C-2', C-6')
142,18 (1C, C-9a)
134,77, 134,31 (2C, C-6, C-7)
134,18 (1C, C-4')
132,74, 132,01 (2C, C-4a, C-8a)
128,36 (1C, C-3')
128,04, 127,16 (2C, C-5, C-8)
124,56 (1C, C-5')
**IR (KBr) : ν (cm**^{**-1**}**)**
1680, 1660 (C=O)

### Exemple 17

### Sulfate de 4,9-Dihydro-4,9-dioxo-2-(4-pyridyl)-naphto[2,3-d]thiazole

A une suspension de 500 mg (1,71 mmole) de 4,9-dihydro-4,9-dioxo-2-(4-pyridyl)-naphto[2,3-d]thiazole dans 60 ml de méthanol, on ajoute 60 ml d'une solution méthanolique contenant 0,18 ml (1,74 mmole) d'acide sulfurique à 98 %. Après une heure de reflux et complet refroidissement, le précipité jaune obtenu est filtré, rincé plusieurs fois avec l'éther éthylique puis séché. On obtient ainsi 500 mg de sulfate de 4,9-dihydro-4,9-dioxo-2-(4-pyridyl)-naphto[2,3-d]thiazole, sous forme de cristaux jaunes.
**Rdt :** 75 %
**F : >** 260°C
**Rf :** 0,50 (CH₂Cl₂/Méthanol, 96/4)
**IR (KBr) :** ν **(cm**^{**-1**}**)**
de 3100 et 2725 (NH+) ; 1686, 1668 (C=O)

### Exemple 18

### 4,9-Dihydro-4,9-dioxo-2-(3-furyl)-naphto[2,3-d]-thiazole

A 6,0 g (29 mmoles) de 2-amino-3-chloro-1,4-dihydro-1,4-dioxo-naphtalène, on ajoute 100 ml d'une solution aqueuse fraîchement préparée contenant 34,8 g (145 mmoles) de sulfure de sodium nonahydraté. Le mélange réactionnel est agité à 70°C pendant 20 minutes, jusqu'à obtention d'une coloration bleue. On additionne alors successivement 2,5 ml (29 mmoles) de 3-furaldéhyde et 7,6 ml (133 mmoles) d'acide acétique glacial. Après deux heures d'agitation à 50°C, puis lheure 40 minutes à température ambiante, le précipité jaune foncé obtenu est filtré et lavé deux fois avec 500 ml d'eau. On obtient ainsi 7,0 g de cristaux qui sont repris par 500 ml de dichlorométhane, lavés trois fois avec 750 ml d'eau puis séchés sur du chlorure de calcium et filtrés. Après évaporation du dichlorométhane sous pression réduite, le solide orange obtenu est purifié par filtration sur lit de silice (dichlorométhane/heptane, 80/20) pour fournir 3,5 g de 4,9-dihydro-4,9-dioxo-2-(3-furyl)-naphto[2,3-d]thiazole sous forme de cristaux jaunes qui sont recristallisés dans l'acétate d'éthyle après décoloration au noir animal.
**Rdt :** 43 %
**F :** 245°C
**Rf :** 0,58 (CH₂Cl₂)
**SM (I.E.) :** m/z 281 (M+.)
**RMN du** ^{**1**}**H (CDCl**_{**3**}**) :** δ **(ppm)**
8,34 (dd, 1H, H-5 ou H-8, J_{H5-H6} = J_{H7-H8} = 8,85 Hz, J_{H5-H7} = J_{H6-H8} = 1,73 Hz)
8,28 (dd, 1H, H-2', J_{H2'-H5'} = 1,50 Hz, J_{H2'-H4'} = 0,90 Hz)
8,23 (dd, 1H, H-5 ou H-8, J_{H5-H6} = J_{H7-H8} = 8,85 Hz, J_{H5-H7} = J_{H6-H8} = 1,73 Hz)
7,81 (m, 2H, H-6, H-7)
7,56 (t, 1H, H-5', J_{H2'-H5'} = 1,50 Hz)
6,98 (dd, 1H, H-4', J_{H2'-H4'} = 0,90 Hz, J_{H4'-H5'} = 1,80 Hz)
**RMN du** ^{**13**}**C (CDCl**_{**3**}**) : δ (ppm)**
178,28, 177,83 (2C, C-4, C-9)
167,28 (1C, C-2)
155,05 (1C, C-3a)
144,77, 143,90 (2C, C-2', C-5')
140,77 (1C, C-9a)
134,39, 134,06 (2C, C-6, C-7)
133,04, 132,63 (2C, C-4a, C-8a)
127,86, 126,90 (2C, C-5, C-8)
120,80 (1C, C-3')
109,19 (1C, C-4')
**IR (KBr) : ν (cm**^{**-1**}**)**
1678, 1656 (C=O)

### Exemple 19

### 2-(5-Chloro-furan-2-yl)-4,9-dihydro-4,9-dioxo-naphto[2,3-d]thiazole

A une solution de 1,78 g (6,3 mmoles) de 4,9-dihydro-4,9-dioxo-2-(2-furyl)-naphto[2,3-d]thiazole dans 600 ml de chloroforme, on fait barboter à 0°C du chlore gazeux pendant deux minutes. Le mélange réactionnel est encore agité pendant 10 minutes jusqu'à obtention d'une solution jaune claire. L'excès de chlore est alors éliminé en faisant passer un courant d'argon. Le produit solide jaune obtenu après évaporation sous pression réduite est ensuite purifié sur colonne flash (silice 6-35 µm ; conditionnement : heptane ; éluant : dichlorométhane/ heptane, 50/50), pour fournir 0,43 g de cristaux jaunes pâles. Après filtration sur lit de silice on obtient 0,40 g de 2-(5-chloro-furan-2-yl)-4,9-dihydro-4,9-dioxo-naphto[2,3-d]thiazole sous forme de cristaux jaunes après recristallisation et décoloration au noir animal.
**Rdt :** 20 %
**F :** 257,7°C
**Rf :** 0,42 (CH₂Cl₂)
**SM (I.E.) :** m/z 315 (M+.)
**RMN du** ^{**1**}**H (CDCl**_{**3**}**) : δ (ppm)**
8,33 (dd, 1H, H-5 ou H-8, J_{H5-H6} = 8,85 Hz, J_{H5-H7} = 1,73 Hz)
8,23 (dd, 1H, H-5 ou H-8, J_{H5-H6} = 8,85 Hz, J_{H5-H7} = 1,73 Hz)
7,81 (m, 2H, H-6, H-7)
7,43 (d, 1H, H-3', J_{H3'-H4'} = 3,58 Hz)
6,45 (d, 1H, H-4', J_{H3'-H4'} = 3,58 Hz)
**RMN du** ^{**13**}**C (CDCl**_{**3**}**) : δ (ppm)**
178,10, 177,92 (2C, C-4, C-9)
163,94 (1C, C-2)
141,10 (1C, C-2')
140,78 (1C, C-3a)
139,58 (1C, C-9a)
134,40, 134,17 (2C, C-6, C-7)
133,10, 132,68 (2C, C-4a, C-8a)
127,86, 126,95 (2C, C-5, C-8)
115,65 (1C, C-3')
113.34 (1C, C-4')
**IR (KBr) :** ν **(cm**^{**-1**}**)**
1678, 1652 (C=O)

### Exemple 20

### 4,9-Dihydro-4,9-dioxo-2-(2-thiényl)-naphto[2,3-d]-thiazole

A 4,00 g (19 mmoles) de 2-amino-3-chloro-1,4-dihydro-1,4-dioxo-naphtalène, on ajoute 150 ml d'une solution aqueuse fraîchement préparée contenant 22,80 g (95 mmoles) de sulfure de sodium nonahydraté. Le mélange réactionnel est porté à 80°C pendant 20 minutes, jusqu'à obtention d'une coloration bleue. On additionne alors successivement 1,8 ml (19 mmoles) de thiophène-2-carboxaldéhyde et 5,0 ml (87 mmoles) d'acide acétique glacial. Après deux heures d'agitation, le précipité noir-marron obtenu est filtré, dissous dans 350 ml de dichlorométhane. La phase organique est lavée trois fois avec 150 ml d'eau, séchée sur du chlorure de calcium et filtrée. Le solide orange obtenu après évaporation du dichlorométhane sous pression réduite est décoloré au noir animal et recristallisé dans du dichlorométhane pour fournir 2,71 g de 4,9-dihydro-4,9-dioxo-2-(2-thiényl)-naphto[2,3-d]thiazole sous forme de cristaux rouge-orangé.
**Rdt :** 48 %
**F : >** 260°C
**Rf :** 0,58 (CH₂Cl₂)
**SM (I.E.) :** m/z 297 (MH+.)
**RMN du** ^{**1**}**H (CDCl**_{**3**}**) :** δ (ppm)
8,32 (dd, 1H, H-5 ou H-8, J_{H5-H6} = J_{H7-H8} = 8,85 Hz, J_{H5-H7} = J_{H6-H8} = 1,73 Hz)
8,21 (dd, 1H, H-5 ou H-8, J_{H5-H6} = J_{H7-H8} = 8,85 Hz, J_{H5-H7} = J_{H6-H8} = 1,73 Hz)
7,82 (m, 3H, H-6, H-7, H-5')
7,62 (dd, 1H, H-3', J_{H3'-H4'} = 4,88 Hz, J_{H3'-H5'} = 1,00 Hz)
7,18 (dd, 1H, H-4', J_{H3'-H4'} = 4,88 Hz, J_{H4'-H5'} = 3,49 Hz)
**RMN du** ^{**13**}**C (CDCl**_{**3**}**) : δ (ppm)**
178,32, 177,92 (2C, C-4, C-9)
169,34 (1C, C-2)
155,14 (1C, C-3a)
135,50 (1C, C-9a)
134,37, 134,02 (2C, C-6, C-7)
132,78, 132,43 (2C, C-4a, C-8a)
130,19 (1C, C-4')
128,50 (1C, C-3')
127,84, 127,61 (2C, C-5, C-8)
126,86 (C-5')
**IR (KBr) : ν (cm**^{**-1**}**)**
1676, 1654 (C=O)

### Exemple 21

### 4,9-Dihydro-4,9-dioxo-2-(3-thiényl)-naphto[2,3-d]-thiazole

A 4,0 g (19 mmoles) de 2-amino-3-chloro-1,4-dihydro-1,4-dioxo-naphtalène, on ajoute 90 ml d'une solution aqueuse fraîchement préparée contenant 22,8 g (95 mmoles) de sulfure de sodium nonahydraté. Le mélange réactionnel est porté à 90°C pendant 20 minutes, jusqu'à obtention d'une coloration bleue. On additionne alors successivement 1,8 ml (19 mmoles) de thiophène-3-carboxaldéhyde et 5,0 ml (87 mmoles) d'acide acétique glacial. Après deux heures d'agitation à 90°C, le précipité jaune-verdâtre obtenu est filtré, lavé trois fois avec 400 ml d'eau et séché. Les cristaux sont repris dans 200 ml d'isopropanol, agités à température ambiante pendant une heure puis filtrés, séchés et recritallisés dans du dichlorométhane après décoloration au noir animal pour fournir 4,0 g de 4,9-dihydro-4,9-dioxo-2-(3-thiényl)-naphto[2,3-d]thiazole sous forme de cristaux jaune-ocres.
**Rdt :** 70 %
**F :** 258°C
**Rf :** 0,55 (CH₂Cl₂/Méthanol, 99,5/0,5)
**SM (I.E.) :** m/z 297 (MH+.)
**RMN du** ^{**1**}**H (CDCl**_{**3**}**) : δ (ppm)**
8,37 (dd, 1H, H-5 ou H-8, J_{H5-H6} ou J_{H7-H8} = 8,85 Hz, J_{H5-H7} ou J_{H6-H8} = 1,73 Hz)
8,23 (m, 2H, H-5 ou H-8, H-2')
7,81 (m, 2H, H-6, H-7)
7,71 (d, 1H, H-5', J_{H4'-H5'} = 4,88 Hz)
7,48 (dd, 1H, H-4', J_{H4'-H5'} = 4,88 Hz, J_{H2'-H4'} = 2,99 Hz)
**RMN du** ^{**13**}**C (CDCl**_{**3**}**) : δ (ppm)**
178,32, 177,92 (2C, C-4, C-9)
169,34 (1C, C-2)
155,14 (1C, C-3a)
140,69 (1C, C-9a)
134,36, 134,04 (2C, C-6, C-7)
133,09, 132,71 (2C, C-4a, C-8a)
128,23 (1C, C-4')
127,84 (1C, C-5')
127,61, 126,89, 126,59 (3C, C-5, C-8, C-2')
**IR (KBr) : ν (cm**^{**-1**}**)**
1674, 1655 (C=O)

### Exemple 22

### 4,9-Dihydro-4,9-dioxo-2-phénylamino-naphto[2,3-d]-thiazole

A une solution de 200 mg (0,8 mmoles) de 2-chloro-4,9-dihydro-4,9-dioxo-naphto[2,3-d]thiazole dans 100 ml d'éthanol, on ajoute à 80°C 730 µl (8 mmoles) d'aniline. Le mélange réactionnel est porté à reflux pendant 3,5 heures et le précipité rouge obtenu est filtré après refroidissement puis purifié sur cake (support : silice 6-35 µm ; éluant : dichlorométhane/heptane, 20/80 à 100/0 puis dichlorométhane/acétate d'éthyle, 99,5/0,5 à 0/100). Les fractions propres sont réunies, filtrées sur micropores et le solvant est évaporé sous pression réduite pour fournir 196 mg de 4,9-dihydro-4,9-dioxo-2-phénylaminonaphto[2,3-d]thiazole sous forme de cristaux rouges.
**Rdt :** 80 %
**F :** > 260°C
**Rf :** 0,44 (CH₂Cl₂/Acétate d'éthyle, 90/10)
**SM (I.E.)** : m/z 306 (M+.)
**RMN du** ^{**1**}**H (DMSO d**_{**6**}**) :** δ **(ppm)**
11,34 (s, 1H, NH)
8,09 (m, 2H, H-5, H-8)
7,86 (m, 2H, H-6, H-7)
7,70 (m, 2H, H-2', H-6')
7,44 (m, 2H, H-3', H-5')
7,14 (m, 1H, H-4')
**RMN du** ^{**13**}**C (DMSO d**_{**6**}**) : δ (ppm)**
178,04, 177,31 (2C, C-4, C-9)
167,72 (1C, C-2)
154,61 (1C, C-3a)
145,95 (1C, C-9a)
139,49 (1C, C-1')
134,02, 133,93 (2C, C-6, C-7)
132,79, 132,06 (2C, C-4a, C-8a)
129,36 (2C, C-3', C-5')
126,76, 125,73 (2C, C-5, C-8)
123,68 (1C, C-4')
118,60 (2C, C-2', C-6')
**IR (KBr) : ν (cm**^{**-1**}**)**
3228 (NH) ; 1677, 1632 (C=O)

### Exemples 23 et 24

### 4,9-Dihydro-4,9-dioxo-8-méthoxy-2-phényl-naphto[2,3-d]thiazole et

### 4,9-Dihydro-4.9-dioxo-5-méthoxy-2-phényl-naphto[2,3-d]thiazole

### Intermédiaires de synthèse :

### 1,4-Dihydro-1,4-dioxo-5-méthoxy-naphtalène

A une solution de 26,45 g (0,147 mole) de 1,4-dihydro-1,4-dioxo-5-hydroxy-naphtalène dans 1300 ml de dichlorométhane, on ajoute goutte à goutte 39 ml (0,303 mole) d'iodomethane puis 73,50 g d'oxyde d'argent. Le mélange réactionnel est agité pendant 72 heures puis filtré. Le filtrat est séché sur du chlorure de calcium puis évaporé sous pression réduite. On obtient 28,50 g de cristaux orange qui sont purifiés sur cake (support : silice 40-60 mm ; éluant : heptane/acétate d'éthyle, 70/30 à 0/100) pour fournir 23,80 g de 1,4-dihydro-1,4-dioxo-5-méthoxy-naphtalène.
**Rdt :** 86 %
**F :** 188°C
**Rf :** 0.50 (Acétate d'éthyle/Heptane, 50/50)
**RMN du** ^{**1**}**H (CDCl**_{**3**}**) : δ (ppm)**
7.72 (m, 2H, H-6, H-8)
7.32 (dd, 1H, H-7, J_{H6-H7} = J_{H7-H8} = 7.63 Hz)
6.88 (m, 2H, H-2, H-3)
4.01 (s, 3H, OCH₃)

### 2,3-Dibromo-1,4-dihydro-1,4-dioxo-5-méthoxy-naphtalène

A une solution de 3,5 g (18,6 mmoles) de 1,4-dihydro-1,4-dioxo-5-méthoxy-naphtalène dans 135 ml de chloroforme, on additionne 3,05 g (37,2 mmoles) d'acétate de sodium et 3 ml (58,4 mmoles) de brome. Le milieu réactionnel est agité pendant 48 heures. Les sels d'acétate formés sont filtrés. Le filtrat est lavé à l'eau distillée, séché sur du chlorure de calcium puis évaporé sous pression réduite pour fournir 7,3 3 g de 1,4-dihydro-1,4-dioxo-2,3-dibromo-5-méthoxy-naphtalène sous forme de cristaux orange.
**Rdt :** 100 %
**F :** 190°C
**RMN du** ^{**1**}**H (DMSO d**_{**6**}**) : δ (ppm)**
7,80 (dd, 1H, H-8, J_{H7-H8} = 7,85 Hz, J_{H6-H8} = 1.53 Hz)
7.73 (dd, 1H, H-7, J_{H6-H7} = 8,34 Hz, J_{H6-H8} = 7.85 Hz)
7,37 (m, 1H, H-6)
3,95 (s, 3H, OCH₃)

### 2-Amino-3-bromo-1,4-dihydro-1,4-dioxo-5-méthoxy-naphtalène et

### 2-Amino-3-bromo-1,4-dihydro-1,4-dioxo-8-méthoxy-naphtalène

A une solution de 500,0 mg (1,5 mmole) de 2,3-dibromo-1,4-dihydro-1,4-dioxo-5-méthoxy-naphtalène dans 25 ml de tétrahydrofurane, on ajoute une goutte d'amoniaque. Le milieu réactionnel prend une coloration noire. On fait ensuite passer un courant d'ammoniac pendant 2 heures à 20°C. Le produit brut obtenu après évaporation des solvants est purifié sur cake (support : silice ; éluant : dichlorométhane/heptane, 80/20) pour fournir 347,3 mg d'un mélange de 2-amino-3-brome-1,4-dihydro-1,4-dioxo-5-méthoxy-naphtalène et de de 2-amino-3-bromo-1,4-dihydro-1,4-dioxo-8-méthoxy-naphtalène.
Rdt global : 82 %

### 2-amino-3-bromo-1,4-dihydro-1,4-dioxo-8-méthoxy-naphtalène

**RMN du** ^{**1**}**H (DMSO d**_{**6**}**) : δ (ppm)**
7.78 (d, 1H, H-8, J_{H7-H8} = 7,94 Hz)
7.67 (dd, 1H, H-7, J_{H6-H7} = 8,54 Hz, J_{H7-H8} = 7.94 Hz)
7.25 (d, 1H, H-6, J_{H6-H7} = 8,54 Hz)
3.99 (s, 3H, OCH₃)
1.73 (s, 2H, NH₂)

### 2-amino-3-bromo-1,4-dihydro-1,4-dioxo-5-méthoxy-naphtalène

**RMN du** ^{**1**}**H (DMSO d**_{**6**}**) :** δ **(ppm)**
7,73 (d, 1H, H-8, J_{H7-H8} = 8,57 Hz)
7,61 (t, 1H, H-7, J_{H7-H6} = J_{H7-H8} = 8,57 Hz)
7,34 (d, 1H, H-6, J_{H6-H7} = 8,57 Hz)
3,97 (s, 3H, OCH₃)
1,73 (s, 2H, NH₂)

### 4,9-Dihydro-4,9-dioxo-8-méthoxy-2-phényl-naphto[2,3-d]thiazole (Exemple 23) et 4,9-Dihydro-4,9-dioxo-5-méthoxy-2-phényl-naphto[2,3-d]thiazole (Exemple 24)

A une solution de 93,70 g (389,00 mmoles) de sulfure de sodium nonahydraté dans 400 ml d'eau, on ajoute 18,30 g (64,87 mmoles) d'un mélange (1/1) de 2-amino-3-bromo-5-méthoxy-1,4-dihydro-1,4-dioxo-naphtalène et de 2-amino-3-bromo-8-méthoxy-1,4-dihydro-1,4-dioxo-naphtalène. Le milieu réactionnel est porté à reflux jusqu'à l'obtention d'une coloration bleue. On ajoute alors successivement 6,6 ml (64,87 mmoles) de benzaldéhyde et goutte à goutte 22,3 ml d'acide acétique glacial. Après 1 heure de reflux, et complet refroidissement, le précipité obtenu est filtré, lavé à l'éthanol et repris dans du chloroforme. La phase organique est lavée avec de l'eau puis séchée sur du chlorure de calcium. On obtient 16,50 g de cristaux jaunes après évaporation des solvants sous pression réduite qui sont purifiés sur colonne flash (support : silice 40-60 mm ; éluant : dichlorométhane/acétate d'éthyle, 100/0 à 97/3) pour fournir après décoloration et recristallisation dans du dichlorométhane 8,90 g de 4,9-dihydro-4,9-dioxo-8-méthoxy-2-phényl-naphto[2,3-d]-thiazole (Exemple 23) et 2,29 g de 4,9-dihydro-4,9-dioxo-5-méthoxy-2-phényl-naphto[2,3-d]thiazole (Exemple 24) sous forme de cristaux jaunes.

### 4,9-Dihydro-4,9-dioxo-8-méthoxy-2-phényl-naphto-[2,3-d]thiazole (Exemple 23)

**Rdt** : 42 %
**F : >** 260°C
**Rf :** 0.55 (CH₂Cl₂/Acétate d'éthyle, 90/10)
**SM (I.E.) :** m/z 321 (M+.)
**RMN du** ^{**1**}**H (CDCl**_{**3**}**) : d (ppm)**
8,14 (dd, 2H, H-2', H-6', J_{H2'-H3'} = J_{H5'-H6'} = 6,10 Hz, J_{H2'-H4'} = J_{H4'-H6'} = 1,80 Hz)
7,90 (d, 1H, H-5, J_{H5-H6} = 7,63 Hz)
7,73 (dd, 1H, H-6, J_{H5-H6} = 7,60 Hz, J_{H6-H7} = 8,50 Hz)
7,52 (m, 3H, H-3', H-4', H-5')
7,39 (d, 1H, H-7, J_{H6-H7} = 8,50 Hz)
4,06 (s, 3H, OCH₃)
**RMN du** ^{**13**}**C (CDCl**_{**3**}**) : d (ppm)**
177,95 (1C, C-9)
177,58 (1C, C-4)
161,00 (1C, Cquat)
135,98 (1C, Cquat)
135,01 (1C, C-6)
132,23 (1C, C-4')
129,20 (2C, C-3', C-5')
127,79 (2C, C-2', C-6')
119,78, 119,02 (2C, C-5, C-7)
56,74 (OCH₃)
**IR (KBr) : ν (cm**^{**-1**}**)**
1671 (C=O)

### 4,9-Dihydro-4,9-dioxo-5-méthoxy-2-phényl-naphto-[2,3-d]thiazole (Exemple 24)

**Rdt :** 11 %
**F :** 245°C
**Rf :** 0,47 (CH₂Cl₂/Acétate d'éthyle, 98/2)
**SM (I.E.) :** m/z 321 (M+.)
**RMN du** ^{**1**}**H (CDCl**_{**3**}**) : d (ppm)**
8,15 (dd, 2H, H-2', H-6', J_{H2'-H3'} = J_{H5'-H6'} = 7,98 Hz, J_{H2'-H4'} = J_{H4'-H6'} = 1,90 Hz)
7,92 (d, 1H, H-8, J_{H7-H8} = 7,60 Hz)
7,73 (t, 1H, H-7, J_{H7-H8} = J_{H6-H7} = 8,00 Hz)
7,53 (m, 3H, H-3', H-4', H-5')
7,39 (d, 1H, H-6, J_{H6-H7} = 8,00 Hz)
4,03 (s, 3H, OCH₃)
**RMN du** ^{**13**}**C (CDCl**_{**3**}**) : d (ppm)**
161,50 (1C, C-5)
135,50 (1C, Cquat)
135,08 (1C, C-7)
132,20 (1C, C-4')
129,70 (2C, C-3', C-5')
127,80 (2C, C-2', C-6')
119,75 (1C, C-6)
119,02 (1C, C-8)
57,00 (1C, OCH₃)
**IR (KBr) : ν (cm**^{**-1**}**)**
1678, 1651 (C=O)

### Exemples 25 et 26

### 4,9-Dihydro-4,9-dioxo-7-méthoxy-2-phényl-naphto[2,3-d]thiazole et

### 4,9-Dihydro-4,9-dioxo-6-méthoxy-2-phényl-naphto[2,3-d]thiazole

A une solution de 9,9 g (41,0 mmoles) de sulfure de sodium nonahydraté dans 27 ml d'eau, on ajoute 1,9 g (6,7 mmoles) d'un mélange (1/1) de 2-amino-3-bromo-6-méthoxy-1,4-dihydro-1,4-dioxo-naphtalène et de 3-amino-2-bromo-6-méthoxy-1,4-dihydro-1,4-dioxo-naphtalène. Le milieu réactionnel est alors porté à 50°C jusqu'à l'obtention d'une coloration bleue. On ajoute alors successivement 0,685 ml (6,7 mmoles) de benzaldéhyde et 2,300 ml d'acide acétique glacial. Après 3 heures de chauffage et complet refroidissement, les cristaux verts obtenus sont filtrés, lavés à l'éthanol puis dissous dans 300 ml de chloroforme. La phase organique est lavée avec 100 ml d'eau, séchée sur du chlorure de calcium et évaporée sous pression réduite. On obtient 4,0 g de cristaux jaunes qui sont purifiés sur une colonne de chromatographie moyenne pression (support : silice 6-35 mm, diamètre interne : 3,0 cm, hauteur : 40 cm, pression : 30 bars, éluant : heptane/dichlorométhane, 100/0 à 65/35). Les cristaux jaunes obtenus sont décolorés et recristallisés dans du dichlorométhane pour fournir 0,2 g de 4,9-dihydro-4,9-dioxo-7-méthoxy-2-phényl-naphto[2,3-d]thiazole (Exemple 25) et 1,2 g de 4,9-dihydro-4,9-dioxo-6-méthoxy-2-phényl-naphto-[2,3-d]thiazole (Exemple 26).

### 4,9-Dihydro-4,9-dioxo-7-méthoxy-2-phényl-naphto-[2,3-d]thiazole (Exemple 25)

**Rdt :** 1,5 %
**F :** > 260°C
**Rf :** 0,47 (CH₂Cl₂)
**SM (I.E.) :** m/z 321 (M+.)
**RMN du** ^{**1**}**H (270 MHz, CDCl**_{**3**}**) : d (ppm)**
8,20 (s, 1H, H-8)
8,15 (dd, 2H, H-2', H-6', J_{H2'-H3'} = J_{H5'-H6'} = 7,21 Hz, J_{H2'-H4'} = J_{H4'-H6'} = 1,93 Hz)
7,78 (d, 1H, H-5, J_{H5-H6} = 8,65 Hz)
7,53 (m, 3H, H-3', H-4', H-5')
7,24 (d, 1H, H-6, J_{H5-H6} = 8,65 Hz)
4,01 (s, 3H, OCH₃)
**RMN du** ^{**13**}**C (270 MHz, CDCl**_{**3**}**) : d (ppm)**
132,68 (1C, C-5)
129,84 (1C, C-4')
129,69 (2C, C-3', C-5')
128,20 (2C, C-2', C-6')
120,64 (1C, C-6)
111,99 (1C, C-8)
56,58 (OCH₃)
**IR (KBr) : ν (cm**^{**-1**}**)**
1679 (C=O)

### 4,9-Dihydro-4,9-dioxo-6-méthoxy-2-phényl-naphto[2,3-d]thiazole (Exemple 26)

**Rdt :** 9 %
**F :** 221°C
**Rf :** 0,65 (CH₂Cl₂/Acétate d'éthyle, 98/2)
**SM (I.E.) :** m/z 321 (M+.)
**RMN du** ^{**1**}**H (270 MHz, CDCl**_{**3**}**)** : **d (ppm)**
8,29 (d, 1H, H-8, J_{H7-H8} = 8,65 Hz)
8,14 (d, 2H, H-2', H-6', J_{H2'-H3'} = J_{H5'-H6'} = 7,21 Hz)
7,67 (d, 1H, H-5, J_{H5-H7} = 2,67 Hz)
7,52 (m, 3H, H-3', H-4', H-5')
7,25 (dd, 1H, H-7, J_{H7-H8} = 8,65 Hz, J_{H5-H7} = 2,67 Hz)
4,01 (s, 3H, OCH₃)
**RMN du** ^{**13**}**C (270 MHz, CDCl**_{**3**}**) : d (ppm)**
164,21 (1C, C-6)
155,88 (1C, Cquat)
135,87 (1C, Cquat)
132,31 (1C, C-5)
130,30 (1C, C-4')
129,25 (2C, C-3', C-5')
127,79 (2C, C-2', C-6')
127,40 (1C, Cquat)
120,27 (1C, C-7)
110,09 (1C, C-8)
56, 05 (OCH₃)
**IR (KBr) : ν (cm**^{**-1**}**)**
1667 (C=O)

### Exemple 27

### 4,9-dihydro-4,9-dioxo-8-hydroxy-2-phényl-naphto[2,3-d]thiazole

Une suspension de 1,00 g (0,003 moles) de 4,9-dihydro-4,9-dioxo-8-méthoxy-2-phényl-naphto[2,3-d]-thiazole (Exemple 23) dans 67 ml (1,160 moles) d'acide acétique et 67 ml (0,570 moles) d'acide bromhydrique est portée à reflux pendant 5 heures et 30 minutes. Après refroidissement à 10°C, le milieu réactionnel est filtré sur verre fritté. Le précipité est dissous dans 200 ml de chloroforme. La phase organique est lavée avec une solution d'ammoniaque à 3 % (3 x 40 ml) et séchée sur du chlorure de calcium. Le solide jaune obtenu après évaporation sous pression réduite du solvant est purifié sur colonne moyenne pression (support : silice 6-35 mm, éluant : toluène/dichlorométhane, 100/0, 50/50, 0/100) puis recristallisé 3 fois dans un mélange toluène/hep-tane, 50/50 pour fournir 0,302 g de 4,9-dihydro-4,9-dioxo-8-hydroxy-2-phényl-naphto[2,3-d]thiazole.
**Rdt :** 33 %
**F :** 263,5°C
**Rf :** 0,51 (heptane/acétate d'éthyle, 70/30)
**SM (I.E.) :** m/z 307 (M+.)
**RMN du** ^{**1**}**H (CDCl**_{**3**}**) : d (ppm)**
8,08 (dd, 2H, H-2', H-6', J_{H2'-H3'} = J_{H5'-H6'} = 8,4 Hz, J_{H2'-H4'} = J_{H4'-H6'} = 1,4 Hz)
7,81 (dd, 1H, H-5, J_{H5-H7} = 7,6 Hz, J_{H5-H6} = 1,4 Hz)
7,67 (t, 1H, H-7)
7,55 (m, 3H, H-3', H-4', H-5')
7,35 (dd, 1H, H-6, J_{H6-H7} = 8,4 Hz)
**RMN du** ^{**13**}**C (CDCl**_{**3**}**) : d (ppm)**
163,11 (1C, C-8)
136,57 (1C, C-6)
132,53 (1C, C-4')
129,32 (2C, C-3', C-5')
127,75 (2C, C-2', C-6')
125,62 (1C, C-7)
120,17 (1C, C-5)
**IR (KBr) : ν (cm**^{**-1**}**)**
1650 (C=O)

### Exemple 28

### 4,9-Dihydro-4,9-dioxo-2-(1-pyrrolyl)-naphto-[2,3]-thiazole

A une suspension de 2,30 g (0,01 mole) de 2-amino-4,9-dihydro-4,9-dioxo-naphto[2,3-d]thiazole dans 25 ml d'acide acétique, on ajoute à chaud 1,3 ml (0,01 mole) de 2,5-diméthoxy-tétrahydrofurane. Le mélange réactionnel est porté à reflux pendant 2 heures. Le précipité marron obtenu est filtré, dissous dans du dichlorométhane puis lavé trois fois avec 200 ml d'eau distillée. La phase organique est séchée sur du chlorure de calcium, filtrée et évaporée sous pression réduite pour fournir 1,80 g d'un solide jaune qui est purifié sur colonne flash (support : silice 6-35 mm ; conditionnement : heptane ; éluant : dichlorométhane/heptane, 90/10). On obtient 0,90 g de 4,9-dihydro-4,9-dioxo-2-(1-pyrrolyl)-naphto[2,3] -thiazole sous forme de cristaux jaunes après recristallisation et décoloration au noir animal.
**Rdt :** 64 %
**F :** > 260°C
**Rf :** 0,41 (CH₂Cl₂)
**SM (I.E.):** m/z 280 (M+.)
**RMN du** ^{**1**}**H (CDCl**_{**3**}**) : d (ppm)**
8,29, 8,20 (2dd, 1H, H-5, H-8, J_{H5-H6} = 8,85 Hz, J_{H5-H7} = 1,73 Hz)
7,79 (m, 2H, H-6, H-7)
7,48 (m, 2H, H-2', H-5')
6,42 (m, 2H, H-3', H-4')
**RMN du** ^{**13**}**C (CDCl**_{**3**}**) : d (ppm)**
177,89, 177,47 (2C, C-4, C-9)
165,60 (1C, C-2)
153,00 (1C, C-3a)
137,14 (1C, C-9a)
134,30, 134,06 (2C, C-6, C-7)
132,87, 132,38 (2C, C-4a, C-8a)
127,75, 126,69 (2C, C-5, C-8)
120,52 (2C, C-2', C-5')
114,23 (2C, C-3', C-4')
**IR (KBr) : ν (cm**^{**-1**}**)**
1680, 1665 (C=O)

### Exemples 29 et 30

### 2-(5-Bromofuran-2-yl)-4,9-dihydro-4,9-dioxo-naphto-[2,3-d]thiazole

### 2-(4,5-Dibromofuran-2-yl)-4,9-dihydro-4,9-dioxo-naphto[2,3-d]thiazole

10,0 g (35,6 mmoles, 1 éq) de 4,9-dihydro-4,9-dioxo-2-(furan-2-yl)naphto[2,3-d] thiazole sont solubilisés dans 750 ml de dichlorométhane préalablement séché sur tamis moléculaire. La solution est refroidie à 0°C, on additionne par petites fractions 11,2 g (81,9 mmoles, 2,3 éq) de chlorure d'aluminium.

Le mélange réactionnel est porté au reflux, on additionne goutte à goutte 8,0 ml (126,0 mmoles, 3,5 éq) de brome en solution dans 20 ml de dichlorométhane, la réaction est poursuivie pendant 5 heures. La solution est refroidie et versée doucement dans une solution saturée d'hydrogénocarbonate de sodium. La phase organique est lavée plusieurs fois avec de l'eau jusqu'à pH neutre, puis séchée sur chlorure de calcium.

10,5 g de produit brut solide (marron-orangé) sont obtenus après évaporation du solvant, puis purifiés sur colonne flash (support : silice 6-35 µm ; éluant : CH₂Cl₂/ Heptane : 50/50, CH₂Cl₂ : 80/20), pour fournir après évaporation 4,5 g de 2-(5-bromofuran-2-yl)-4,9-dihydro-4,9-dioxonaphto[2,3-d]-thiazole, sous forme de cristaux oranges et 380 mg de 2-(4,5-dibromofuran-2-yl)-4,9-dihydro-4,9-dioxonaphto[2,3-d]thiazole, sous forme de cristaux jaunes.

### 2-(5-Bromo-furan-2-yl)-4,9-dihydro-4,9-dioxonaphto-[2,3-d]thiazole

**Rdt :** 42,8 %
**F : >** 260°C
**Rf :** 0,47 (CH₂Cl₂)
**SM (I.E) :** m/z 359-361 (M⁺)
**RMN du** ^{**1**}**H (CDCl**_{**3**}**) : d (ppm)**
8,34 (m, 1H, H-5 ou H-8)
8,23 (m, 1H, H-5 ou H-8)
7,81 (m, 2H, H-6, H-7)
7,40 (d, 1H, H-3', J_{H3'-H4'} = 3,74 Hz)
6,59 (d, 1H, H-4', J_{H3'-H4'} = 3,67 Hz)
**RMN du** ^{**13**}**C (CDCl**_{**3**}**) : d (ppm)**
178,28, 177,99 (2C, C-4, C-9)
162,67 (C-2)
162,06 (C-2')
155,35 (C-3a)
149,88 (C-9a)
140,88 (C-5')
134,58, 134,27 (2C, C-6, C-7)
133,30, 132,83 (2C, C-4a, C-8a)
127,24, 127,13 (2C, C-5, C-8)
115,23, 115,50 (2C, C-3', C-4')
**IR (KBr) : ν (cm**^{**-1**}**)**
1682 et 1656 (C=O)

### 2-(4,5-Dibromofuran-2-yl)-4,9-dihydro-4,9-dioxo-naphto[2,3-d]thiazole

**Rdt :** 2,4 %
**F :** > 260°C
**Rf :** 0,63 (CH₂Cl₂)
**SM (APcI-) :** m/z 438 (M-H)
**RMN du** ^{**1**}**H (CDCl**_{**3**}**) : d (ppm)**
8,35 (m, 1H, H-5 ou H-8)
8,24 (m, 1H, H-5 ou H-8)
7,82 (m, 2H, H-6, H-7)
7,46 (s, 1H, H-3')
**RMN du** ^{**13**}**C (CDCl**_{**3**}**) : δ (ppm)**
134,54, 134,25 (2C, C-6, C-7)
127,92, 127,03 (2C, C-5, C-8)
117,59 (1C, C-3')
**IR (KBr) : ν (cm**^{**-1**}**)**
1685 et 1655 (C=O)

### Exemple 31

### 2-(3-Bromo-furan-2-yl)-4,9-dihydro-4,9-dioxonaphto-[2,3-d]thiazole

A 150 ml d'une solution d'hydroxyde de sodium (pH = 10,7), on ajoute 5,33 g (22,2 mmoles, 1 éq.) de sulfure de sodium nonahydraté. La solution est portée à 90°C et est agitée sous atmosphère d'argon. On additionne 4,61 g (22,2 mmoles, 1 éq.) de 2-amino-3-chloro-1,4-dihydro-1,4-dioxonaphtalène, on agite la solution jusqu'à obtention d'une coloration bleue. La solution est refroidie entre 20 et 25 °C et on ajoute au milieu réactionnel 3,89 g (22,2 mmoles) de 3-bromo-2-furaldéhyde (N°CAS 14757-78-9). Après cinq minutés on remplace le bullage d'argon par de l'air comprimé pendant 1 heure, on additionne ensuite goutte à goutte 5 ml d'acide acétique, le milieu devient marron-rouge.

L'agitation est maintenue pendant cinq minutes, le précipité noir formé est filtré sur verre fritté, lavé avec de l'eau et séché pour donner 9,30 g de produit qui sont purifiés plusieurs fois sur colonne flash (support : silice 6-35 µm ; Ø 4,5 cm, h 30 cm éluant : Dichlorométhane/Heptane : 50/50).

Après évaporation du solvant, on obtient 2,15 g de 2-(3-bromo-2-furan-2-yl)-4,9-dihydro-4,9-dioxonaphto-[2,3-d]thiazole sous forme de cristaux orange.
**Rdt :** 26,9 %
**F : >** 250°C
**Rf :** 0,30 (CH₂Cl₂)
**SM (APcI+) :** m/z 361 (M+H⁺)
**RMN du** ^{**1**}**H(CDCl**_{**3**}**) : δ (ppm)**
8,36 (m, 1H, H-5 ou H-8)
8,25 (m, 1H, H-5 ou H-8)
7,82 (m, 2H, H-6, H-7)
7,65 (d, 1H, H-5', J_{H4'-H5'} = 2,14 Hz)
6,75 (d, 1H, H-4', J_{H4'-H5'} = 2,14 Hz)
**RMN du** ^{**13**}**C (DMSO-d**_{**6**}**) :** δ **(ppm)**
147,95 (1C, C-5')
134,82, 134,42 (2C, C-6, C-7)
127,24, 126,44 (2C, C-5, C-8)
117,50 (1C, C-4')
**IR (KBr) : ν (cm**^{**-1**}**)**
1680 et 1655 (C=O)

### Exemple 32

### 2-(4-Bromofuran-2-yl)-4,9-dihydro-4,9-dioxonaphto-[2,3-d]thiazole

A 0,257 g (1,24 mmole, 1 éq) de 2-amino-3-chloro-1,4-dihydro-1,4-dioxonaphtalène dans 9 ml d'eau, on ajoute 1,19 g (4,96 mmoles, 4 éq) de sulfure de sodium nonahydrate. Le mélange est porté au reflux jusqu'à ce que la coloration du milieu réactionnel soit devenue complètement bleue. 0,26 g (1,48 mmole, 1,19 éq) de 4-bromo-2-furaldéhyde sont alors ajoutés à 90°C. Le milieu réactionnel est alors refroidi à température ambiante avant l'ajout de 0,28 ml d'acide acétique. Il y a alors formation d'un précipité. Le milieu réactionnel est agité une heure à température ambiante. Le précipité est ensuite filtré, lavé à l'eau. Le précipité de couleur marron (0,35 g) est purifié sur colonne flash (support : alumine ; conditionnement : dichlorométhane/heptane 70/30 ; éluant : dichlorométhane/heptane 70/30 puis 80/20 puis 100/00 puis dichlorométhane/méthanol 99,6/0,4) pour fournir 0,145 g de 2-(4-bromofuran-2-yl)-4,9-dihydro-4,9-dioxonaphto[2,3-d]thiazole sous forme de cristaux oranges.
**Rdt :** 32 %
**F :** > 260°C
**Rf :** 0,23 (dichlorométhane/heptane, proportion 70/30) sur support en alumine
**SM (IE) :** m/z 360 (M⁺)
**RMN du** ^{**1**}**H (CDCl**_{**3**}**) :** δ **(ppm)**
8,34 (m, 1H, H-5 ou H-8)
8,25 (m, 1H, H-8 ou H-5)
7,83 (m, 2H, H-6, H-7)
7,64 (d, 1H, H-5', J_{H3'-H5'} = 0,95 Hz)
7,46 (d, 1H, H-3', J_{H3'-H5'} = 0,95 Hz)
**RMN du** ^{**13**}**C (CDCl**_{**3**}**) : δ (ppm)**
143,76 (1C, C-5')
134,48, 134,17 (2C, C-6, C-7)
127,89, 126,99 (2C, C-5, C-8)
116,07 (1C, C-3')
**IR (KBr) : ν (cm**^{**-1**}**)**
1680, 1657 (C=O)

### Exemple 33

### 4,9-Dihydro-4,9-dioxo-2-(5-nitro-furan-2-yl)naphto-[2,3-d]thiazole

A 5 g (17,8 mmoles) de 4,9-dihydro-4,9-dioxo-2-(furan-2-yl)naphto[2,3-d]thiazole on ajoute à température ambiante 20 ml d'acide nitrique fumant et 20 ml d'acide sulfurique concentré. Le mélange réactionnel est porté au reflux pendant 72 heures et le précipité formé est filtré sur verre fritté, lavé à l'eau et rincé à l'éther. La poudre jaune foncé obtenue est recristallisée dans le DMF après décoloration au noir animal. On obtient ainsi 2 g de 4,9-dihydro-4,9-dioxo-2-(5-nitro-furan-2-yl)naphto-[2,3-d]thiazole sous forme d'un solide jaune foncé.
**Rdt :** 34 %
**F :** > 300°C
**Rf :** 0,30 (CH₂Cl₂)
**S.M. (APcI-)** : m/z 326 (M-)
**RMN du** ^{**1**}**H (CDCl**_{**3**}**) : δ (ppm)**
8,39 (m, 1H, H-5 ou H-8)
8,27 (m, 1H, H-5 ou H-8)
7,85 (m, 2H, H-6, H-7)
7,59 (d, 1H, H-4', J_{H3'-H4'} = 3,73 Hz)
7,50 (d, 1H, H-3', J_{H3'-H4'} = 3,74 Hz)
**IR (KBr) : ν (cm**^{**-1**}**)**
1675, 1656 (C=O)

### Exemple 34

### 2-(5-Amino-furan-2-yl)-4,9-dihydro-4,9-dioxonaphto-[2,3-d]thiazole

Dans un tricol, 2 g (6,10 mmoles, 1 éq) de 4,9-dihydro-4,9-dioxo-2-(5-nitrofuran-2-yl)-naphto[2,3-d]-thiazole sont solubilisés dans 1500 ml d'éthanol absolu. Le montage est mis sous atmosphère inerte, on ajoute une pointe de spatule de charbon palladié à 30 %. La solution est portée au reflux et sont additionnés en cinq fois 360 µl (7,36 mmoles ; 1,2 éq) d'hydrazine. La réaction est poursuivie pendant une heure, la solution passe du jaune-vert au noir-violet. La solution est refroidie et filtrée sur célite. On obtient 1,65 g d'un solide noir après évaporation du solvant au rotavapeur. Ce solide est purifié sur colonne flash (support : silice 6-35 µm, gradient d'éluant : dichlorométhane puis dichlorométhane/ méthanol, 98/2), pour fournir 0,36 g de 2-(5-amino-furan-2-yl)-4,9-dihydro-4,9-dioxo-naphto[2,3-d]thiazole sous forme de cristaux bleus.
**Rdt :** 20 %
**F :** > 260°C
**Rf :** 0,29 (Dichlorométhane/Acétate d'éthyle, 99/1)
**SM (APcI+) :** m/z 297 (M+H⁺)
**RMN du** ^{**1**}**H (DMSO-d**_{**6**}**) : δ (ppm)**
8,24 (m, 1H, H-5 ou H-8)
8,18 (m, 1H, H-5 ou H-8)
7,99 (m, 2H, H-6, H-7)
7,60 (d, 1H, H-3', J_{H3'-H4'} = 3,97 Hz)
7,36 (s, 2H, NH₂)
5,47 (d, 1H, H-4', J_{H3'-H4'} = 3,97 Hz)
**RMN du** ^{**13**}**C (DMSO-d**_{**6**}**) :** δ **(ppm)**
177,8, 177,2 (2C, C-4, C-9)
161,0 (1C, C-5')
155,0 (1C, C-2)
138,0 (1C, C-2')
134,3, 134,1 (2C, C-6, C-7)
133,1, 132,4 (2C, C-4a, C-8a)
127,0, 126,0 (2C, C-5, C-8)
122,1 (1C, C-3')
87,5 (1C, C-4')
**IR (KBr) : ν (cm**^{**-1**}**)**
3350 (NH₂) ; 1680 et 1625 (C=O)

### Exemple 35

### 2-(5-Acétamidofuran-2-yl)-4,9-dihydro-4,9-dioxo-naphto[2,3-d]thiazole

Dans un tricol, à 0,300 g (1,01 mmole, 1 éq) de 2-(5-aminofuran-2-yl)-4,9-dihydro-4,9-dioxo-naphto-[2,3-d]-thiazole, on ajoute, goutte à goutte et à température ambiante 200 µl (2,02 mmoles ; 2 éq) d'anhydride acétique puis 60 µl (1,01 mmole ; 1 éq) d'acide acétique. La suspension obtenue est chauffée à 50°C et devient rouge-bordeaux. Après 2 heures de réaction, la suspension est refroidie et solubilisée dans 500 ml de dichlorométhane. La solution est lavée deux fois avec une solution saturée d'hydrogénocarbonate de sodium, puis plusieurs fois à l'eau jusqu'à obtention d'un pH neutre de la phase aqueuse. Après séchage de la phase organique sur du chlorure de calcium et évaporation des solvants, on obtient 0,330 g de produit brut solide (rouge-bordeaux). Ce solide est purifié sur colonne flash (support : silice 6-35 µm, éluant : Dichlorométhane/Méthanol, 98/2). Le composé obtenu est solubilisé dans du dichlorométhane, filtré sur micropores. Le filtrat est concentré et le précipité formé est filtré sur fritté pour fournir 0,145 g de 2-(5-acétamidofuran-2-yl)-4,9-dihydro-4,9-dioxonaphto[2,3-d]thiazole sous forme de cristaux rouge-brique.
**Rdt :** 42 %
**F :** > 260°C
**Rf :** 0,34 (Dichlorométhane/Méthanol, 95/5)
**SM (APcI+) :** m/z 339 (M+H⁺)
**RMN du** ^{**1**}**H (DMSO-d**_{**6**}**) : δ (ppm)**
11,75 (s, 1H, NH)
8,28 (m, 1H, H-5 ou H-8)
8,23 (m, 1H, H-5 ou H-8)
8,03 (m, 2H, H-6, H-7)
7,69 (d, 1H, H-3', J_{H3'-H4'} = 3,73 Hz)
6,62 (d, 1H, H-4', J_{H3'-H4'} = 3,74 Hz)
2,22 (s, 3H, CH₃)
**RMN du** ^{**13**}**C (DMSO-d**_{**6**}**) : δ (ppm)**
177,9, 176,5 (2C, C-4, C-9)
167,5 (1C, C-5')
155,2 (1C, C-2)
139,0 (1C, C-2')
134,6, 134,4 (2C, C-6, C-7)
132,7, 132,6 (2C, C-4a, C-8a)
127,2, 126,4 (2C, C-5, C-8)
118,1 (1C, C-3')
97,0 (1C, C-4')
23,4 (1C, CH₃)
**IR (KBr) : ν (cm**^{**-1**}**)**
3033 (N-H) ; 1682 et 1655 (C=O)

### Exemple 36

### 4,9-dihydro-4,9-dioxo-2-(5-hydroxyméthylfuran-2-yl) naphto[2,3-d]thiazole

17,36 g (72,2 mmoles, 5 éq) de sulfure de sodium nonahydraté sont solubilisés dans 70 ml d'eau. La solution est chauffée à 60°C, puis on additionne 3,00 g (14,4 mmoles, 1 éq) de 2-amino-3-chloro-1,4-dihydro-1,4-dioxo-naphtalène. Après 30 minutes d'agitation à 60°C, la solution est refroidie à température ambiante. On ajoute au milieu réactionnel devenu bleu 2,43 g (14,5 mmoles, 1 éq) de 5-acétoxyméthyl-2-furaldéhyde, puis après 5 minutes, on additionne goutte à goutte 3 ml d'acide acétique. Le milieu devient marron-orangé et le précipité formé est filtré sur verre fritté, lavé avec de l'eau et séché pour fournir 3,30 g de produit brut qui sont purifiés sur colonne flash (support : silice 6-35 *µ*m ; ⌀ 5 cm, h 15 cm, éluant : CH₂Cl₂/MeOH, 96/4) . Le produit orange obtenu est recristallisé dans le diméthylformamide, décoloré sur noir animal et filtré sur célite et micropores pour fournir 0,80 g de 4,9-dihydro-4,9-dioxo-2-(5-hydroxyméthylfuran-2-yl)naphto[2,3-d]thiazole sous forme de cristaux ocres.
**Rdt :** 17 %
**F : >** 260°C
**Rf :** 0,60 (CH₂Cl₂/MeOH, 96/4)
**SM (I.E)** : m/z 311 (M⁺)
**RMN du** ^{**1**}**H (DMSO-d**_{**6**}**) : δ (ppm)**
8,20 (m, 1H, H-5 ou H-8)
8,11 (m, 1H, H-5 ou H-8)
7,91 (m, 2H, H-6, H-7)
7,46 (d, 1H, H-3', J_{H3'-H4'} = 3,1 Hz)
6,65 (d, 1H, H-4', J_{H3'-H4'} = 3,1 Hz)
5,55 (t, 1H, OH, J_{O}_{H}_{-CH2} = 5,6 Hz)
4,54 (d, 2H, CH₂, J_{C}_{H}_{2-OH} = 5,6 Hz)
**RMN du** ^{**13**}**C (DMSO-d**_{**6**}**) : δ (ppm)**
177,6, 176,6 (2C, C-4, C-9)
160,0 (1C, C-5')
158,4 (1C, C-2)
146,6 (1C, C-2')
134,4 (2C, C-6, C-7)
132,3, 132,1 (2C, C-4a, C-8a)
127,0, 126,2 (2C, C-5, C-8)
114,9 (1C, C-3')
110,5 (1C, C-4')
55,6 (1C, CH₂)
**IR (KBr) : ν (cm**^{**-1**}**)**
3374 (OH), 1677 et 1656 (C=O)

### Exemple 37

### 2-(5-Acétoxyméthylfuran-2-yl)-4,9-dihydro-4,9-dioxo-naphto [2,3-d]thiazole

A 5,00 g (24 mmoles) de 2-amino-3-mercapto-1,4-dihydro-1,4-dioxonaphtalène, on ajoute à 0°C sous atmosphère d'argon 40 ml de N-méthylpyrrolidone. Le mélange réactionnel est agité pendant 10 minutes puis on additionne à 0°C, 4,10 g (24 mmoles) de 5-acétoxyméthyl-2-furaldéhyde. Après 5 heures d'agitation à cette température, on laisse revenir le mélange à température ambiante. Le contenu du tricol est versé dans 250 ml d'eau et le précipité marron formé est dissous dans l'acétate d'éthyle. La phase organique est extraite, séchée sur du sulfate de magnésium, filtrée et évaporée sous pression réduite.

Le solide marron obtenu est purifié une première fois par colonne (support : silice 6-35 µm ; éluant : CH₂Cl₂/MeOH/AcOEt, 97/1/2) pour donner 3,29 g de produit.

Un échantillon de 0,500 g est prélevé puis purifié une seconde fois par plaques préparatives (support : silice; éluant : CH₂Cl₂/MeOH/AcOEt, 97/1/2), pour fournir 0,107 mg de 2-(5-acétoxyméthylfuran-2-yl)-4,9-dihydro-4,9-dioxo-naphto[2,3-d]thiazole sous forme de cristaux jaunes.
**Rdt :** 38 %
**F :** 204°C
**Rf :** 0,52 (Heptane/AcOEt, 50/50)
**SM (I.E.) :** m/z 353 (M⁺)
**RMN du** ^{**1**}**H (CDCl**_{**3**}**) : δ (ppm)**
8,34 (m, 1H. H-5 ou H-8)
8,24 (m, 1H, H-5 ou H-8)
7,81 (m, 2H, H-6, H-7)
7,41 (d, 1H, H-3', J_{H3'-H4'} = 3,74 Hz)
6,64 (d, 1H, H-4', J_{H4'-H3'} = 3,32 Hz)
5,15 (S, 2H, CH₂)
2,14 (s, 3H, CH₃)
**RMN du** ^{**13**}**C (CDCl**_{**3**}**) : δ (ppm)**
134,39, 134,14 (2C, C-6, C-7)
133,30, 132,83 (2C, C-4a, C-8a)
127,86, 126,94 (2C, C-5, C-8)
114,52, 113,77 (2C, C-3',C-4')
57,67 (1C, CH₂)
20,83 (1C, CH₃)
**IR (KBr) : ν (cm**^{**-1**}**)**
1734,1686 et 1669 (C=O)

### Exemple 38

### 4,9-Dihydro-4,9-dioxo-2-(5-méthyl-2-furyl)naphto-[2,3-d]thiazole

A une solution tampon pH 11( contenant 6,2 g de H₃BO₃ et 4 g de NaOH par litre), on ajoute 4,6 g (19,3 mmoles) de sulfure de sodium nonahydraté. Le mélange est agité à 15°C sous atmosphère d'argon jusqu'à dissolution complète, puis on additionne 2 g (9,6 mmoles) de 2-amino-3-chloro-1,4-dihydro-1,4-dioxonaphtalène. Après 20 minutes, on ajoute au milieu réactionnel devenu bleu 0,96 ml (9,6 mmoles) de 5-méthyl-2-furfural.

Après 4 heures d'agitation le contenu du tricol est versé dans 100 ml d'acétate d'éthyle et le tricol est rincé avec 50 ml d'eau. La phase organique est lavée trois fois avec 80 ml d'eau et séchée sur du sulfate de magnésium. On obtient après évaporation du solvant sous pression réduite 1 g de produit orange qui est purifié sur cake (support : silice 6-35 *µ*m ; éluant : CH₂Cl₂/Heptane : 50/50, 90/10 et 100) pour fournir 0,560 g de cristaux orange qui sont recristallisés dans un mélange AcOEt/CH₂Cl₂ : 70/30 après décoloration au noir animal.
**Rdt :** 35 %
**F :** 254°C
**Rf :** 0,48 (CH₂Cl₂)
**SM (I.E) :** m/z 295 (M⁺)
**RMN du** ^{**1**}**H (CD**_{**2**}**Cl**_{**2**}**) : δ (ppm)**
8,22(dd, 1H, H-5 ou H-8, J_{H5-H6} ou J_{H7-H8} = 8,85 Hz, J_{H5-H7} ou J_{H6-H8} = 1,73 Hz)
8,16(m, 1H, H-5 ou H-8)
7,80(m, 2H, H-6, H-7)
7,28(d, 1H, H-3', J_{H3'-H4'} = 3,35 Hz)
6,29(d, 1H, H-4', J_{H3'-H4'} = 3,35 Hz)
2,44(s, 3H, CH₃)
**RMN du** ^{**13**}**C (CDCl**_{**3**}**) : δ (ppm)**
178,43, 178,12(C-4, C-9)
164,07(C-2)
157,79(C-5')
155,00(C-3a)
146,92(C-2')
140,60(C-9a)
134,54, 134,32(C-6, C-7)
133,50, 133,04(C-4a, C-8a)
127, 72, 126,94(C-5, C-8)
115,43, 110,16(C-3', C-4')
14,09(CH₃)
**IR (KBr) : ν (cm**^{**-1**}**)**
1684 et 1653 (C=O)

### Exemple 39

### 4,9-Dihydro-2-(4,5-diméthyl-2-furyl)4,9-dioxonaphto-[2,3-d]thiazole

A 200 ml d'une solution d'hydroxyde de sodium (pH = 10,66), on ajoute 6,94 g (28,9 mmoles) de sulfure de sodium nonahydraté. Le mélange est agité à 15°C sous atmosphère d'argon jusqu'à dissolution complète, puis on additionne 3,00 g (14,4 mmoles) de 2-amino-3-chloro-1,4-dihydro-1,4-dioxonaphtalène. Après 3 heures d'agitation à température ambiante, on ajoute au milieu réactionnel devenu bleu 1,80 g (14,4 mmoles) de 4,5-diméthyl-2-furaldéhyde, puis après cinq minutes, on additionne goutte à goutte 5 ml d'acide acétique, le milieu devient orangé.

L'agitation est maintenue pendant cinq minutes, puis on remplace le bullage d'argon par de l'air comprimé pendant trois minutes.

Le précipité noir formé est filtré sur verre fritté, lavé avec de l'eau et séché pour donner 4,00 g de produit qui sont purifiés sur colonne flash (support : silice 6-35 *µ*m ; éluant : Heptane/AcOEt : 85/15).

Le produit orange obtenu après évaporation du solvant, est décoloré au noir animal, puis recristallisé dans l'acétate d'éthyle pour fournir 2,50 g de 4,9-dihydro-2-(4,5-diméthyl-2-furyl)4,9-dioxonaphto[2,3-d]-thiazole sous forme de cristaux orange.
**Rdt :** 90 %
**F :** 250°C
**Rf :** 0,2 (CH₂Cl₂)
**SM (I.E) :** m/z 309 (M⁺)
**RMN du** ^{**1**}**H(CDCl**_{**3**}**) :** δ **(ppm)**
8,31 (dd, 1H, H-5 ou H-8, J_{H5-H6} ou J_{H5-H7} = 8,85 Hz, J_{H5-H7} ou J_{H6-H8} = 1,73 Hz)
8,22 (dd, 1H, H-5 ou H-8, J_{H5-H6} ou J_{H5-H7} = 8,85 Hz, J_{H5-H7} ou J_{H6-H8} = 1,73 Hz)
7,80 (m, 2H, H-6, H-7)
7,23 (s, 1H, H-3')
2,35 (s, 3H, CH₃ en 5')
2,17 (s, 3H, CH₃ en 4')
**RMN du** ^{**13**}**C (CDCl**_{**3**}**) : δ (ppm)**
178,43, 178,12 (2C, C-4, C-9)
172 (C-2)
164,21 (C-2')
155,35 (C-3a)
153,18 (C-5')
145,32 (C-9a)
134,13, 133,97 (2C, C-6, C-7)
133,24, 132,89 (2C, C-4a, C-8a)
127,72, 126,79 (2C, C-5, C-8)
118,78 (C-4')
117,51 (C-3')
12,50 (CH₃ en 5')
10,00 (CH₃ en 4')
**IR (KBr) : ν (cm**^{**-1**}**)**
1682 et 1656 (C=O)

### Exemple 40

### 4,9-Dihydro-4,9-dioxo-2-(5-phényl-2-oxazolyl)-naphto[2,3-d]thiazole

Au 2-amino-3-chloro-1,4-dihydro-1,4-dioxonaphtalène (0,21 g, 1,0 mmole) dans de l'eau distillée (7 ml) est ajouté sous atmosphère d'argon du sulfure de sodium nonahydraté (0,98 g, 4,1 mmoles). Le mélange est porté au reflux sous atmosphère d'argon jusqu'à ce que la coloration du milieu réactionnel soit devenue complètement bleue. Le 5-phényl-2-oxazolecarbaldéhyde (N° CAS 96829-89-9) (0,21 g, 1,4 mmole) en solution dans du tétrahydrofuranne (6 ml) est alors ajouté ainsi que de l'acide acétique (0,25 ml). Le milieu réactionnel est alors agité sous atmosphère d'argon à température ambiante pendant une heure. Il y a alors formation d'un précipité orange. Le précipité est alors filtré, lavé à l'eau. Le précipité orange (0,28 g) est alors solubilisé partiellement dans du diéthyléther (21 ml). Après filtration des insolubles, le filtrat est concentré sous vide et chromatographié sur colonne flash (support : silice 6-35 *µ*m; éluant : dichlorométhane/méthanol : 98/2). Le produit orange obtenu après évaporation est décoloré au noir animal puis filtré sur micropore. Des cristaux orange (0,17 g) de 4,9-dihydro-4,9-dioxo-2-(5-phényl-2-oxazolyl)naphto[2,3-d]thiazole sont ainsi obtenus après évaporation.
**Rdt :** 79 %
**F** : > 260°C
**Rf :** 0,64 (acétonitrile en phase inverse)
0,76 (dichlorométhane/méthanol, 98/2)
**SM (I.E) :** m/z 358 (M⁺), 330 (M⁺ - CO)
**RMN du** ^{**1**}**H (CDCl**_{**3**}**) :** δ **(ppm)**
8,36 (m, 1H, H-5 ou H-8)
8,26 (m, 1H, H-5 ou H-8)
7,85 (m, 4H, H-6, H-7, H-2", H-6")
7,60 (d, 1H, H-4', J = 0,9 Hz)
7,55 - 7,35 (m, 2H, H-3", H-5")
7,27 (d, 1H, H-4", J = 0,9 Hz)
**RMN du** ^{**13**}**C (CDCl**_{**3**}**) : δ (ppm)**
178,23, 177,36 (2C, C-4 et C-9)
160,34 (1C, C-2)
155,20 (1C, C-2')
154,60 (1C, C-3a)
154,37 (1C, C-5')
142,98 (1C, C-9a)
134,71, 134,27 (2C, C-6 et C-7)
133,04, 132,77 (2C, C-4a et C-8a)
129,92 (1C, C-8 ou C-5)
129,15 (2C, C-2" et C-6")
128,02 (1C, C-5 ou C-8)
127,16 (1C, C-4')
126,51 (1C, C-1'')
125,22 (2C, C-3" et C-5")
124,66 (1C, C-4")
**IR (KBr) :** ν **cm**^{**-1**}
1681 et 1654 (C=O), 1589 (N = C-O)

### Exemple 41

### 4,9-Dihydro-4,9-dioxo-2-(2-thiazolyl)naphto[2,3-d]-thiazole

A 1,12 g (5,39 mmoles) de 2-amino-3-chloro-1,4-dihydro-1,4-dioxonaphtalène dans 38 ml d'eau, on ajoute 5,18 g (21,56 mmoles) de sulfure de sodium nonahydraté. Le mélange est porté au reflux jusqu'à ce que la coloration du milieu réactionnel soit devenue complètement bleue. 0,73 g (6,46 mmoles) de 2-thiazolecarboxaldéhyde puis 1,3 ml (22,75 mmoles) d'acide acétique sont alors ajoutés à 90°C. Le milieu réactionnel est alors refroidi immédiatement à 0°C par un bain de glace. Il y a alors formation d'un précipité. Le milieu réactionnel est agité une heure à 0°C. Le précipité est ensuite filtré, lavé à l'eau. Le précipité est alors solubilisé partiellement dans du dichlorométhane. Lors de la filtration et de la solubilisation dans le dichlorométhane, un changement de coloration survient : le précipité de couleur marron prend une couleur jaune-brun. Après filtration des insolubles et évaporation du solvant, 0,3 g de 4,9-dihydro-4,9-dioxo-2-(2-thiazolyl)naphto[2,3-d]thiazole est alors obtenu sous forme de cristaux jaune-brun.
**Rat :** 19 %
**F :** > 260°C
**Rf :** 0,22 (dichlorométhane)
**SM (APcI-) :** m/z 298 (M-)
**RMN du** ^{**1**}**H** (**CDCl**_{**3**}) : δ **(ppm**)
8,36 (m, 1H, H-5 ou H-8)
8,26 (m, 1H, H-8 ou H-5)
8,03 (d, 1H, H-4', J_{H4'-H5'} = 3,06 Hz)
7,84 (m, 2H, H-6, H-7)
7,67 (d, 1H, H-5', J_{H4'-H5'} = 3,05 Hz)
**RMN du** ^{**13**}**C (CDCl**_{**3**}**) : δ (ppm)**
145,18 (1C, C-4')
134,91, 134,53 (2C, C-6, C-7)
128,24, 127,41(2C, C-8, C-5)
124,59 (1C, C-5')
**IR (KBr) : ν (cm**^{**-1**}**)**
1675, 1652 (C=O)

### Exemples 42 et 43

### 4,9-Dihydro-4,9-dioxo-6-fluoro-2-(2-furyl)-naphto[2,3-d]thiazole et

### 4,9-Dihydro-4,9-dioxo-7-fluoro-2-(2-furyl)-naphto-[2,3-d]thiazole

### Intermédiaires de synthèse :

### 2,3-dibromo-1,4-dihydro-1,4-dioxo-6-fluoronaphtalène

A une solution de 1,4-dihydro-1,4-dioxo-6-fluoro-naphtalène (N°CAS 148541-61-1) (12,5 g, 71 mmoles) dans le chloroforme (250 ml), on ajoute 36 ml (710 mmoles) de brome. La solution est portée au reflux pendant 12 heures puis ramenée à température ambiante. Après un barbotage d'air comprimé, la solution est concentrée sous pression réduite et le solide obtenu est lavé cinq fois à l'heptane. On obtient 15,0 g d'une poudre beige de 2,3-dibromo-1,4-dihydro-1,4-dioxo-6-fluoronaphtalène.
**Rdt :** 65 %
**F :** 158°C
**Rf :** 0,80 (dichlorométhane)
**SM (APcI-) :** m/z 332, 334, 336 (M⁻)
**RMN du** ^{**1**}**H (CDCl**_{**3**}**) : δ (ppm)**
8,22 (dd, 1H, H-8, J_{H7-H8} = 8,55 Hz, J_{H-F} = 5,19 Hz)
7,81 (dd, 1H, H-5, J_{H-F} = 8,55 Hz, J_{H5-H7} = 2,75 Hz)
7,45 (td, 1H, H-7, J_{H-F} = J_{H7-H8} = 8,55 Hz, J_{H5-H7} = 2,75 Hz)
**IR (KBr) : ν (cm**^{**-1**}**)**
1680 (C=O)

### 2-amino-3-bromo-1,4-dihydro-1,4-dioxo-6-fluoro-naphtalène et

### 2-amino-3-bromo-1,4-dihydro-1,4-dioxo-7-fluoro-naphtalène

Dans une solution de 6-fluoro-2,3-dibromo-1,4-dihydro-1,4-dioxonaphtalène (10,00 g, 30 mmoles) dans le tétrahydrofuranne (500 ml), on fait barboter de l'ammoniac à température ambiante pendant 2 heures, puis de l'air comprimé est passé à travers la solution pendant 15 minutes. Après évaporation du solvant sous pression réduite, le solide obtenu est d'abord nettoyé sur cake de gel de silice (support : silice 6-35 *µ*m ; ø 5 cm ; h = 15 cm ; éluant : dichlorométhane/heptane, 90/10) puis purifié par trois chromatographies flash successives sur colonne de gel de silice (support : silice 6-35 *µ*m ; ø 5 cm ; h 30 cm ; éluant : dichlorométhane/heptane, 90/10). On obtient 4,78 g d'une poudre rouge, mélange de 2-amino-3-bromo-6-fluoro-1,4-dihydro-1,4-dioxonaphtalène et 2-amino-3-bromo-7-fluoro-1,4-dihydro-1,4-dioxonaphtalène.
**Rdt :** 60 % (rapport des isomères : 75/25)
**F :** 190-195°C
**Rf :** 0,40 (dichlorométhane)
**SM (APcI-) :** m/z 270 (M-)
**RMN du** ^{**1**}**H (acétone-d**_{**6**}**) : δ (ppm)**
8,27 (dd, 1H, H-5 ou H-8, J_{H5-H6} ou J_{H7-H8} = 8,54 Hz, J_{H-F} = 5,18 Hz)
7,85 (dd, 1H, H-5 ou H-8, J_{H-F} = 8,54 Hz, J_{H5-H7} ou J_{H6-H8} = 2,74 Hz)
7,71 (td, 1H, H-6 ou H-7 isomère minoritaire, J_{H-F} = J_{H5-H6} ou J_{H7-H8} = 8,54 Hz, J_{H5-H7} ou J_{H6-H8} = 2,74 Hz)
7,64 (td, 1H, H-6 ou H-7 isomère majoritaire, J_{H-F} = J_{H5-H6} ou J_{H7-H8} = 8,54 Hz, J_{H5-H7} ou J_{H6-H8} = 2,74 Hz)
**IR (KBr) : ν (cm**^{**-1**}**)**
3357 (NH₂), 1685 (C=O)

### 4,9-Dihydro-4,9-dioxo-6-fluoro-2-(2-furyl)naphto-[2,3-d]thiazole et

### 4,9-Dihydro-4,9-dioxo-7-fluoro-2-(2-furyl)naphto-[2,3-d]thiazole

A 0,50 g (1,8 mmole ; 1,0 éq) d'un mélange 75/25 de 2-amino-3-bromo-1,4-dihydro-1,4-dioxo-6-fluoronaphtalène et 2-amino-3-bromo-1,4-dihydro-1,4-dioxo-7-fluoronaphtalène, on ajoute 25 ml d'une solution d'hydroxyde de sodium (5.10⁻⁴ M) contenant 1,11 g (4,6 mmoles ; 2,5 éq) de sulfure de sodium nonahydraté. La suspension rouge est chauffée 30 minutes à 80°C jusqu'à obtention d'une solution bleu foncé. On additionne alors 0,3 ml (3,6 mmoles ; 2,0 éq) de 2-furaldéhyde. Après 90 minutes de chauffage à 80 °C, la solution rouge foncé obtenue est ramenée à température ambiante. Quelques gouttes d'acide acétique glacial sont ajoutées puis le précipité orange formé est filtré, lavé trois fois à l'eau et séché. On obtient 0,5 g d'un mélange des deux isomères de 4,9-dihydro-4,9-dioxo-6-fluoro-2-(2-furyl)naphto[2,3-d]thiazole et 4,9-dihydro-4,9-dioxo-7-fluoro-2-(2-furyl)naphto[2,3-d]-thiazole sous forme de cristaux rouges.

Les isomères sont séparés par chromatographie flash sur colonne de gel de silice (silice 6-35 *µ*m ; ⌀ 4,5 cm ; h 30 cm ; éluant : dichlorométhane), puis par HPLC préparative (colonne : Dynamax 60-A Si 83-141C ; éluant : heptane/acétate d'éthyle : 80/20) pour fournir 0,125 g du produit le moins polaire et 0,375g du produit le plus polaire.
**Rdt :** 90 % (rapport des isomères : 75/25)

### Produit le moins polaire

**F :** > 265°C
**Rf :** 0,32 (heptane/acétate d'éthyle, 80/20)
**SM (APcI-) :** m/z 299 (M-)
**RMN du** ^{**1**}**H (CD**_{**2**}**Cl**_{**2**}**) : δ (ppm)**
8,17 (dd, 1H, H-5 ou H-8, J_{H5-H6} ou J_{H7-H8} = 8,55 Hz, J_{H-F} = 5,50 Hz)
7,85 (dd, 1H, H-5 ou H-8, J_{H-F} = 8,55 Hz, J_{H5-H7} ou J_{H6-H8} = 2,44 Hz)
7,61 (m, 1H, H-5')
7,39 (td, 1H, H-6 ou H-7, J_{H5-H6} ou J_{H7-H8} = J_{H-F} = 8,55 Hz, J_{H5-} _{H7} ou J_{H6-H8} = 2,44 Hz)
7,33 (d, 1H, H-3', J_{H3'-H4'} = 3,66 Hz)
6,61 (dd,1H, H-4', J_{H3'-H4'} = 3,66 Hz, J_{H4'-H5'} = 1,83 Hz)
**IR (KBr) : ν (cm**^{**-1**}**)**
1680 et 1655 (C=O).

### Produit le plus polaire

**F :** > 265°C
**Rf :** 0,25 (heptane/acétate d'éthyle, 80/20)
**SM (APcI-) :** m/z 299 (M-)
**RMN du** ^{**1**}**H (CD**_{**2**}**Cl**_{**2**}**) : δ (ppm)**
8,23 (dd, 1H, H-5 ou H-8, J_{H5-H6} ou J_{H7-H8} = 8,54 Hz, J_{H-F} = 5,50 Hz)
7,78 (dd, 1H, H-5 ou H-8, J_{H-F} = 8,54 Hz, J_{H5-H7} ou J_{H6-H8} = 2,75 Hz)
7,61 (m, 1H, H-5')
7,40 (td, 1H, H-6 ou H-7, J_{H5-H6} ou J_{H7-H8} = J_{H-F} = 8,55 Hz, J_{H5-H7} ou J_{H6-H8} = 2,75 Hz)
7,33 (d, 1H, H-3', J_{H3'-H4'} = 3,66 Hz)
6,60 (dd, 1H, H-4', J_{H3'-H4'} = 3,66 Hz, J_{H4'-H5'} = 1,83 Hz)
**IR (KBr) : ν (cm**^{**-1**}**)**
1680 et 1660 (C=O).

### Exemples 44 et 45

### 4,9-Dihydro-4,9-dioxo-6-fluoro-2-phénylnaphto[2,3-d]thiazole et

### 4,9-Dihydro-4,9-dioxo-7-fluoro-2-phénylnaphto[2,3-d]thiazole

A 0,80 g (2,96 mmoles ; 1,0 éq) d'un mélange 75/25 de 2-amino-3-bromo-1,4-dihydro-1,4-dioxo-6-fluoro-naphtalène et 2-amino-3-bromo-1,4-dihydro-1,4-dioxo-7-fluoro-naphtalène, on ajoute 40 ml d'une solution aqueuse de sulfure de sodium nonahydraté (1,78 g ; 7,40 mmoles ; 2,5 éq). La suspension rouge est chauffée 0,5 heure à 80°C jusqu'à obtention d'une solution bleu foncé. On additionne alors 0,6 ml (5,90 mmoles ; 2,0 éq) de benzaldéhyde, et la solution est encore agitée 2 heures à 80°C. La solution marron obtenue est ramenée à température ambiante, puis quelques gouttes d'acide acétique glacial sont ajoutées. Le précipité vert foncé formé est filtré, lavé trois fois à l'eau et séché. On obtient 0,820 g d'un mélange de 4,9-dihydro-4,9-dioxo-6-fluoro-2-phénylnaphto-[2,3-d]thiazole et 4,9-dihydro-4,9-dioxo-7-fluoro-2-phénylnaphto[2,3-d]thiazole sous forme de cristaux jaunes.

Les isomères sont séparés par trois chromatographies flash successives sur colonne de gel de silice (support : silice 6-35 *µ*m ; ⌀ 9,5 cm ; h 25 cm ; éluant : dichlorométhane/heptane, 70/30) pour fournir 0,205 g du produit le moins polaire et 0,615 g du produit le plus polaire. **Rdt :** 89 % (rapport des isomères : 75/25)

### Produit le moins polaire

**F :** 261°C
**Rf :** 0,48 (dichlorométhane/heptane : 80/20)
**SM (APcI-) :** m/z 309 (M-)
**RMN du** ^{**1**}**H (CD**_{**2**}**Cl**_{**2**}**) : δ (ppm)**
8,20 (dd, 1H, H-5 ou H-8, J_{H5-H6} ou J_{H7-H8} = 8,54 Hz, J_{H-F} = 5,18 Hz)
8,09 (m, 2H, H-2' et H-6')
7,90 (dd, 1H, H-5 ou H-8, J_{H-F} = 8,54 Hz, J_{H5-H7} ou J_{H6-H8} = 2,74 Hz)
7,50 (m, 4H, H-6 ou H-7, H-3', H-4' et H-5')
**IR (KBr) : ν (cm**^{**-1**}**)**
1680 et 1660 (C=O).

### Produit le plus polaire

**F :** 241°C
**Rf :** 0,41 (dichlorométhane/heptane : 80/20)
**SM (APcI-) :** m/z 309 (M-)
**RMN du** ^{**1**}**H (CD**_{**2**}**Cl**_{**2**}**) : δ (ppm)**
8,28 (dd, 1H, H-5 ou H-8, J_{H5-H6} ou J_{H7-H8} = 8,54 Hz, J_{H-F} = 5,19 Hz)
8,08 (dd, 2H, H-2' et H-6', J_{H2'-H3'} = J_{H5'-H6'} = 8,05 Hz, J_{H4'-H6'} = J_{H2'-H4'} = 1,65 Hz)
7,81 (dd, 1H, H-5 ou H-8, J_{H-F} = 8,54 Hz, J_{H5-H7} ou J_{H6-H8} = 2,44 Hz)
7,50 (m, 4H, H-6 ou H-7, H-3' et H-4' et H-5')
**IR (KBr) : ν (cm**^{**-1**}**)**
1680 et 1660 (C=O).

### Exemples 46 et 47

### 4,9-Dihydro-4,9-dioxo-6-fluoro-2-(5-méthyl-2-furyl)-naphto[2,3-d] thiazole et

### 4,9-Dihydro-4,9-dioxo-7-fluoro-2-(5-méthyl-2-furyl)-naphto[2,3-d]thiazole

A 0,80 g (2,96 mmoles ; 1,0 éq) d'un mélange 75/25 de 2-amino-3-bromo-1,4-dihydro-1,4-dioxo-6-fluoro-naphtalène et 2-amino-3-bromo-1,4-dihydro-1,4-dioxo-7-fluoro-naphtalène, on ajoute 40 ml d'une solution aqueuse de sulfure de sodium nonahydraté (1,78 g ; 7,40 mmoles ; 2,5 éq). La suspension rouge est chauffée 30 minutes à 80°C jusqu'à obtention d'une solution bleu foncé. On additionne alors 0,59 ml (5,90 mmoles ; 2,0 éq) de 5-méthyl-2-furaldéhyde, et la solution est encore agitée 90 minutes à 80°C. La solution brun foncé obtenue est ramenée à température ambiante, puis quelques gouttes d'acide acétique glacial sont ajoutées. Le précipité verdâtre formé est filtré, lavé trois fois à l'eau et séché. On obtient 0,705 g d'un mélange 4,9-dihydro-4,9-dioxo-6-fluoro-2-(5-méthyl-2-furyl)naphto[2,3-d]thiazole et 4,9-dihydro-4,9-dioxo-7-fluoro-2- (5-méthyl-2-furyl)naphto-[2,3-d]thiazole sous forme de cristaux rouge foncé.

Les isomères sont séparés par trois chromatographies flash successives sur colonne de gel de silice (support : silice 6-35 *µ*m ; ⌀ 5 cm ; h 35 cm ; éluant : dichlorométhane/heptane, 90/10) pour fournir 0,177 g du produit le moins polaire et 0,528 g du produit le plus polaire.
**Rdt :** 76 % (rapport des isomères : 75/25)

### Produit le moins polaire

**F :** 260°C
**Rf :** 0,40 (dichlorométhane/heptane : 80/20)
**SM (APcI-) :** m/z 313 (M-)
**RMN du** ^{**1**}**H (CD**_{**2**}**Cl**_{**2**}**) : δ (ppm)**
8,18 (dd, 1H, H-5 ou H-8, J_{H5-H6} ou J_{H7-H8} = 8,54 Hz, J_{H-F} = 5,18 Hz)
7,86 (dd, 1H, H-5 ou H-8, J_{H-F} = 8,54 Hz, J_{H5-H7} ou J_{H6-H8} = 2,75 Hz)
7,41 (td, 1H, H-6 ou H-7, J_{H-F} = J_{H5-H6} ou J_{H7-H8} = 8,54 Hz, J_{H5-H7} ou J_{H6-H8} = 2,75 Hz)
7,25 (d, 1H, H-3', J_{H3'-H4'} = 3,36 Hz)
6,24 (d, 1H, H-4', J_{H3'-H4'} = 3,36 Hz)
2,39 (s, 3H, CH₃)
**IR (KBr) : ν (cm**^{**-1**}**)**
1685 et 1650 (C=O).

### Produit le plus polaire

**F :** 238°C
**Rf :** 0,30 (dichlorométhane/heptane, 80/20)
**SM (APcI-) :** m/z 313 (M-)
**RMN du** ^{**1**}**H (CD**_{**2**}**Cl**_{**2**}**) : δ (ppm)**
8,25 (dd, 1H, H-5 ou H-8, J_{H5-H6} ou J_{H7-H8} = 8,54 Hz, J_{H-F} = 5,18 Hz)
7,80 (dd, 1H, H-5 ou H-8, J_{H-F} = 8,54 Hz, J_{H5-H7} ou J_{H6-H8} = 2,75 Hz)
7,42 (td, 1H, H-6 ou H-7, J_{H5-H6} ou J_{H7-H8} = J_{H-F} = 8,54 Hz, J_{H5-H7} ou J_{H6-H8} = 2,75 Hz)
7,25 (d, 1H, H-3', J_{H3'-H4'} = 3,36 Hz)
6,24 (d, 1H, H-4', J_{H3'-H4'} = 3,36 Hz)
2,39 (s, 3H, CH₃)
**IR (KBr) : ν (cm**^{**-1**}**)**
1675 et 1655 (C=O).

### Exemples 48 et 49

### 4,9-Dihydro-4,9-dioxo-6-fluoro-2-(4-fluorophényl)-naphto[2,3-d]thiazole et

### 4,9-Dihydro-4,9-dioxo-7-fluoro-2-(4-fluorophényl)-naphto [2,3-d]thiazole

A 0,80 g (2,96 mmoles ; 1,0 éq) d'un mélange 75/25 de 2-amino-3-bromo-1,4-dihydro-1,4-dioxo-6-fluoro-naphtalène et 2-amino-3-bromo-1,4-dihydro-1,4-dioxo-7-fluoronaphtalène, on ajoute 40 ml d'une solution aqueuse de sulfure de sodium nonahydraté (1,78 g ; 7,40 mmoles ; 2,5 éq). La suspension rouge est chauffée 30 minutes à 80°C jusqu'à obtention d'une solution bleu foncé. On additionne alors 0,63 ml (5,80 mmoles ; 2,0 éq) de 4-fluorobenzaldéhyde, et la solution est encore agitée 90 minutes à 80°C. La solution brun foncé obtenue est ramenée à température ambiante, puis quelques gouttes d'acide acétique glacial sont ajoutées. Le précipité verdâtre formé est filtré, lavé trois fois à l'eau et séché. On obtient 0,570 g d'un mélange de 4,9-dihydro-4,9-dioxo-6-fluoro-2-(4-fluorophényl)naphto[2,3-d]thiazole et 4,9-dihydro-4,9-dioxo-7-fluoro-2-(4-fluorophényl)naphto[2,3-d]thiazole sous forme de cristaux jaune vif.

Les isomères sont séparés par trois chromatographies flash successives sur colonne de gel de silice (support : silice 6-35 *µ*m ; ⌀ 5,5 cm ; h 40 cm ; éluant : dichlorométhane/heptane, 80/20) puis chaque fraction est ensuite lavée plusieurs fois à l'heptane pour fournir 0,143 g du produit le moins polaire et 0,427 g du produit le plus polaire.
**Rdt :** 59 % (rapport des isomères : 75/25)

### Produit le moins polaire

**F :** >265°C
**Rf :** 0,42 (dichlorométhane/heptane, 80/20)
**SM (APcI-) :** m/z 327 (M-)
**RMN du** ^{**1**}**H (CD**_{**2**}**Cl**_{**2**}**) : δ (ppm)**
8,21 (dd, 1H, H-5 ou H-8, J_{H5-H6} ou J_{H7-H8} = 8,54 Hz, J_{H-F} = 5,19 Hz)
8,10 (dd, 2H, H-2' et H-6', J_{H2'-H3'} = J_{H5'-H6'} = 8,85 Hz, J_{H2'-F} = J_{H6'-F} = 5,19 Hz)
7,89 (dd, 1H, H-5 ou H-8, J_{H-F} = 8,54 Hz, J_{H5-H7} ou J_{H6-H8} = 2,75 Hz)
7,43 (td, 1H, H-6 ou H-7, J_{H-F} = J_{H5-H6} ou J_{H7-H8} = 8,54 Hz, J_{H5}-_{H7} ou J_{H6-H8} = 2,75 Hz)
7,20 (t, 2H, H-3' et H-5', J_{H-F} = J_{H2'-H3'} = J_{H5'-H6'} = 8,85 Hz)
**IR (KBr) : ν (cm**^{**-1**}**)**
1675 et 1655 (C=O)

### Produit le plus polaire

**F :** >265°C
**Rf :** 0,40 (dichlorométhane/heptane, 80/20)
**SM (APcI-) :** m/z 327 (M-)
**RMN du** ^{**1**}**H (CD**_{**2**}**Cl**_{**2**}**) : δ (ppm)**
8,28 (dd, 1H, H-5 ou H-8, J_{H5-H6} ou J_{H7-H8} = 8,54 Hz, J_{H-F} = 5,19 Hz)
8,11 (dd, 2H, H-2' et H-6', J_{H2'-H3'} = J_{H5'-H6'} = 8,85 Hz, J_{H2'-F} = J_{H6'-F} = 5,19 Hz)
7,81 (dd, 1H, H-5 ou H-8, J_{H-F} = 8,54 Hz, J_{H5-H7} ou J_{H6-H8} = 2,75 Hz)
7,45 (td, 1H, H-6 ou H-7, J_{H-F} = J_{H5-H6} ou J_{H7-H8} = 8,54 Hz, J_{H5-} _{H7} ou J_{H6-H8} = 2,75 Hz)
7,20 (t, 2H, H-3' et H-5', J_{H-F} = J_{H2'-H3'} = J_{H5'-H6'} = 8,85 Hz)
**IR (KBr) : ν (cm**^{**-1**}**)**
1675 et 1660 (C=O)

### Exemples 50 et 51

### 4,9-Dihydro-4,9-dioxo-6-fluoro-2-(4-méthylphényl)-naphto[2,3-d]thiazole et

### 4,9-Dihydro-4,9-dioxo-7-fluoro-2-(4-méthylphényl)-naphto[2,3-d]thiazole

A 1 g (3,70 mmoles ; 1,0 éq) d'un mélange 75/25 de 2-amino-3-bromo-1,4-dihydro-1,4-dioxo-6-fluoronaphtalène et de 2-amino-3-bromo-1,4-dihydro-1,4-dioxo-7-fluoro-naphtalène, on ajoute 50 ml d'une solution aqueuse de sulfure de sodium nonahydraté (2,22 g ; 9,20 mmoles ; 2,5 éq). La suspension rouge est chauffée 30 minutes à 80°C jusqu'à obtention d'une solution bleu foncé. On additionne alors 0,87 ml (7,40 mmoles ; 2,0 éq) de 4-méthylbenzaldéhyde, et la solution est encore agitée 90 minutes à 80°C. La solution brun foncé obtenue est ramenée à température ambiante, puis quelques gouttes d'acide acétique glacial sont ajoutées. Le précipité verdâtre formé est filtré, lavé trois fois à l'eau et séché. On obtient 0,623 g d'un mélange de 4,9-dihydro-4,9-dioxo-6-fluoro-2-(4-méthylphényl)naphto[2,3-d]thiazole et de 4,9-dihydro-4,9-dioxo-7-fluoro-2-(4-méthylphényl)naphto[2,3-d]thiazole sous forme de cristaux jaunes.

Les isomères sont séparés par chromatographie flash sur colonne de gel de silice (support : silice 6-35 *µ*m ; Ø 9 cm ; h 35 cm ; éluant : dichlorométhane/heptane, 70/30). Les fractions réunies sont concentrées sous vide et le produit solide formé est lavé sur verre fritté deux fois avec le minimum de méthanol et cinq fois à l'heptane pour fournir 0,467 g du produit le plus polaire et 0,156 g du produit le moins polaire sous forme de cristaux jaunes.
**Rdt :** 52 % (rapport des isomères : 75/25)

### Produit le plus polaire

**F :** >260°C
**Rf :** 0,42 (dichlorométhane/heptane : 70/30)
**SM (APcI-) :** m/z 323 (M-)
**RMN du** ^{**1**}**H** (**CD**_{**2**}**Cl**_{**2**}) : **δ (ppm)**
8,27 (dd, 1H, H-5 ou H-8, J_{H5-H6} ou J_{H7-H8} = 8,54 Hz, J_{H-F} = 5,19 Hz)
7,96 (d, 2H, H-2' et H-6', J_{H2'-H3'} = J_{H5'-H6'} = 8,24 Hz)
7,80 (dd, 1H, H-5 ou H-8, J_{H-F} = 8,54 Hz, J_{H5-H7} ou J_{H6-H8} = 2,75 Hz)
7,44 (td, 1H, H-6 ou H-7, J_{H-F} = J_{H5-H6} ou J_{H7-H8} = 8,54 Hz, J_{H5-H7} Ou J_{H6-H8} = 2,75 Hz)
7,30 (d, 2H, H-3' et H-5', J_{H2'-H3'} = J_{H5'-H6'} = 8,24 Hz)
2,38 (s, 3H, CH₃)
**IR (KBr) : ν (cm**^{**-1**}**)**
1670 et 1665 (C=O),

### Produit le moins polaire

**F :** > 260°C
**Rf :** 0,46 (dichlorométhane/heptane : 70/30)
**SM (APcI-) :** m/z 323 (M-)
**RMN du** ^{**1**}**H (CD**_{**2**}**Cl**_{**2**}**) : δ (ppm)**
8,20 (dd, 1H, H-5 ou H-8, J_{H5-H6} ou J_{H7-H8} = 8,54 Hz, J_{H-F} = 5,19 Hz)
7,97 (d, 2H, H-2' et H-6', J_{H2'-H3'} = J_{H5'-H6'} = 8,24 Hz)
7,88 (dd, 1H, H-5 ou H-8, J_{H-F} = 8,85 Hz, J_{H5-H7} ou J_{H6-H8} = 2,75 Hz)
7,42 (td, 1H, H-6 ou H-7, J_{H-F} = J_{H5-H6} ou J_{H7-H8} = 8,54 Hz, J_{H5-H7} ou J_{H6-H8} = 2,75 Hz)
7,31 (d, 2H, H-3' et H-5', J_{H2'-H3'} = J_{H5'-H6'} = 8,24 Hz)
2,39 (s, 3H, CH₃)
**IR (KBr) : ν (cm**^{**-1**}**)**
1675 et 1655 (C=O).

### Exemples 52 et 53

### 4,9-Dihydro-4,9-dioxo-5-fluoro-2-(2-furyl)naphto-[2,3-d]thiazole

### 4,9-Dihydro-4,9-dioxo-8-fluoro-2-(2-furyl)naphto-[2,3-d]thiazole

### Intermédiaires de synthèse

### 2,3-Dibromo-1,4-dihydro-1,4-dioxo-5-fluoronaphtalène

A une solution de 1,4-dihydro-1,4-dioxo-5-fluoro-naphtalène (N° CAS 62784-46-7) 2,45 g (71 mmoles) dans le chloroforme (60 ml), on ajoute 7,34 ml (143 mmoles) de brome. La solution est portée au reflux pendant 12 heures puis ramenée à température ambiante. Après un barbotage d'air comprimé, la solution est concentrée sous pression réduite et le produit solide beige obtenu est purifié sur colonne flash (support : silice ; conditionnement : heptane ; éluant : CH₂Cl₂/heptane) pour fournir 3,44 g de 2,3-dibromo-1,4-dihydro-1,4-dioxo-5-fluoronaphtalène sous forme de cristaux beiges.
**Rdt :** 74 %
**F :** 100°C
**Rf :** 0,63 (dichlorométhane/heptane : 80/20)
**SM (I.E.) :** m/z 333, 335, 337 (M⁺+1)
**RMN du** ^{**1**}**H (CDCl**_{**3**}**) : δ (ppm)**
8,01 (d, 1H, H-8, J_{H7-H8} = 7,94 Hz)
7,77 (m, 1H, H-7)
7,52 (m, 1H, H-6)
**IR (KBr) : ν (cm**^{**-1**}**)**
1704 (C=O)

### 2-Amino-3-bromo-1,4-dihydro-1,4-dioxo-5-fluoro-naphtalène

### 2-Amino-3-bromo-1,4-dihydro-1,4-dioxo-8-fluoro-naphtalène

Dans une solution de 2,3-dibromo-1,4-dihydro-1,4-dioxo-5-fluoronaphtalène (33 mg; 0,098 mmole) dans le tétrahydrofuranne (5 ml), on fait barboter de l'ammoniac à température ambiante pendant 1 heure, puis de l'air comprimé est passé à travers la solution pendant 15 minutes pour éliminer l'excès d'ammoniac. Après évaporation du solvant sous pression réduite, le produit solide rouge obtenu est purifié sur plaque préparative de silice (éluant CH₂Cl₂/Heptane = 90/10). On obtient 20,5 mg d'une poudre rouge, mélange de 2-amino-3-bromo-1,4-dihydro-1,4-dioxo-5-fluoronaphtalène et de 2-amino-3-bromo-1,4-dihydro-1,4-dioxo-8-fluoronaphtalène.
**Rdt :** 77 %
**F :** 208°C
**Rf :** 0,44 (dichlorométhane)
**SM (APcI-) :** m/z 269, 271 (M-)
**RMN du** ^{**1**}**H (CDCl**_{**3**}**) : δ (ppm)**
7,98 (d, 1H, H-5 ou H-8, J_{H5-H6} ou J_{H7-H8} = 7,63 Hz)
7,64 (m, 1H, H-6 ou H-7)
7,31 (dd, 1H, H-6 ou H-7, J_{H5-H6} ou J_{H7-H8} = J_{H-F} = 8,55 Hz)
6,40-5,00 (sl, 2H, NH₂)
**IR (KBr) : ν (cm**^{**-1**}**)**
3466, 3355 (NH₂), 1778, 1633 (C=O)

### 4,9-Dihydro-4,9-dioxo-5-fluoro-2-(2-furyl)naphto-[2,3-d]thiazole

### 4,9-Dihydro-4,9-dioxo-8-fluoro-2-(2-furyl)naphto-[2,3-d]thiazole

A 0,15 g (0,55 mmole ; 1,0 éq) d'un mélange de 2-amino-3-bromo-1,4-dihydro-1,4-dioxo-5-fluoronaphtalène et de 2-amino-3-bromo-1,4-dihydro-1,4-dioxo-8-fluoro-naphtalène dans 25 ml d'eau, on ajoute 0,33 g (1,38 mmole ; 2,5 éq) de sulfure de sodium nonahydraté. La suspension rouge est chauffée 30 minutes à 80°C jusqu'à obtention d'une solution bleu foncé. On additionne alors 0,1 ml (1,1 mmole ; 2,0 éq) de 2-furaldéhyde. Après 90 minutes de chauffage à 80°C, la solution brun foncé obtenue est ramenée à température ambiante. Quelques gouttes d'acide acétique glacial sont ajoutées puis le précipité marron formé est filtré, lavé trois fois à l'eau et séché. Le mélange de produits obtenu est purifié par chromatographie sur couche mince préparative (silice 2 mm ; éluant : dichlorométhane/heptane/acétate d'éthyle, 80/10/10), puis les deux isomères du composé attendu sont séparés par chromatographie sur couche mince préparative (silice 2 mm ; éluant : dichlorométhane) pour fournir 0,020 g d'un mélange de 4,9-dihydro-4,9-dioxo-5-fluoro-2-(2-furyl)naphto[2,3-d]thiazole et de 4,9-dihydro-4,9-dioxo-8-fluoro-2-(2-furyl)naphto[2,3-d]thiazole sous forme de cristaux orange.
Rdt :12 % (rapport des isomères 47/53)

### Isomère le moins polaire

**F : >** 260°C
**Rf :** 0,34 (dichlorométhane)
**SM (APcI+) :** m/z 300 (M+H+)
**RMN du** ^{**1**}**H (CD**_{**2**}**Cl**_{**2**}**) : δ (ppm)**
8,09 (dd, 1H, H-5 ou H-8, J_{H5-H6} ou J_{H7-H8} = 8,24 Hz, J_{H5-H7} ou J_{H6-H8} = 1,22 Hz)
7,75 (td, 1H, H-6 ou H-7, J_{H5-H6} ou J_{H7-H8} = J_{H6-H7} = 7,94 Hz, J_{H-F} = 4,58 Hz)
7,65 (dd, 1H, H-5', J_{H3'-H5'} = 0,92 Hz, J_{H4'-H5'} = 1,84 Hz)
7,46 (ddd, 1H, H-6 ou H-7, J_{H-F} = 10,98 Hz, J_{H6-H7} = 8,24 Hz, J_{H5-H7} ou J_{H6-H8} = 1,22 Hz)
7,35 (d, 1H, H-3', J_{H3'-H4'} = 3,67 Hz)
6,63 (dd,1H, H-4', J_{H3'-H4'} = 3,67 Hz, J_{H4'-H5'} = 1,84 Hz)
**IR (KBr) :** ν **(cm**^{**-1**}**)**
1680 et 1655 (C=O).

### Isomère le plus polaire

**F :** > 260°C
**Rf :** 0,28 (dichlorométhane)
**SM (APcI+) :** m/z 300 (M+H+)
**RMN du** ^{**1**}**H (CD**_{**2**}**Cl**_{**2**}**) : δ (ppm)**
8,02 (dd, 1H, H-5 ou H-8, J_{H5-H6} ou J_{H7-H8} = 7,63 Hz, J_{H5-H7} ou J_{H6-H8} = 1,22 Hz)
7,73 (td, 1H, H-6 ou H-7, J_{H5-H6} ou J_{H7-H8} = J_{H6-H7} = 8,24 Hz, J_{H-F} = 4,58 Hz)
7,64 (dd, 1H, H-5', J_{H3'-H5'} = 0,91 Hz, J_{H4'-H5'} = 1,83 Hz)
7,46 (ddd, 1H, H-6 ou H-7, J_{H-F} = 11,29 Hz, J_{H5-H6} ou J_{H7-H8} = 8,54 Hz, J_{H5-H7} ou J_{H6-H8} = 1,22 Hz)
7,37 (d, 1H, H-3', J_{H3'-H4'} = 3,66 Hz)
6,64 (dd,1H, H-4', J_{H3'-H4'} = 3,66 Hz, J_{H4'-H5'} = 1,83 Hz)
**IR (KBr) : ν (cm**^{**-1**}**)**
1680 et 1655 (C=O).

### Exemples 54 et 55

### 6-Chlore-4,9-dihydro-4,9-dioxo-2-(2-furyl)-naphto-[2,3-d]thiazole

### 7-Chlore-4,9-dihydro-4,9-dioxo-2-(2-furyl)-naphto-[2,3-d]thiazole

A 435 mg (1,79 mmole) d'un mélange de 2-amino-3,6-dichloro-1,4-dihydro-1,4-dioxonaphtalène et de 2-amino-3,7-dichloro-1,4-dihydro-1,4-dioxonaphtalène on ajoute à température ambiante et sous argon 1,72 g (7,16 mmoles) de sulfure de sodium nonahydraté en solution dans 15 ml de soude pH 10,6. Après 30 minutes à 60°C et sous argon, on ajoute au milieu réactionnel devenu totalement bleu 296 µl (3,58 mmoles) de 2-furaldéhyde. Après 15 minutes de reflux, l'arrivée d'argon est supprimée, et le mélange réactionnel est dilué avec 250 ml d'eau et extrait 3 fois avec 150 ml de dichlorométhane. La phase organique est ensuite lavée avec 300 ml d'eau et évaporée à sec pour fournir 450 mg du mélange de 6-chloro-4,9-dihydro-4,9-dioxo-2-(2-furyl)-naphto[2,3-d]thiazole et de 7-chloro-4,9-dihydro-4,9-dioxo-2-(2-furyl)-naphto[2,3-d]thiazole sous forme de cristaux orange.

Les deux isomères sont séparés sur cake de silice (silice 40-60 mm, diamètre 7 cm, hauteur 14 cm, éluant : dichlorométhane/heptane, 90/10) et on obtient 80 mg du produit le moins polaire et 150 mg du produit le plus polaire.

### Produit le moins polaire

**Rdt :** 40 %
**F :** > 260°C
**Rf :** 0,42 (CH₂Cl₂/MeOH, 99/1)
**S.M. (APcI-) :** m/z 315 et 317 (M-)
**RMN du** ^{**1**}**H (CDCl**_{**3**}**) : δ (ppm)**
8,28 (d 1H, H-5 ou H-8, J_{H5-H-7} ou J_{H6-H8} = 2,08 Hz)
8,17 (d, 1H, H-5 ou H-8, J_{H7-H8} ou J_{H5-H6} = 8,31 Hz)
7,75 (dd, 1H, H-6 ou H-7, J_{H7-H8} ou J_{H5-H6} = 8,31 Hz, J_{H5-H7} ou J_{H6-H8} = 2,07 Hz)
7,65 (m, 1H, H-5')
7,46 (d, 1H, H-3', J_{H3'-H4'} = 3,74 Hz)
6,66 (dd, 1H, H-4', J_{H3'-H4'} = 3,74 Hz, J_{H3'-H5'} = 1,66 Hz)
**RMN du** ^{**13**}**C (CDCl**_{**3**}**) : δ (ppm)**
146,81 (C-5')
134,72 (C-6 ou C-7)
129,31, 128,53 (2C, C-5, C-8)
114,75 et 114,11 (2C, C-3', C-4')
**IR (KBr) :** ν **(cm**^{**-1**}**)**
1675, 1665 (C=O)

### Produit le plus polaire

**Rdt :** 26 %
**F :** > 260°C
**Rf :** 0,36 (CH₂Cl₂/MeOH, 99/1)
**S.M. (APcI+) :** m/z 316 et 318 (M+H+)
**RMN du** ^{**1**}**H (CDCl**_{**3**}**) : δ (ppm)**
8,28 (d 1H, H-5 ou H-8, J_{H7-H8} ou J_{H5-H6} = 8 Hz)
8,18 (d, 1H, H-5 ou H-8, J_{H5-H7} ou J_{H6-H8} = 1,84 Hz)
7,77 (dd, 1H, H-6 ou H-7, J_{H7-H8} ou J_{H5-H6} = 8,04 Hz , J_{H5-H-7} ou J_{H6-H8} = 1,84 Hz)
7,67 (1s, 1H, H-5')
7,47 (d, 1H, H-3', J_{H3'-H4'} = 3,45 Hz)
6,66 (m, 1H, H-4')
**RMN du** ^{**13**}**C (CDCl**_{**3**}**) : δ (ppm)**
178,50, 177,90 (2C, C-4, C-9)
148,5 (C-2')
146,8 (C-5')
141,8 (C-6 ou C-7)
134,9 (C-6 ou C-7)
131,8 (2C, C-4a, C-8a)
130,09, 127,64 (2C, C-5, C-8)
114,80 (C-3')
114,11 (C-4')
**IR (KBr) : ν (cm**^{**-1**}**)**
1675, 1650 (C=O)

### Exemple 56

### 4,9-Dihydro-4,9-dioxo-2-(2-furyl)-5-méthoxynaphto-[2,3-d]thiazole ou

### 4,9-Dihydro-4,9-dioxo-2-(2-furyl)-8-méthoxynaphto-[2,3-d]thiazole

Sous atmosphère inerte, à 3,64 g (12,9 mmoles) d'un mélange de 2-amino-3-bromo-1,4-dihydro-1,4-dioxo-5-méthoxynaphtalène et de 2-amino-3-bromo-1,4-dihydro-1,4-dioxo-8-méthoxynaphtalène sont ajoutés 140 ml d'une solution aqueuse contenant 7,74 g (32,2 mmoles) de sulfate de sodium nonahydraté. La suspension ainsi obtenue est portée à 82°C jusqu'à donner une solution «bleue encre». 2,1 ml (25,8 mmoles) de 2-Furaldéhyde sont alors additionnés au milieu réactionnel. Le mélange devient progressivement rouge brique. Après 1,25 heure, 6,73 g (38,7 mmoles) d'hydrosulfite de sodium sont ajoutés au mélange réactionnel, un précipité marron apparaît progressivement. Le précipité est filtré à chaud sur verre fritté et lavé à l'eau. Le produit brut est recristallisé dans le DMF. Une deuxième recristallisation est réalisée dans le dichlorométhane. Le filtrat, après évaporation sous pression réduite, est purifié sur cake (support : silice 6-35 *µ*m ; conditionnement : CH₂Cl₂/heptane, 80/20 ; éluant : CH₂Cl₂/MeOH, 100/0 à 90/10). Le solide obtenu est lavé à l'heptane, décoloré sur noir animal dans du dichloro-méthane, puis filtré sur micropore pour fournir après évaporation sous pression réduite 0,32 g de 4,9-dihydro-4,9-dioxo-2-(2-furyl)-5-méthoxynaphto[2,3-d]thiazole ou de 4,9-dihydro-4,9-dioxo-2-(2-furyl)-8-méthoxynaphto[2,3-d]thiazole sous forme de cristaux orange.
**Rdt :** 8 %
**F :** > 260°C
**Rf :** 0,60 (CH₂Cl₂/MeOH, 98/2)
**S.M. (APcI+) :** m/z 312 (M⁺H⁺)
**RMN du** ^{**1**}**H (CD**_{**2**}**Cl**_{**2**}**) :** δ **(ppm)**
7,78 (dd, 1H, H-8 ou H-5, J_{H7-H8} = 7,63 Hz, J_{H6-H8} = 1,22 Hz)
7,65 (t, 1H, H-7 ou H-6, J_{H7}-_{H8} # J_{H6}-_{H7} = 7,62 Hz)
7,60 (dd, 1H, H-5', J_{H4'-H5'} = 1,53 Hz, J_{H3'-H5'} = 0,92 Hz)
7,33 (dd, 1H, H-6 ou H-7 en a du groupement méthoxy, J_{H6-H7} = 8,24 Hz, J_{H6}-_{H8} ou J_{H5}-_{H7} = 0,92 Hz)
7,31 (s, 1H, H-3')
6,59 (dd, 1H, H-4', J_{H3'}-_{H4'} = 3,67 Hz, J_{H4'}-_{H5}, 1,83 Hz)
3,96 (s, 3H, OCH₃)
**IR (KBr) : ν (cm**^{**-1**}**)**
3112 (C-H) ; 1674 (C=O) ; 1655 (C = N)

### Exemple 57

### 4,9-Dihydro-4,9-dioxo-2-(2-furyl)-5-hydroxonaphto-[2,3-d]thiazole ou

### 4,9-Dihydro-4,9-dioxo-2-(2-furyl)-8-hydroxynaphto-[2,3-d]thiazole

A une solution de 4,9-dihydro-4,9-dioxo-2-(2-furyl)-5-méthoxynaphto[2,3-d]thiazole ou de 4,9-dihydro-4,9-dioxo-2-(2-furyl)-8-méthoxynaphto[2,3-d]thiazole (230 mg, 0,73 mmole) dans 20 ml d'acide acétique est ajouté 0,90 ml d'acide bromhydrique à 47 % (7,69 mmoles). Le mélange réactionnel sous agitation est porté à reflux durant 5 heures. Après retour à température ambiante, de l'eau (30 ml) est additionnée au milieu réactionnel. Cette solution est alors extraite au dichlorométhane. La phase organique est lavée à l'eau jusqu'à pH neutre. Après séchage sur du chlorure de calcium, la phase organique est évaporée sous pression réduite. Le brut réactionnel est purifié sur cake (support : silice 6-35 *µ*m ; éluant CH₂Cl₂). Le produit, dissous dans le dichlorométhane, est décoloré sur noir animal. Après un lavage à l'heptane, on obtient 110 mg de 4,9-dihydro-4,9-dioxo-2-(2-furyl)-5(ou 8)-hydroxynaphto[2,3-d]thiazole sous forme d'une poudre orange.
**Rdt :** 50 %
**F :** 257°C
**Rf :** 0,50 (CH₂Cl₂)
**S.M.** (APcI+) : m/z 298 (M+H⁺)
**RMN du** ^{**1**}**H (CD**_{**2**}**Cl**_{**2**}**) : δ (ppm)**
12,25 (s, 1H, OH)
7,77 (dd, 1H, H-8 ou H-5, J_{H7-H8} ou J_{H5}-_{H6} = 7,63 Hz, J_{H5-H7} = 1,22 Hz)
7,70 (dd, 1H, H-5', J_{H4'-H5'} = 1,84 Hz, J_{H3'-H5'} = 0,92 Hz)
7,68 (t, 1H, H-7 ou H-6 en b du groupement hydroxy ,J_{H7}-_{H8} ou J_{H5}-_{H6} = 7,63 Hz, J_{H6-H7} = 8,23 Hz )
7,41 (d, 1H, H-3', J_{H3'-H4'} = 3,67 Hz)
7,34 (dd, 1H, H-6 ou H-7 en a du groupement hydroxy, J_{H6}-_{H7} = 8,23 Hz, J_{H6}-_{H8} ou J_{H5}-_{H7} = 1,22 Hz)
6,69 (dd, 1H, H-4', J_{H3'-H4'} = 3,66 Hz, J_{H4'}-_{H5'} = 1,83 Hz)
**RMN du** ^{**13**}**C (CD**_{**2**}**Cl**_{**2**}**) : δ (ppm)**
183,72 (1C, C-4 ou C-9)
177,73 (1C, C-9 ou C-4)
164,31 (1C, C-5 ou C-8)
163,45 (1C, C-2)
155,15 (1C, C-2')
148,41 (1C, C-3a)
146,68 (1C, C-5')
142,00 (1C, C-9a)
137,07 (1C, C-7 ou C-6 en β du groupement hydroxy)
133,76 (1C, C-8a ou C-4a)
125,74 (1C, C-8 ou C-5)
120,32 (1C, C-6 ou C-7 en α du groupement hydroxy)
115,69 (1C, C-4a ou C-8a)
113,75 et 114,07 (2C⁻, C-3' et C-4')
**IR (KBr) : ν (cm**^{**-1**}**)**
3400 (OH) ; 3124 (C-H) ; 1644 (C=O) ; 1584 (C-C)

### Exemples 58 et 59

### 4,9-Dihydro-4,9-dioxo-2-(2-furyl)-6-méthoxnaphto-[2,3-d]thiazole et

### 4,9-Dihydro-4,9-dioxo-2-(2-furyl)-7-méthoxynaphto-[2,3-d]thiazole

Sous atmosphère inerte, à 2,00 g (7,1 mmoles) d'un mélange de 2-amino-3-bromo-1,4-dihydro-1,4-dioxo-6-méthoxynaphtalène et 3-amino-2-bromo-1,4-dihydro-1,4-dioxo-6-méthoxynaphtalène sont ajoutés 75 ml d'une solution aqueuse contenant 4,25 g (17,7 mmoles) de sulfure de sodium nonahydraté. La suspension ainsi obtenue est portée à 80°C, la naphtoquinone se dissout progressivement jusqu'à donner une solution «bleue encre». 1,2 ml (14,2 mmoles) de 2-furaldéhyde est alors additionné au milieu réactionnel. Le mélange devient progressivement rouge brique. Après une heure et demie et refroidissement à 50°C, 2,45 g (14,2 mmoles) d'hydrosulfite de sodium sont ajoutés au mélange réactionnel, un précipité marron apparaît. Le précipité est filtré à chaud sur verre fritté et lavé à l'eau jusqu'à ce que les eaux de lavage soient incolores. Après séchage à l'étuve sous vide, le produit brut est purifié sur cake de gel de silice (silice 6-35 mm, éluant CH₂Cl₂/MeOH, 98/2). 1,15 g (3,7 mmoles) du mélange de 4,9-dihydro-4,9-dioxo-2-(2-furyl)-6-méthoxy-naphto-[2,3-d]thiazole et 4,9-dihydro-4,9-dioxo-2-(2-furyl)-7-méthoxynaphto[2,3-d]thiazole.

La séparation des deux isomères est réalisée par trois chromatographies basse pression successives du mélange (silice 6-35 *µ*m, éluant CH₂Cl_{2/}AcOEt, 99/1). Après décoloration et recristallisation dans du dichlorométhane de chaque isomère on obtient 0,210 g de cristaux orange du produit le plus polaire et 0,300 g de cristaux orange du produit le moins polaire.

### Produit le plus polaire

**Rdt :** 9,5 %
**F : >** 260°C
**Rf :** 0,58 (CH₂Cl_{2/}AcOEt, 90/10)
**S.M. (I.E.) :** m/z 311 (M⁺)
**R.M.N. du** ^{**1**}**H (CD**_{**2**}**Cl**_{**2**}**) : δ (ppm)**
8,22 (d, 1H, H-8 ou H-5, J_{H5-H6} ou J_{H7-H8} = 8,85 Hz)
7,69 (sl, 1H, H-5')
7,66 (d, 1H, H-5 ou H-8 en a du groupement méthoxy, J_{H5-H7} ou J_{H6-H8} = 2,74 Hz)
7,40 (d, 1H, H-3', J_{H3'-H4'} = 3,66 Hz)
7,27 (dd, 1H, H-7 ou H-6, J_{H5-H6} ou J_{H7-H8} = 8,55 Hz, J_{H6-H8} ou
J_{H5-H7} = 2,45 Hz)
6,68 (dd, 1H, H-4', J_{H3'-H4'} = 3,66 Hz, J_{H4'-H5'} = 1,83 Hz)
3,98 (s, 3H, CH₃O en 6 ou 7)
**I.R. (KBr) : ν (cm**^{**-1**}**)**
1675 (C=O),1650 (C = N), 1589

### Produit le moins polaire

**Rdt :** 13,5 %
**F :** > 260°C
**Rf :** 0,68 (CH₂Cl_{2/}AcOEt, 90/10)
**S.M. (I.E.) :** m/z 311 (M+)
**R.M.N. du** ^{**1**}**H (CD**_{**2**}**Cl**_{**2**}**) :** δ **(ppm)**
8,15 (d, 1H, H-8 ou H-5, J_{H5-H6} ou J_{H7-H8} = 8,55 Hz)
7,73 (d, 1H, H-5 ou H-8 en a du groupement méthoxy, J_{H5-H7} ou J_{H6-H8} = 2,75 Hz)
7,68 (d, 1H, H-5', J_{H4'-H5'} = 1,83 Hz)
7,38 (d, 1H, H-3', J_{H3'-H4'} = 3,67 Hz)
7,25 (dd, 1H, H-7 ou H-6, J_{H5-H6} ou J_{H7-H8} = 8,55 Hz, J_{H6-H8} ou
J_{H5-H7} = 2,75 Hz )
6,68 (dd, 1H, H-4', J_{H3'-H4'} = 3,66 Hz, J_{H4'-H5'} = 1,83 Hz)
3,99 (s, 3H, CH₃O en 6 ou 7)
**I.R. (KBr) : ν (cm**^{**-1**}**)**
1681 (C=O), 1645 (C = N), 1586

### Exemples 60 et 61

### 4,9-Dihydro-4,9-dioxo-2-furyl-6-méthylnaphto[2,3-d]-thiazole et

### 4,9-Dihydro-4,9-dioxo-2-furyl-7-méthylnaphto[2,3-d]-thiazole

### Intermédiaire de synthèse :

### 2-Amino-3-chloro-1,4-dihydro-1,4-dioxo-6-méthyl-naphtalène et

### 2-Amino-3-chloro-1,4-dihydro-1,4-dioxo-7-méthyl-naphtalène

A une solution formée de 6,00 g (25 mmoles, 1,0 éq) d'un mélange de 2-chloro-1,4-dihydro-1,4-dioxo-6-méthyl-naphtalène (N° CAS 87 170-60-3) et de 2-chloro-1,4-dihydro-1,4-dioxo-7-méthylnaphtalène (N° CAS 87 170-61-4) et de 250 ml d'acide acétique glacial, on ajoute en une seule fois, 2,60 g (40 mmoles, 1,6 éq) de nitrure de sodium en solution dans 16 ml d'eau distillée. Ce milieu est chauffé à 80°C pendant 5 heures ; sa couleur passe du jaune à l'orange. Après refroidissement, le milieu réactionnel est évaporé à sec et le produit brut obtenu est purifié sur lit de silice (support : silice 6-35 *µ*m, d = 10 cm, h = 5 cm, dépôt solide, éluant : Heptane/Acétace d'éthyle, 92/8) pour fournir après évaporation des solvants 0,44 g d'un mélange de 2-amino-3-chloro-1,4-dihydro-1,4-dioxo-6-méthylnaphtalène et 2-amino-3-chloro-1,4-dihydro-1,4-dioxo-7-méthylnaphtalène sous forme de cristaux rouges.
**Rdt :** 8 %
**Rf :** 0,38 (Heptane/Acétate d'éthyle, 70/30)
**SM (APcI+) :** m/z 222,224 (M⁺H⁺)
**RMN du** ^{**1**}**H (CDCl**_{**3**}**) : δ (ppm)**
7,99 et 7,95 (2d, 2H, protons en b du CH₃, J = 7,93 Hz) 7,87 (s, 2H, protons en a du CH₃)
7,56 et 7,48 (2d, 2H, protons en a du CH₃)
2,49 et 2,47 (2s, 6H, 2 x CH₃)

### 4,9-Dihydro-4,9-dioxo-2-furyl-6-méthylnaphto[2,3-d]-thiazole et

### 4,9-Dihydro-4,9-dioxo-2-furyl-7-méthylnaphto[2,3-d]-thiazole (Exemples 60 et 61)

A une solution formée de 1,3 g (5,4 mmoles, 6,0 éq) de sulfure de sodium nonahydraté et 3,2 ml d'une solution d'hydroxyde de sodium préalablement préparée à pH 10,7, on ajoute 0,2 g (0,9 mmole, 1,0 éq) d'un mélange de 2-amino-3-chloro-1,4-dihydro-1,4-dioxo-6-méthylnaphtalène et de 2-amino-3-chloro-1,4-dihydro-1,4-dioxo-7-méthyl-naphtalène. Cette suspension, chauffée à 45°C conduit en 30 minutes à une solution bleue. On ajoute alors 149 *µ*l (1,8 mmole, 2,0 éq) de 2-furaldéhyde puis au bout de 15 minutes 97 *µ*l (1,7 mmole, 1,9 éq) d'acide acétique glacial 55°C. Le milieu réactionnel devenu marron est extrait par 6 x 100 ml de dichlorométhane. Les phases aqueuses sont réunies, séchées sur du sulfate de magnésium, filtrées puis évaporées à sec. Le produit obtenu est purifié sur lit de silice (support : silice 6-35 *µ*m, h = 15 cm, d = 5 cm, dépôt solide, éluant : heptane/acétate d'éthyle, 90/10) pour fournir après évaporation des solvants 0,13 g du mélange de 4,9-dihydro-4,9-dioxo-2-furyl-6-méthylnaphto-[2,3-d]-thiazole et 4,9-dihydro-4,9-dioxo-2-furyl-7-méthylnaphto-[2,3-d]thiazole des deux isomères sous forme de cristaux orange.

Les isomères sont ensuite séparés par plaques préparatives (support : alumine, éluant : dichlorométhane/ heptane, 70/30).
**Rdt :** 48,8 % (mélange des deux isomères)

### Produit le plus polaire

**Rf :** 0,42 (dichlorométhane/heptane, 70/30, alumine)
**SM (APcI+) :** m/z 296 (M+H⁺)
**RMN du** ^{**1**}**H (CD**_{**2**}**Cl**_{**2**}**) : δ (ppm)**
8,20 (d, 1H, H-5 ou H-8 en b du CH₃, J_{H5-H6} ou J_{H7-H8} = 7,93 Hz)
8,05 (m, 1H, H-5 ou H-8 en a du CH₃)
7,73 (dd, 1H, H-5', J_{H4'-H5'} = 1,83 Hz, J_{H3'-H5'} = 0,61 Hz)
7,67 (d, 1H, H-6 ou H-7, J_{H5-H6} ou J_{H7-H8} = 7,94 Hz, en a du CH₃)
7,44 (dd, 1H, H-3', J_{H3'-H4'} = 3,67 Hz, J_{H3'-H5'} = 0,61 Hz)
6,72 (dd, 1H, H-4', J_{H3'-H4'} = 3,66 Hz, J_{H4'-H5'} = 1,83 Hz)
2,57 (s, 3H, CH₃)
**RMN du** ^{**13**}**C (CD**_{**2**}**Cl**_{**2**}**) : δ (ppm)**
146,41 (1C, C-5')
135,31 (1C, C-6 ou C-7, en a du CH₃)
127,99 (1C, C-5 ou C-8)
127,55 (1C, C-5 ou C-8)
113,70 (1C, C-3')
113,59 (1C, C-4')
21,94 (1C, CH₃)

### Produit le moins polaire

**Rf :** 0,50 (Dichlorométhane/Heptane, 70/30, alumine)
**SM (APcI+) :** m/z 296 (M+H⁺)
**RMN du** ^{**1**}**H (CD**_{**2**}**Cl**_{**2**}**) : δ (ppm)**
8,01 (m, 2H, H-5, H-8)
7,60 (dd, 1H, H-5', J_{H4'-H5'} = 1,83 Hz, J_{H3'-H5'} = 0,61 Hz)
7,53 (d, 1H, H-6 ou H-7, J_{H5-H6} ou J_{H7-H8} = 7,33 Hz, en a du CH₃)
7,31 (dd, 1H, H-3', J_{H3'-H4'} = 3,66 Hz, J_{H3'-H5'} = 0,61 Hz)
6,60 (dd, 1H, H-4', J_{H3'-H4'} = 3,66 Hz, J_{H4'-H5'} = 1,83 Hz)
2,46 (s, 3H, CH₃)
**RMN du** ^{**13**}**C (CD**_{**2**}**Cl**_{**2**}**) : δ (ppm)**
176,28 (2C, C=O)
145,99 (1C, C-5')
134,64 (1C, C-6 ou C-7, en a du CH₃)
127,94 (1C, C-5 ou C-8)
126,83 (1C, C-5 ou C-8)
113,24 (1C, C-3')
113,18 (1C, C-4')
21, 74 (1C, CH₃)

### Exemples 62 et 63

### 4,9-Dihydro-4,9-dioxo-6-méthyl-2-phénylnaphto[2,3-d]thiazole et

### 4,9-Dihydro-4,9-dioxo-7-méthyl-2-phénylnaphto[2,3-d]thiazole

A une solution formée de 1,3 g (5,4 mmoles, 6,0 éq) de sulfure de sodium nonahydraté et de 3,2 ml d'une solution d'hydroxyde de sodium préalablement préparée à pH 10,7, on ajoute 0,5 g (0,9 mmole, 1,0 éq) d'un mélange de 2-amino-3-chloro-1,4-dihydro-1,4-dioxo 6-méthyl-naphtalène et de 2-amino-3-chloro-1,4-dihydro-1,4-dioxo-7-méthylnaphtalène. Cette solution, chauffée à 45°C devient bleue en 30 minutes. On ajoute alors au milieu réactionnel 184 *µ*l (1,8 mmole, 2,0 éq) de benzaldéhyde. Le mélange, chauffé à 55°C devient vert en 30 minutes. Après addition de 291 *µ*l (5,0 mmoles, 5,5 éq) d'acide acétique glacial, il se forme un précipité marron qui est filtré sur verre fritté puis lavé par du dichlorométhane. Le produit brut obtenu est purifié sur lit de silice (support : silice 6-35 *µ*m, d = 5 cm, h = 5 cm, dépôt solide, éluant : Heptane/Acétate d'éthyle, 90/10) pour fournir après évaporation des solvants 90 mg du mélange de 4,9-dihydro-4,9-dioxo-6-méthyl-2-phényl-naphto[2,3-d]-thiazole et 4,9-dihydro-4,9-dioxo-7-méthyl-2-phényl-naphto[2,3-d]thiazole sous forme de cristaux jaunes. Ces isomères sont séparés sur plaques préparatives (support : alumine, éluant : dichlorométhane/heptane, 70/30).
**Rdt :** 33 % (mélange des deux isomères)

### Produit le plus polaire

**Rf :** 0,50 (Dichlorométhane/Heptane, 70/30, alumine)
**SM (APcI+) :** m/z 306 (M⁺H⁺)
**RMN du** ^{**1**}**H (CD**_{**2**}**Cl**_{**2**}**) : δ (ppm)**
8,10 (d, 1H, H-5 OU H-8, J_{H5-H6} ou J_{H7-H8} = 7,63 Hz)
8,03 (m, 2H, H-2' et H-6')
7,93 (bs, 1H, H-5 ou H-8, en a du CH₃)
7,55 (d, 1H, H-6 ou H-7, (J_{H5-H6} ou J_{H7-H8} = 7,93 Hz)
7,46 (m, 3H, H-3', H-4', H5')
2,45 (s, 3H, CH₃)
**RMN du** ^{**13**}**C (CD**_{**2**}**Cl**_{**2**}**) : δ (ppm)**
135,36 (1C, C-6 ou C-7)
132,60 (1C, C-4')
129,65 (2C, C-2', C-6')
128,01 (1C, C-5 ou C-8)
127,90 (2C, C-3', C-5')
127,55 (1C, C-5 ou C-8)
21,93 (1C, CH₃)

### Produit le moins polaire

**Rf :** 0,62 (Dichlorométhane/Heptane, 70/30, alumine)
**SM (APcI+) :** m/z 306 (M+H⁺)
**RMN du** ^{**1**}**H (CD**_{**2**}**Cl**_{**2**}**) : δ (ppm)**
8,09 à 8,00 (m, 4H, H-5, H-8, H-2', H-6')
7,53 (d, 1H, H-6 ou H-7, J_{H5-H6} ou J_{H7-H8} = 7,93 Hz)
7,46 (m, 3H, H-3', H-4', H-5')
2,46 (s, 3H, CH₃)
**RMN du** ^{**13**}**C (CD**_{**2**}**Cl**_{**2**}**) : δ (ppm)**
134,98 (1C, C-6 ou C-7)
132,59 (1C, C-4')
129,66 (2C, C-2', C-6')
128,36 (1C, C-5 ou C-8)
127,90 (2C, C-3', C-5')
127,25 (1C, C-5 ou C-8)
22,0 (1C, CH₃)

### Exemples 64 et 65

### 4,9-Dihydro-4,9-dioxo-2-furyl-5-méthylnaphto[2,3-d]-thiazole et

### 4,9-Dihydro-4,9-dioxo-2-furyl-8-méthylnaphto[2,3-d]-thiazole

### Intermédiaires de synthèse

### 2,3-Dibromo-1,4-dihydro-1,4-dioxo-5-méthylnaphtalène

A 14,5 g (84 mmoles, 1 éq) de 1,4-dihydro-1,4-dioxo-5-méthylnaphtalène, on ajoute 200 ml de tétrachlorure de carbone puis 17,2 ml (337 mmoles, 4 éq) de brome. La solution devient rouge, on ajoute alors 22,94 g (168 mmoles, 2 éq) d'acétate de sodium. Après 96 heures au reflux, le milieu réactionnel est filtré, lavé avec du tétrachlorure de carbone et évaporé à sec ; Le produit est purifié sur cake (ø = 6,5 cm, hauteur = 5 cm, dépôt = solide, support = silice, éluant CH₂Cl₂) ; on obtient après évaporation à sec une pâte marron orangé. Une première cristallisation avec du dichlorométhane délivre 8,25 g de 2,3-dibromo-1,4-dihydro-1,4-dioxo-5-méthylnaphtalène sous forme de cristaux jaunes ; une seconde recristallisation avec de l'acétonitrile fournit 11,90 g de 2,3-dibromo-1,4-dihydro-1,4-dioxo-5-méthylnaphtalène sous forme de cristaux jaunes.
**Rdt :** 72 %
**Rf :** 0,70 (acétate d'éthyle/heptane, 50/50)
**SM (APcI-) :** m/z 328, 330, 332 (M-)
**RMN du** ^{**1**}**H (CDCl**_{**3**}**) : δ (ppm)**
8,11 (dd, 1H, H-8, J_{H7-H8} = 7,02 Hz, J_{H6-H8} = 1,53 Hz)
7,63 (m, 2H, H-6, H-7)
2,76 (s, 3H, CH₃)
**IR (KBr) : ν (cm**^{**-1**}**)**
1670 (C=O) ; 1570 (C = C)

### 2-amine-3-bromo-1,4-dihydro-1,4-dioxo-5-méthyl-naphtalène

### 2-amino-3-bromo-1,4-dihydro-1,4-dioxo-8-méthyl-naphtalène

A 8 g (24 mmoles, 1 éq) de 2,3-dibromo-1,4-dihydro-1,4-dioxonaphtalène, on ajoute 200 ml d'acide acétique glacial (3,5 mmoles, 6,85 éq) puis 2,52 g (38 mmoles) de nitrure de sodium en solution dans 17,5 ml d'eau. Après 12 h à 70°C, la solution devient rouge. Le milieu réactionnel est refroidi et évaporé à sec. Le produit est purifié sur un cake (ø = 5 cm, h = 5 cm, dépôt = solide, support = silice, éluant = gradient de concentration d'acétate d'éthyle/heptane 10/90 puis 20/80). On obtient 525 mg du mélange de 2-amino-3-bromo-1,4-dihydro-1,4-dioxo-5-méthylnaphtalène et de 2-amino-3-bromo-1,4-dihydro-1,4-dioxo-8-méthylnaphtalène sous forme de cristaux rouge-orangé.
**Rdt :** 8 %
**Rf :** 0,58 (Acétate d'éthyle/heptane, 50/50)
**SM (APcI-) :** m/z 264 266(M-)
**RMN du** ^{**1**}**H (CDCl**_{**3**}**) : δ (ppm)**
8,12 (d, 1H, H-5 ou H-8, J_{H7-H8} ou J_{H5-H6} = 7,32 Hz)
7,58 (t, 1H, H-6 ou H-7, J_{H5-H6} ou J_{H7-H8} = 7,63 Hz)
7,44 (d, 1H, H-6 ou H-7, J_{H5-H6} ou J_{H7-H8} = 7,63 Hz)
2,74 (s, 3H, CH₃)

### 4,9-Dihydro-4,9-dioxo-2-furyl-5-méthylnaphto[2,3-d]-thiazole et

### 4,9-Dihydro-4,9-dioxo-2-furyl-8-méthylnaphto[2,3-d]-thiazole

A une solution formée de 1,35 g (5,63 mmoles) de sulfure de sodium nonahydraté et de 3,38 ml (1,8.10⁻¹ mole) d'une solution d'hydroxyde de sodium préalablement préparée à pH = 9, on ajoute 0,25 g (0,93 mmole) d'un mélange de 2-amino-3-bromo-1,4-dihydro-1,4-dioxo-5-méthyl-naphtalène et de 2-amino-3-bromo-1,4-dihydro-1,4-dioxo-8-méthylnaphtalène. Cette suspension est chauffée à 45°C et devient bleu au bout de 40 minutes. On ajoute alors 156 ml (1,87 mmole) de 2-furaldéhyde puis au bout de 15 minutes, 102 *µ*l (1,78 mmole) d'acide acétique glacial à 55°C. Le milieu réactionnel devenu marron est alors extrait avec 2 x 100 ml de dichlorométhane, séché avec du sulfate de magnésium, filtré et évaporé à sec. Un cake d'alumine est alors réalisé (éluant : gradient de concentration : 50/50 à 70/30 en dichlorométhane/heptane.

On obtient alors 51 mg (18 %) d'un mélange de 4,9-dihydro-4,9-dioxo-2-furyl-5-méthylnaphto[2,3-d]thiazole et 4,9-dihydro-4,9-dioxo-2-furyl-8-méthylnaphto[2,3-d]-thiazole. La séparation des isomères sur plaque préparative (support : alumine, éluant : dichlorométhane/heptane) fournit un produit plus polaire et un produit moins polaire.

### Produit le moins polaire

**Rf :** 0,61 (dichlorométhane/heptane, 70/30, alumine)
**SM (APcI+) :** m/z 296 (M+H+)
**RMN du** ^{**1**}**H (CD**_{**2**}**Cl**_{**2**}**) : δ (ppm)**
8,07 (d, 1H, H-5 ou H-8 en g du CH₃, J_{H5-H6} ou J_{H7-H8} = 9,55 Hz)
7,58 (m, 3H, H-6 et H-7 en α et β du CH₃ et H-5')
7,32 (d, 1H, H-3', J_{H3'-H4'} = 3,32 Hz)
6,60 (dd, 1H, H-4', J_{H3'-H4'} = 3,1 Hz, J_{H4'-H5'} = 1,2 Hz)
2,77 (s, 3H, CH₃)
**RMN du** ^{**13**}**C (CD**_{**2**}**Cl**_{**2**}**) :** δ **(ppm)**
145,24 (1C, C-5')
137,82-132,24 (2C, C-6 et C-7)
124,90 (1C, C-5 ou C-8)
112,54 et 112,44 (2C, C-3' et C-4')

### Produit le plus polaire

**Rf :** 0,53 (dichlorométhane/heptane, 70/30, alumine)
**SM (APcI+) :** m/z 296 (M+H+)
**RMN du** ^{**1**}**H (CD**_{**2**}**Cl**_{**2**}**) : δ (ppm)**
8,07 (d, 1H, H-5 ou H-8, J_{H5-H6} ou J_{H7-H8} = 4,98 Hz, J_{H5-H7} ou J_{H6-H8} = 1,5 Hz)
7,60 (m, 3H, H-6 et H-7 en α et β du CH₃ et H-5')
7,32 (d, 1H, H-3', J_{H3'-H4'} = 3,73 Hz)
6,45 (dd, 1H, H-4', J_{H3'-H4'} = 3,32 Hz, J_{H4'-H5'} = 1,66 Hz)
2,77 (s, 3H, CH₃)
**RMN du** ^{**13**}**C (CD**_{**2**}**Cl**_{**2**}**) : δ (ppm)**
189,69 et 184,57 (2C, C-4 et C-9)
145,38 (1C, C-5')
129,29 (1C, C-6 ou C-7 en β du CH₃)
126,04-132,24 (1C, C-6 ou C-7 en a du CH₃)
123,45 (1C, C-5 ou C-8 en γ du CH₃)
113,67 et 113,57 (2C, C-3' et C-4')
23,39 (1C, CH₃)

### Exemples 66 et 67

### 4,9-Dihydro-4,9-dioxo-5-méthyl-2-phénylnaphto[2,3-d]thiazole et

### 4,9-Dihydro-4,9-dioxo-8-méthyl-2-phénylnaphto[2,3-d]thiazole

A une solution formée de 1,35 g (5,63 mmoles, 6 éq) de sulfure de sodium nonahydraté et 3,38 ml (890 mmoles, 193 éq) d'une solution d'hydroxyde de sodium préalablement préparée à pH = 9, on ajoute 0,25 g (0,93 mmole) du mélange de 2-amino-3-bromo-1,4-dihydro-1,4-dioxo-6-méthylnaphtalène et de 2-amino-3-bromo-1,4-dihydro-1,4-dioxo-7-méthylnaphtalène. Cette suspension chauffée à 45°C devient bleue au bout de 30 minutes. On ajoute 191 ml (1,87 mmole, 2 éq) de benzaldéhyde puis après 15 min 102 ml (1,78 mmole) d'acide acétique glacial à 55°C. Le milieu réactionnel devenu marron est alors extrait avec 3 fois 100 ml de dichlorométhane, lavé jusqu'à neutralité et séché sur du sulfate de magnésium pour fournir 38 mg (13 %) de produit brut. Après séparation des deux isomères sur plaque préparative (support alumine, éluant dichlorométhane/heptane 70/30) on obtient deux produits le 4,9-dihydro-4,9-dioxo-5-méthyl-2-phénylnaphto [2,3-d]-thiazole et le 4,9-dihydro-4,9-dioxo-8-méthyl-2-phényl-naphto[2,3-d]thiazole.

### Produit le moins polaire

**Rf :** 0,58 (dichlorométhane/heptane, 70/30, alumine)
**SM (IE) :** m/z 305(M+)
**RMN du** ^{**1**}**H (CD**_{**2**}**Cl**_{**2**}**) : δ (ppm)**
8,07 (m, 3H, H-2', H-6', H-5 ou H-8)
7,61 (m, 2H, H-6, H-7)
7,49 (m, 2H, H-3', H-5')
7,30 (m, 1H, H-4')
2,76 (s, 3H, CH,)

### Produit le plus polaire

**Rf :** 0,51 (dichlorométhane/heptane, 70/30, alumine)
**SM (IE) :** m/z 305(M+)
**RMN du** ^{**1**}**H (CD**_{**2**}**Cl**_{**2**}**) : δ (ppm)**
8,07 (m, 3H, H-2', H-6', H-5 ou H-8 en g du CH₃)
7,60 (m, 2H, H-6, H-7)
7,49 (m, 2H, H-3', H-5')
7,30 (m, 1H, H-4')
2,77 (s, 3H, CH₃)

### Exemple a

### 4,9-Dihydro-4,9-dioxo-2-méthyl-1H-napht[2,3-d]-imidazole

**Référence : C.A.** 67 97905t
**Rdt :** 76 %
**F :** > 260°C
**Rf :** 0,44 (CH₂Cl₂/Méthanol, 97/3)
**SM (I.E.) :** m/z 212 (M+.)
**RMN du** ^{**1**}**H (DMSO d**_{**6**}**) :** δ **(ppm)**
13,74 (s, 1H, NH)
8,05 (dd, 2H, H-5, H-8, J_{H5-H6} = J_{H7-H8} = 8,85 Hz; J_{H5-H7} = J_{H6-H8} = 1,73 Hz)
7,82 (m, 2H, H-6, H-7)
2,45 (S, 3H, CH₃)
**RMN du** ^{**13**}**C (DMSO d**_{**6**}**) : δ (ppm)**
178,15 ; 176,53 (2C, C-4, C-9)
153,80 (1C, C-2)
137,14 (1C, C-3a)
133,98, 133,99 (2C, C-6, C-7)
133,27, 133,10, 132,84 (3C, C-8a, C-9a, C-4a)
126,82 (2C, C-5, C-8)
14,01 (1C, CH₃)
**IR (KBr) : ν (cm**^{**-1**}**)**
de 3134 à 2897 (NH) ; 1678, 1672 (C=O)

### Exemple b

### 4,9-Dihydro-4,9-dioxo-2-méthyl-1-phényl-1H-napht-[2,3-d]imidazole

**Référence :** C.A. 67 97905t
**Rdt :** 72 %
**F :** 242°C
**Rf :** 0,43 (CH₂Cl₂/Méthanol, 99/1)
**SM (I.E.) :** m/z 288 (M+.)
**RMN du** ^{**1**}**H (DMSO, d**_{**6**}**) : δ (ppm)**
8,23 (d, 1H, H-5 ou H-8, J_{H5-H6} ou J_{H7-H8} = 6,71 Hz)
7,90 (d, 1H, H-5 ou H-8, J_{H-5-H6} ou J_{H7-H8} = 6,71 Hz)
7,68 (m, 3H, H-3', H-4', H-5')
7,60 (m, 2H, H-6, H-7)
7,37 (m, 2H, H-2', H-6')
2,40 (s, 3H, CH₃)
**RMN du** ^{**13**}**C (CDCl**_{**3**}**) : δ (ppm)**
179,13, 175,03 (2C, C-4, C-9)
153,66 (1C, C-2)
143,14 (1C, C-1')
135,11 (1C, C-3a)
133,68, 133,51 (2C, C-6, C-7)
133,15, 133,01, 132,63 (3C, C-8a, C-9a, C-4a)
129,95, 129,69 (3C, C-3', C-4', C-5')
126,93, 126,76, 126,43 (4C, C-2', C-6', C-5, C-8)
13,77 (1C, CH₃)
**IR (KBr) : ν (cm**^{**-1**}**)**
1674, 1663 (C=O)

### Exemple c

### Sulfate de 4,9-Dihydro-4,9-dioxo-2-méthyl-1-phényl-1H-napht[2,3-d]imidazole

**Référence : C.A.** 68 8764b
**Rdt :** 47 %
**F :** > 260°C
**Rf :** 0,53 (CH₂Cl₂/Méthanol, 97,5/2,5)
**RMN du** ^{**1**}**H (DMSO, d**_{**6**}**) : δ (ppm)**
8,11 (dd, 1H, H-5 ou H-8, J_{H5-H6} ou J_{H7-H8} = 8,85 Hz; J_{H5-H7} ou J_{H6-H8} = 1,73 Hz)
8,00 (dd, 1H, H-5 ou H-8, J_{H5-H6} ou J_{H7-H8} = 8,85 Hz; J_{H5-H7} ou J_{H6-H8} = 1,73 Hz)
7,94 (m, 2H, H-6, H-7)
7,82 (m, 2H, H-2', H-6')
7,51 (m, 3H, H-3', H-4', H-5')
5,54 (s, 1H, NH+)
2,30 (s, 3H, CH₃)
**IR (KBr) :** ν **(cm**^{**-1**}**)**
3414 à 2400 (bande large NH+) ; 1736, 1681 (C=O)

### Exemple d

### 4,9-Dihydro-4,9-dioxo-1,2-diméthyl-1H-napht[2,3-d]-imidazole

**Référence : C.A.** 66 104957w
**Rdt :** 70 %
**F : >** 253°C
**Rf :** 0,49 (CH₂Cl₂/Méthanol, 98/2)
**SM (I.E.) :** m/z 226 (M+.)
**RMN du** ^{**1**}**H (CDCl**_{**3**}**) : δ (ppm)**
8,21, 8,09 (2m, 2H, H-5, H-8)
7,70 (m, 2H, H-6, H-7)
4,01 (s, 3H, CH₃)
2,56 (s, 3H, CH₃)
**RMN du** ^{**13**}**C (CDCl**_{**3**}**) : δ (ppm)**
179,22, 178,44 (2C, C-4, C-9)
154,46 (1C, C-2)
134,18, 134,11 (2C, C-6, C-7)
133,37, 133,17 (2C, C-4a, C-8a)
127,37, 126,75 (2C, C-5, C-8)
32,74 (1C, CH₃)
13,64 (1C, CH₃)
**IR (KBr) : ν (cm**^{**-1**}**)**
1674 (C=O)

### Exemple e

### 4,9-Dihydro-4,9-dioxo-2-phényl-1H-napht[2,3-d]-imidazole

**Référence : C.A.** 68 8764b
**Rdt :** 34 %
**F : >** 260°C
**Rf :** 0,51 (CH₂Cl₂/Acétate d'éthyle, 90/10)
**SM (I.E.) :** m/z 274 (M+.)
**RMN du** ^{**1**}**H (DMSO d**_{**6**}**) : δ (ppm)**
14,40 (s, 1H, NH)
8,26 (m, 2H, H-5, H-8)
8,12 (m, 2H, H-2', H-6')
7,87 (m, 2H, H-6, H-7)
7,54 (m, 3H, H-3', H-4', H-5')
**RMN** ^{**13**}**C (DMSO d**_{**6**}**) : δ (ppm)**
179,13, 175,03 (2C, C-4, C-9)
152,60 (1C, C-2)
133,89 (2C, C-6, C-7)
132,60 (2C, C-9a, C-3a)
130,00 (2C, C-4a, C-8a)
129,03 (3C, C-3', C-4', C-5')
126,82, 126,32 (4C, C-5, C-8, C-2', C-6')
**IR (KBr) : ν (cm**^{**-1**}**)**
3232 (NH) ; 1681, 1664 (C=O)

### Exemple f

### 4,9-Dihydro-4,9-dioxo-2-phényl-napht[2,3-d]oxazole

**Référence : C.A.** 87 53134z
**Rdt :** 75 %
**F :** > 260°C
**Rf :** 0,60 (CH₂Cl₂/Heptane, 80/20)
**SM (I.E.) :** m/z 275 (M+.)
**RMN du** ^{**1**}**H (CDCl**_{**3**}**) : δ (ppm)**
8,33 (d, 2H, H-2', H-6', J_{H2'-H3'} = J_{H5'-H6'} = 6,71 Hz)
8,27 (m, 2H, H-5, H-8)
7,82 (m, 2H, H-6, H-7)
7,58 (m, 3H, H-3', H-4', H-5')
**RMN du** ^{**13**}**C (CDCl**_{**3**}**) : δ (ppm)**
178,50, 173,05 (2C, C-4, C-9)
167,76 (1C, C-2)
134,52, 132,99 (2C, C-6, C-7)
129,21 (3C, C-3', C-4', C-5')
128,33 (2C, C-2', C-6')
127,50, 127,04 (2C, C-5, C-8)
**IR (KBr) : ν (cm**^{**-1**}**)**
1693, 1678 (C=O)

### Exemple g

### 4,9-Dihydro-4,9-dioxo-2-(4-méthylphényl)-napht[2,3 d]oxazole

**Référence : C.A.** 87 53134z
**Rdt :** 44 %
**F :** > 260°C
**Rf :** 0,50 (CH₂Cl₂)
**SM (I.E.) :** m/z 289 (M+.)
**RMN du** ^{**1**}**H (CDCl**_{**3**}**) :** δ **(ppm)**
8,27 (m, 2H, H-5, H-8)
8,23 (d, 2H, H-2', H-6', J_{H2'-H3'} = J_{H5'-H6'} = 8,24 Hz)
7,81 (m, 2H, H-6, H-7)
7,38 (m, 2H, H-3', H-5')
2,46 (s, 3H, CH₃)
**RMN du** ^{**13**}**C (CDCl**_{**3**}**) : δ (ppm)**
178,77, 173,80 (2C, C-4, C-9)
165,51 (1C, C-2)
143,95 (1C, C-3a)
135,80 (1C, C-4')
134,29, 132,12 (3C, C-1', C-6, C-7)
131,70, 131,38 (2C, C-4a, C-8a)
129,93, 128,28 (4C, C-2', C-3', C-5', C-6')
127,44, 126,99 (2C, C-5, C-8)
122,41 (1C, C-1')
21,81 (1C, CH₃)
**IR (KBr) : ν (cm**^{**-1**}**)**
1668, 1678 (C=O)

### Exemple h

### 4,9-Dihydro-4,9-dioxo-2-méthyl-naphto[2,3-d]thiazole

**Référence : C.A.** 120 270267z
**Rdt :** 11 %
**F** : > 260°C
**Rf :** 0,41 (CH₂Cl₂/Méthanol, 99/1)
**SM (I.E.) :** m/z 229 (M+.)
**RMN du** ^{**1**}**H (CDCl**_{**3**}**) : δ (ppm)**
8,33, 8,21(2dd, 2H, H-5, H-8, J_{H5-H6} = J_{H7-H8} = 8,85 Hz)
7,80 (m, 2H, H-6, H-7)
2,91 (s, 3H, CH₃)
**RMN du** ^{**13**}**C (CDCl**_{**3**}**) : δ (ppm)**
177,95, 176,95 (2C, C-4, C-9)
173,86 (1C, C-2)
153,10 (1C, C-3a)
142,14 (1C, C-9a)
133,90, 133,51 (2C, C-6, C-7)
132,06, 131,72 (2C, C-4a, C-8a)
127,29, 126,47 (2C, C-5, C-8)
19,83 (1C, CH₃)
**IR (KBr) : ν (cm**^{**-1**}**)**
1677, 1655 (C=O)

### Exemple i

### 2-Amino-4,9-dihydro-4,9-dioxo-naphto[2,3-d]thiazole

**Référence : C.A.** 120 270267z
**Rdt :** 96 %
**F :** > 260°C
**Rf :** 0,24 (CH₂Cl₂/Méthanol, 96/4)
**SM (I.E.) :** m/z 230 (MH+.)
**RMN du** ^{**1**}**H (DMSO d**_{**6**}**) : δ (ppm)**
8,56 (s, 2H, NH₂)
8,03 (m, 2H, H-5, H-8)
7,83 (m, 2H, H-6, H-7)
**RMN du** ^{**13**}**C (DMSO d**_{**6**}**) :** δ **(ppm)**
178,04, 177,31 (2C, C-4, C-9)
173,45 (1C, C-2)
154,61 (1C, C-3a)
145,95 (1C, C-9a)
134,34, 134,03 (2C, C-6, C-7)
133,18, 132,24 (2C, C-4a, C-8a)
126,99, 125,97 (2C, C-5, C-8)
**IR (KBr) : ν (cm**^{**-1**}**)**
3460, 3420 (NH₂), 1690, 1660 (C=O)

### Exemple j

### 4,9-Dihydro-4,9-dioxo-2-phényl-naphto[2,3-d]thiazole

**Référence : C.A.** 67 11450f
**Rdt :** 68 %
**F :** 249°C
**Rf :** 0,45 (CH₂Cl₂)
**SM (I.E.) :** m/z 291 (M+.)
**RMN du** ^{**1**}**H (CDCl**_{**3**}**) : δ (ppm)**
8,30, 8,21 (2dd, 2H, H-5, H-8, J_{H5-H6} = J_{H7-H8} = 8,85 Hz, J_{H6-H8} = J_{H5-H7} = 1.73 Hz)
8,14 (m, 2H, H-2' , H-6')
7,83 (m, 2H, H-6, H-7)
7,56 (m, 3H, H-3', H-4', H-5')
**RMN du** ^{**13**}**C (CDCl**_{**3**}**) : δ (ppm)**
178,45, 172,67 (2C, C-4, C-9)
134,82, 134,44 (2C, C-6, C-7)
133,50, 133,24, 132,74, 132,01 (4C, C-9a, C-1', C-4a, C-8a)
129,75, 128,01 (5C, C-2', C-3', C-4', C-5', C-6')
127,94, 127,18 (2C, C-5, C-8)
**IR (KBr) : ν (cm**^{**-1**}**)**
1675, 1660 (C=O)

### Exemple k

### 4,9-Dihydro-4,9-dioxo-2-(2-pyridyl)-naphto[2,3-d]-thiazole

**Référence : C.A.** 109 130788t
**Rdt :** 75 %
**F :** > 260°C
**Rf :** 0,60 (CH₂Cl₂/Méthanol, 97/3)
**SM (I.E.) :** m/z 292 (M+.)
**RMN du** ^{**1**}**H (CDCl**_{**3**}**) : δ (ppm)**
8,69 (d, 1H, H-6', J_{H5'-H6'} = 5,50 Hz)
8,47 (d,1H, H-3', J_{H3'-H4'} = 5,50 Hz)
8,36, 8,26 (2dd, 2H, H-5, H-8, J_{H5-H6} = J_{H7-H8} = 8,85 Hz, J_{H5-H7} = J_{H6-H8} = 1,73 Hz)
7,82 (m, 3H, H-4', H-6, H-7)
7,46 (m, 1H, H-5')
**RMN du** ^{**13**}**C (CDCl**_{**3**}**) : δ (ppm)**
178,17, 177,43 (2C, C-4, C-9)
158,76 (1C, Cquat)
151,11 (1C, C-6')
147,86 (1C, C-4')
134,77, 134,31 (2C, C-6, C-7)
133,50, 132,74 (2C, C-4a, C-8a)
128,04, 127,16 (2C, C-5, C-8)
121,15 (1C, C-3')
118,45 (1C, C-5')
**IR (KBr) : ν (cm**^{**-1**}**)**
1688, 1667 (C=O)

### Exemple l

### 4,9-Dihydro-4,9-dioxo-2-(4-pyridyl)-naphto[2,3-d]-thiazole

**Référence : C.A.** 109 130788t
**Rdt :** 75 %
**F :** > 260°C
**Rf :** 0,30 (CH₂Cl₂/Méthanol, 97/3)
**SM (I.E.) :** m/z 292 (M+.)
**RMN du** ^{**1**}**H (CDCl**_{**3**}**) : δ (ppm)**
8,84 (d, 2H, H-2', H-6', J_{H2'-H3'} = J_{H5'-H6'} = 5,50 Hz)
8,39, 8,26 (dd, 2H, H-5, H-8, J_{H5-H6} = J_{H7-H8} = 8,85 Hz, J_{H6-H8} = J_{H5-H7} = 1,73 Hz)
7,99 (d, 2H, H-3', H-5', J_{H2'-H3'} = J_{H5'-H6'} = 5,50 Hz)
7,84 (m, 2H, H-6, H-7)
**RMN du** ^{**13**}**C (CDCl**_{**3**}**) : δ (ppm)**
178,17, 172,50 (2C, C-4, C-9)
151,11 (2C, C-2', C-6')
134,77, 134,31 (2C, C-6, C-7)
133,50, 132,74, 132,01 (4C, C-9a, C-4', C-4a, C-8a)
128,04, 127,16 (2C, C-5, C-8)
121,04 (2C, C-3', C-5')
**IR (KBr) : ν (cm**^{**-1**}**)**
1688, 1667 (C=O)

### Exemple m

### 4,9-Dihydro-4,9-dioxo-2-(2-pyrrolyl)-naphto[2,3-d]-thiazole

**Référence** : C.A. 109 130788t
**Rdt** : 17 %
**F :** 208°C (dec)
**Rf :** 0,44 (CH₂Cl₂/Ethanol, 99/1)
**SM (I.E.) :** m/z 280(M+.)
**RMN du** ^{**1**}**H (CD**_{**2**}**Cl**_{**2**}**) : δ (ppm)**
9,85 (1s, 1H, NH)
8,21, 8,17 (2dd, 2H, H-5, H-8, J_{H5-H6} = J_{H7-H8} = 8,85 Hz, J_{H5-H7} = J_{H6-H8} = 1,73 Hz)
7,79 (m, 2H, H-6, H-7)
7,07 (m, 1H, H-5')
6,96 (m, 1H, H-3')
6,35 (m, 1H, H-4')
**IR (KBr) :** ν **(cm**^{**-1**}**)**
3286 (NH) ; 1676, 1647 (C=O)

### Exemple n

### 4,9-Dihydro-4,9-dioxo-2-(2-furyl)-naphto[2,3-d]-thiazole

**Référence : C.A.** 67 11450f
**Rdt :** 48 %
**F :** > 260°C
**Rf :** 0,55 (CH₂Cl₂/Méthanol, 99,5/0,5)
**SM (I.E.) :** m/z 281 (MH+.)
**RMN du** ^{**1**}**H (CDCl**_{**3**}**) : δ (ppm)**
8,37 (dd, 1H, H-5 ou H- 8 , J_{H5-H6} ou J_{H7-H8} = 8,85 Hz , J_{H5-H7} ou J_{H6-H8} = 1,73 Hz)
8,23 (m, 1H, H-5 ou H-8)
7,81 (m, 2H, H-6, H-7)
7,65 (d, 1H, H-5', J_{H4'-H5'} = 1,97 Hz)
7,46 (d, 1H, H-3', J_{H3'-H4'} = 3,94 Hz)
6,65 (dd, 1H, H-4', J_{H3'-H4'} = 3,94 Hz, J_{H4'-H5'} = 1,97 Hz)
RMN du ¹³C (CDCl₃) : δ (ppm)
178,25, 177,90 (2C, C-4, C-9)
163,94 (1C, C-2)
155,25 (1C, C-2')
148,00 (1C, C-3a)
145,93 (1C, C-5')
140,62 (1C, C-9a)
134,34, 134,09 (2C, C-6, C-7)
133,13, 132,68 (2C, C-4a, C-8a)
127,83, 126,91 (2C, C-5, C-8)
113,80 (1C, C-3')
113,34 (1C, C-4')
**IR (KBr) : ν (cm**^{**-1**}**)**
1683, 1658 (C=O)

### Propriétés pharmacologiques:

L'étude des composés de la présente invention et de leurs sels éventuels a démontré qu'ils possèdent diverses propriétés pharmacologiques. Ainsi, la plupart des composés sont sélectivement veinotoniques, n'affectant le système artériel qu'à des concentrations largement supérieures à celles actives sur les veines, excepté certaines artères, en particulier cérébrales (carotides, basilaires...). Les composés ne montrent aucune affinité ou une affinité très faible pour la majorité des récepteurs pharmacologiques membranaires connus. Par ailleurs, ils augmentent la résistance capillaire, diminuent l'hyperperméabilité vasculaire induite par certains agents inflammatoires. Ces propriétés sont mises en évidence chez les mammifères tels que les hamsters, rats, cobayes et lapins, dans des conditions in vitro (vaisseaux ou réseaux vasculaires isolés) et in vivo.

Pour les études in vitro, les composés sont solubilisés en solution aqueuse pure ou contenant du DMSO (diméthylsulfoxyde).

Pour les études in vivo, ils sont administrés par voie intraveineuse ou intrapéritonéale sous forme de solution aqueuse contenant ou non du DMSO, ou par voie orale en suspension dans la carboxyméthylcellulose à 1 %, administrés à l'aide d'une sonde de gavage sous un volume de 10 ml/kg.

### Modèles d'études pharmacologiques

### Effets contractiles :

Les effets contractiles sont mesurés *in vitro* dans des conditions statiques sur des anneaux vasculaires à capacitance ou à résistance de veines saphènes, fémorales, jugulaires, mésentériques, caves... et sur artères fémorales, carotides, basilaires, mésentériques, aorte thoracique ou abdominale... de rat (Wistar, 200 à 250 g), lapin (New Zealand, 2 à 2,5 kg), cobaye ( Dunkin Hartley 250 à 300 g).

Les anneaux sont placés en chambre d'organe isolé (25 ml pour les vaisseaux à capacitance et 2,5 ml pour les vaisseaux à résistance selon Mulvany), maintenus dans des conditions isométriques par deux fils rigides insérés à l'intérieur du vaisseau, en évitant d'endommager l'endothélium. Les vaisseaux sont baignés par une solution de Krebs modifiée (en mM: NaCl = 118 ; KCl = 4,6 ; CaCl₂ = 2,5; MgSO₄ = 1,2 ; KH₂PO₄ = 1,17; NaHCO₃ = 25 ; glucose = 11), aérée en permanence par un mélange gazeux à 95 % O₂ et 5 % CO₂, à pH = 7,4 et thermostatés à 37°C. Les anneaux sont amenés à leur point optimal de la relation tension-longueur.

Les tensions développées génèrent un signal électrique par l'intermédiaire d'un capteur de force (pont de Wheastone) . Ce signal est amplifié avant d'être soit visualisé sur enregistreur Kipp & Zonen , soit digitalisé pour être traité par ordinateur (IOS, EMKA). Les études pharmacologiques sont réalisées après quelques stimulations contractiles préliminaires standardisées par une solution dépolarisante (hyperpotassiques obtenues en remplacant du Na Cl par du KCl en quantités équimolaires), rinçages et périodes d'équilibration en solution physiologique pure. La présence d'endothélium est vérifiée par la relaxation induite par des concentrations croissantes d'acetylcholine après stabilisation d'une précontraction vasculaire.

Les forces de contraction développées par les anneaux vasculaires en réponse aux différents composés sont étudiées sur des vaisseaux quiescents ou stimulés électriquement (5-8 Hz), par une solution "physiologique" dépolarisante hyperpotassique (KCl : 20, 40 mM), par la nor-adrénaline (concentrations croissantes), la sérotonine (concentrations croissantes)...

Les contractions sont exprimées en mg force ou en pourcentage de la contraction maximale à la dépolarisation par une solution "physiologique" hyperpotassique.

Les effets contractiles sont également mesurés *in vitro* dans des conditions dynamiques de flux, par la pression développée par des réseaux vasculaires perfusés à débit constant. Au niveau mésentérique, la veinosélectivité est étudiée sur le modèle de double perfusion simultanée et séparée des réseaux artériels et veineux, modèle développé par T. WARNER (British J. Pharmacol. 1990, 99, 427-433). La séparation des deux réseaux est réalisée en coupant les vaisseaux et les tissus le long de la bordure intestinale. Les réseaux sont perfusés à 2 ml.min⁻¹ par une solution de Krebs (37,5°C) aérée à 95 % O₂ et 5 % CO₂.

*In vivo*, les pressions artérielles et veineuses sont mesurées chez l'animal anesthésié, dans des conditions basales et après arrêt circulatoire provoqué par le gonflement d'un cathéter à ballonnet introduit au niveau de l'oreillette droite. Lors de l'arrêt cardiaque, le tonus veineux (pression moyenne circulatoire de remplissage à volume sanguin constant) est calculé à partir des pressions veineuses et artérielles mesurées à l'équilibre et corrigées en fonction des différences relatives de compliance entre ces deux réseaux (SAMAR & COLEMAN, Am. J. Physiol. 1978, 234:H94-100; YAMAMOTO et al., Am. J. Physiol. 1980, 238:H823-828).

Chez l'animal éveillé, les pressions artérielles sont mesurées selon la méthode classique dérivée de Riva Rocci, par analyse de l'onde acoustique transmise au niveau artériel et transformée par un transducteur piezo céramique placé sur la queue du rat, en aval d'un manchon gonflé automatiquement par un générateur de pression

Au niveau microcirculatoire, les variations de section veinulaire et artériolaire sont étudiées *in vivo* dans le modèle de la chambre cutanée dorsale de hamster vigile, après enregistrement vidéomicroscopique (microscope Leitz Ergolux équipé d'une source halogène pour l'éclairage et une caméra vidéo CDD noir et blanc HPR 610) et analyse informatique (logiciel Visicap, Pack ICAP) des images.

Après anesthésie au pentobarbital sodique (60 mg/kg en i.p.), le dos de l'animal est tondu et épilé de manière à pouvoir placer une chambre d'observation (Prof. GEBHARD, Heidelberg) sur la peau de dos. Les deux parties de la chambre sont cousues après avoir enlevé avec précaution les épaisseurs cutanées pouvant gêner l'observation. Un cathéter jugulaire est placé pour l'administration i.v. des produits, 48 heures après l'opération.

### Effets sur l'hyperperméabilité capillaire induite :

La perméabilité vasculaire est étudiée *in vivo* par la mesure de l'extravasation d'albumine dont la quantité est déterminée grâce à un colorant liant l'albumine (Bleu Evans).

L'hyperperméabilité est induite par injection intradermique d'une solution d'histamine, de bradykinine ou de zymosan.

La technique est dérivée de celle décrite par BEACH & STEINETZ, J. Pharmacol. Exp. Therap., 1961, 131: 400-406.

Les rats (Wistars, 200 à 230 g) sont tondus sur leur paroi abdominale une heure avant le début de l'expérimentation. Le produit à tester est injecté par voie i.p. ou per os 1 heure à 4 heures avant le sacrifice. Les rats sont anesthésiés par un mélange d'halothane. Ensuite, ils reçoivent une injection intradermique sur l'abdomen de 0,10 ou 0,15 ml (soit pour l'histamine 6,7 ou 10 microgrammes) d'agent inflammatoire et une injection intraveineuse d'un ml d'une solution de bleu Evans à 0,5 % dans la veine du pénis. Ces injections sont réalisées 30 minutes avant l'euthanasie.

30 minutes après ces deux injections, les rats sont euthanasiés par dislocation cervicale.

A l'endroit de l'injection de l'agent inflammatoire, la peau est découpée et placée dans des tubes en verre à col rôdé contenant 3 ml d'acide chlorhydrique fumant. La digestion de la peau est réalisée par un contact d'au moins une heure au bain marie à 37°C. Trois ml de chlorure de benzalkonium à 12,8 % sont ensuite ajoutés. Après avoir laissé reposer durant trente minutes, 7 ml de dichlorométhane sont additionnés. Les tubes sont agités périodiquement pendant une heure. La phase aqueuse est éliminée par aspiration et la phase organique "dichlorométhane" est filtrée. Les densités optiques sont quantifiées par spectrophotométrie d'absorption à une longueur d'onde de 620 nm, contre un blanc contenant uniquement le dichlorométhane.

Les moyennes des densités optiques des différents lots d'animaux traités ou témoins sont calculées, puis un pourcentage de variation des valeurs correspondant aux animaux traités par rapport à celles des animaux témoins est calculé.

L'effet des composés sur l'hyperperméabilité induite par des agents inflammatoires, comme l'histamine et la bradykinine est également étudié après injection intraveineuse par bolus dans le modèle de chambre cutanée dorsale de hamster et selon la méthode développée par GIMENO et al., décrite précédemment («A new technique using intravital videomicroscopy for macromolecular permeability measurement, 18° Congrès Européen de microcirculation, Rome 1994») par videomicroscopie et analyse d'images par quantification de la répartition de la fluorescence intra et extravasculaire du marqueur fluorescent (FITC-Dextran) injecté en bolus par le cathéter jugulaire (63 mg/kg pour un volume défini à 1 ml/kg). Le microscope est équipé d'une source fluorescente et d'une combinaison de filtres (excitation dans le bleu 450-490 nm et filtre d'arrêt 515 nm).

### Effets sur la résistance capillaire :

L'augmentation de la résistance capillaire est appréciée par la modification de l'index pétéchial (pression négative induisant l'extravasation des érythrocytes), mesuré par une méthode dérivée de l'angiosterromètre de Parrot.

L'étude se réalise sur des rats mâles Wistar d'un poids moyen de 200 g (âgés d'environ six semaines). La région du bas du dos est rasée puis épilée à l'aide d'une pâte à base d'un dérivé de l'acide thioglycolique et d'hydroxyde de calcium. Après environ trente minutes, la peau est abondamment rinçée et séchée.

Le jour de l'étude, les rats sont maintenus sans contrainte. Une dépression de 80 mm de mercure est appliquée. Si les pétéchies (extravasation d'érythrocytes) ne sont pas apparues dans les 15 secondes, la dépression est augmentée par pallier en maintenant la ventouse au même endroit.

La dépression minimale pour laquelle apparaissent les pétéchies exprime, en mm de mercure, la valeur de résistance capillaire de base (avant tout traitement). Deux mesures sont réalisées pour chaque essai à des emplacements différents du dos.

Les rats sont traités par voie orale. Après un temps déterminé (généralement 2, 4, 6 heures) suivant le traitement, le test est renouvellé sur des plages de peau différentes, jusqu'à l'apparition des pétéchies, procurant un nouvel index de dépression. Toutes les mesures se font en aveugle.

Un pourcentage de variation des résistances capillaires des animaux traités par rapport à leur résistance capillaire de base est calculé pour chaque composé étudié, à chaque temps de traitement et comparé avec le groupe témoin (excipient seul) ou le groupe de référence.

### Effets sur la pleuresie induite chez le rat :

L'activité antiinflammatoire des composés est également étudiée par la mesure de l'inhibition de l'oedème et de la migration leucocytaire après induction d'une pleuresie chez le rat par injection de carraghénine dans la cavité pleurale (ALMEIDA et al., J. Pharmacol. Exp. Therap., 1980, 214:74).

Les rats sont traités per os par les composés 2 heures avant l'injection de carraghénine et deux et quatre heures après cette injection. Après un temps déterminé (6 heures) suivant l'induction de la pleuresie, les rats sont euthanasiés et le liquide pleural récupéré par aspiration et son volume est mesuré. Les cellules leucocytaires sont comptées par la technique dite "cell counter". Les résultats sont exprimés en nombre de leucocytes dans l'exudat rapporté à 100 g de poids de l'animal et comparés à ceux du lot témoin.

### Effets contre le choc septique :

L'activité dans le choc septique est étudiée chez le rat après induction du choc par une injection intraveineuse en bolus d'une endotoxine lipopolysaccharidique (LPS : 15 mg/kg) d'E. Coli, méthode voisine de celle décrite par TERASHITA et al., Eur. J. Pharmacol., 1985, 109: 257-261. Les pressions artérielles sont mesurées en fonction du temps et comparativement entre groupes traités et groupes témoins (excipient seul). Les composés sont administré par voie i.v. ou per os, 5 minutes ou deux heures respectivement avant l'injection de LPS.

### Exemples d'effets pharmacologiques :

Les composés de l'invention et leurs sels éventuels augmentent sélectivement dans la majorité des cas la contraction des veines animales produite par la noradrénaline, par la stimulation électrique ou par une solution hyperpotassique dépolarisante.

A titre illustratif, figure l'effet contractile de différents composés sur la veine saphène de lapin précontractée par une solution "physiologique" dépolarisante de concentration potassique égale à 40 mM; l'effet maximal produit par chaque composé est exprimé en pourcentage de la contraction maximale induite par des solutions hyperpotassiques dépolarisantes et en valeur d'ED₅₀) :

| **Composés** | **Emax ( % Contr.max.)** | **ED**_{**50**} **(nM)** |
|---|---|---|
| **Exemple f** | 15 ± 1 | 21 |
| **Exemple j** | 16 ± 6 | 30 |
| **Exemple k** | 26 ± 9 | 70 |
| **Exemple h** | 29 ± 6 | 86 |
| **Exemple i** | 18 ± 1 | 90 |
| **Exemple n** | 24 ± 3 | 110 |
| **Exemple 3** | 17 ± 6 | 36 |
| **Exemple 4** | 29 ± 11 | 42 |
| **Exemple 7** | 17 ± 2 | 36 |
| **Exemple 13** | 21 ± 3 | 57 |
| **Exemple 18** | 39 ± 6 | 88 |
| **Exemple 19** | 20 ± 8 | 75 |
| **Exemple 20** | 24 ± 4 | 110 |
| **Exemple 28** | 29 ± 2 | 37 |
| **Exemple 38** | 12 ± 1 | 160 |
| **Exemple 59** | 18 ± 4 | 53 |
| **Exemple 57** | 22 ± 3 | 41 |

A titre illustratif, l'administration orale de certains composés de l'invention et de leurs sels éventuels augmente la résistance capillaire du rat à des doses comprises généralement entre 0,01 et 5 mg/kg :

| **Composés** | | **Effet à 4 heures (en % du témoin)** | **Effet à 6 heures (en % du témoin)** |
|---|---|---|---|
| **Exemple 3** | 5 mg/kg | 27 | 18 |
| **Exemple 18** | 5 mg/kg | 25 | 28 |
| **Exemple 21** | 5 mg/kg | 10 | 22 |
| **Exemple f** | 0,1 mg/kg | 7 | 17 |
| **Exemple h** | 0,1 mg/kg | 19 | 19 |
| **Exemple 1** | 0,1 mg/kg | 37 | 35 |
| **Exemple n*** | 0,1 mg/kg | 45 | 45 |
| **Exemple 2** | 0,1 mg/kg | 25 | 28 |
| **Exemple 10** | 0,1 mg/kg | 25 | 46 |
| **Exemple 15** | 0,1 mg/kg | 20 | 27 |
| **Exemple 28**** | 0,1 mg/kg | 10 | 13 |

| | | | |
|---|---|---|---|
| * (granulométrie :-0,5 à 0,6 mm) | | | |
| ** (granulométrie : 0,6 à 0,7 mm) | | | |

A titre illustratif, l'administration orale de certains composés de l'invention et de leurs sels éventuels réduit l'hyperperméabilité inflammatoire induite par le zymosan chez le rat à des doses comprises entre 0,1 et 5 mg/kg :

| **Composés** | | **Effet à 2 heures (en % du témoin)** | **Effet à 4 heures (en % du témoin)** |
|---|---|---|---|
| **Exemple 1** | 5 mg/kg | -28 | -28 |
| **Exemple n** | 5 mg/kg | 0 | -21 |
| **Exemple 21** | 5 mg/kg | -22 | -21 |
| **Exemple 28** | 5 mg/kg | -29 | 0 |
| **Exemple f** | 0,1 mg/kg | -15 | -22 |
| **Exemple j** | 0,1 mg/kg | -14 | -32 |
| **Exemple 3** | 0,1 mg/kg | -22 | -26 |
| **Exemple 14** | 0,1 mg/kg | -31 | -15 |
| **Exemple 17** | 0,1 mg/kg | -15 | -23 |
| **Exemple 25** | 0,1 mg/kg | -14 | -17 |
| **Exemple 26** | 0,1 mg/kg | -14 | -24 |
| **Exemple 38** | 0,1 mg/kg | -12 | - 3 |
| **Exemple 59** | 0,1 mg/kg | -13 | +21 |

Par ailleurs, les composés de l'invention et leurs sels éventuels sont très peu toxiques. Par exemple, après une administration orale unique de 500 mg/kg chez la souris, aucun effet toxique observable et aucune mortalité n'est observée pour la majorité des composés en particulier pour **l'Exemple f (1 g/kg), l'Exemple j, l'Exemple h (1 g/kg ; diarrhées), l'Exemple n (1 g/kg ; urines rouges), l'Exemple 3 (légères diarrhées), l'Exemple 5, l'Exemple 6, l'Exemple 13 (1 g/kg).**

La plupart des composés se révèlent non cytotoxiques (la viabilité cellulaire étant mesurée par quantification de l'incorporation cellulaire de rouge neutre) jusqu'à des concentrations égales à leur solubilité en milieu aqueux sur des lignées cellulaires fibroblastiques de souris (L929), en particulier **l'Exemple f, l'Exemple** j, **l'Exemple 3, l'Exemple 4, l'Exemple 20, l'Exemple 21...**

Parmi les composés actifs de l'invention, on retient tout particulièrement **l'Exemple n.**

**L'Exemple n** par exemple potentialise sélectivement la réponse contractile de la veine saphène de lapin à la noradrénaline (valeur d'ED50 réduite d'un facteur dix et Emax augmenté de 30 %), à la stimulation électrique (augmentation de 200 % à 0,3 micromolaire), à la sérotonine, en plus des effets contractiles observés en présence d'une réponse à une solution hyperpotassique. Dans ces conditions hyperpotassiques dépolarisantes, le produit contracte par exemple la veine jugulaire de lapin (ED₅₀ = 16 nM), de rat (ED₅₀ = 50 nM), le réseau veineux mésentérique perfusé de rat (ED₅₀ = 300 nM), l'artère carotide de rat (ED₅₀ = 300 nM), de lapin (ED₅₀ = 120 nM), l'artère basilaire de lapin.

Sur le modèle de chambre cutanée dorsale chez le hamster, **l'Exemple n** (28 microgrammes/kg injectés en bolus i.v.) réduit le diamètre veinulaire mais non artériolaire après injection par voie i.v. d'histamine (1 mg/kg) et réduit l'hyperperméabilité vasculaire induite par celle-ci.

L'effet per os de **l'Exemple n** sur la résistance capillaire du rat est dose-dépendant sur une large gamme de doses et sa durée d'action , d'au moins six heures, est corrélée avec ses concentrations plasmatiques mesurées.

Après deux heures, l'administration orale de 0,1 mg/ kg de produit de l'Exemple n diminue l'hyperper-méabilité induite par l'histamine de 26 pourcent par rapport au groupe témoin chez le rat.

L'administration orale de 3 x 5 mg de ce produit diminue significativement le volume d'exudat pulmonaire après induction d'une pleuresie par la carraghénine chez le rat.

Administré en i.v. bolus à la dose de 28 microgrammes/kg 5 minutes avant induction du choc septique, le produit de l'Exemple n augmente la pression artérielle moyenne de 20 mm de mercure par rapport au groupe de rats témoins.

Le produit de l'exemple n n'affecte pas la pression artérielle du rat anesthésié après injection en i.v. en bolus au moins jusqu'à 28 microgrammes/kg, ni celle du rat vigile après administration orale de 0,1-5-50 mg/kg.

Ce qui précède montre que les composés de l'invention et leurs sels éventuels peuvent être utilisés en thérapeutique humaine et animale. Ils sont en particulier indiqués dans l'insuffisance veineuse fonctionnelle, organique et les pathologies hémorroidaires par leurs composantes vasculaires et antiinflammatoires, ainsi que dans les affections typiquement inflammatoires et dans les états de chocs constitués par une chute importante de la pression artérielle. Dans ce dernier cas, une amélioration du retour veineux est susceptible de maintenir le débit cardiaque et dès lors, par voie de conséquence, la pression artérielle.

L'insuffisance veineuse fonctionnelle se caractérise par une dilatation et une hyperdistensibilité des veines superficielles des membres inférieurs, oedèmes, paresthésies à type d'impatience, de jambe sans repos. Ce type de pathologie peut évoluer vers l'insuffisance veineuse organique caractérisée par le développement de varices, d'incontinences valvulaires, voire vers la phlébothrombose et les troubles trophiques aboutissant à des lésions ulcéreuses.

Dans cette pathologie veineuse, une composante inflammatoire s'installe dans les premiers stades et se manifeste plus clairement dans les stades avancés.

Par leurs effets veinoconstricteurs, antiinflammatoires en particulier sur l'hyperperméabilité vasculaire et leurs effets contractiles sur les artères cérébrales, les composés de l'invention et leurs sels éventuels sont également indiqués dans la migraine.

La présente invention comprend donc l'utilisation des composés mentionnés ci-dessus et de leurs sels éventuels, comme substances actives pour la préparation de médicaments et de compositions pharmaceutiques à usage humain et vétérinaire, comprenant au moins un desdits composés et sels en association avec un support ou diluant physiologiquement acceptable.

La forme de ces médicaments et compositions pharmaceutiques dépendra bien évidemment de la voie d'administration souhaitée notamment orale, parentérale, topique (cutanée) et rectale, et ils peuvent être formulés selon les techniques classiques avec mise en oeuvre de supports et véhicules usuels.

Ainsi, dans le cas d'une administration par voie orale, ils peuvent se présenter sous la forme de comprimés, tablettes, gélules, solutions, sirops, émulsions, suspensions, poudre, granulés, capsule molle, lyophilisat, microcapsule, microgranule.

Les comprimés, tablettes et gélules contiennent la substance active conjointement avec un diluant (par exemple lactose, dextrose, sucrose, mannitol, maltitol, xylitol, sorbitol ou cellulose), un lubrifiant (par exemple silice, talc ou stéarate), un liant (par exemple amidon, méthylcellulose ou gomme arabique), un agent de désintégration (alginate par exemple) et ils sont fabriqués par des techniques connues par exemple de mélange, de granulation, de pastillage, d'enrobage, de compression, etc.

Les sirops peuvent contenir, à titre de support, glycérol, mannitol et/ou sorbitol. Les solutions et suspensions peuvent comprendre de l'eau et d'autres solvants physiologiquement compatibles et un support tel qu'une gomme naturelle, de la gélose, de l'alginate de sodium ou de l'alcool polyvinylique.

Pour l'administration par voie parentérale, les médicaments et compositions peuvent prendre la forme de solutions, d'émulsions ou de suspensions comprenant la substance active et un support ou solvant approprié tel que l'eau stérile ou des solutions salines isotoniques stériles.

Pour l'application cutanée, les médicaments et compositions peuvent prendre la forme d'onguent, crème ou gel, sous forme d'émulsion ou de suspension, solution, mousse, poudre.

Pour l'application rectale, les médicaments et compositions peuvent prendre la forme de capsule, crème, émulsion, gel , mousse, pommade, suppositoire.

## Revendications

1. Utilisation de dérivés tricycliques et de leurs sels acceptables du point de vue pharmaceutique répondant à la formule générale : 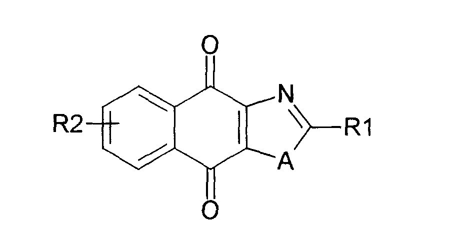 dans laquelle, Me désignant un radical méthyle et Ph désignant un radical phényle :
A est soit :
- un atome de soufre ou d'oxygène, ou
- un radical NR3 où R3 est un atome d'hydrogène, un radical alkyle en C1 à C5, ou un phényle,
R1 est soit :
- un radical alkyle en C1 à C5,
- un radical phényle,
- un radical R4NH où R4 est un atome d'hydrogène, ou
- un radical du groupe comprenant : 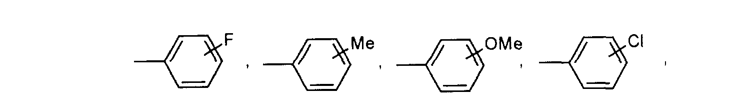 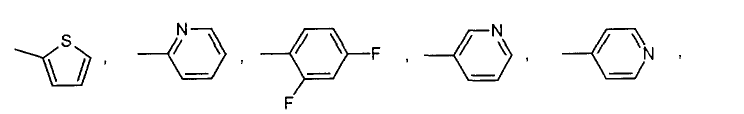 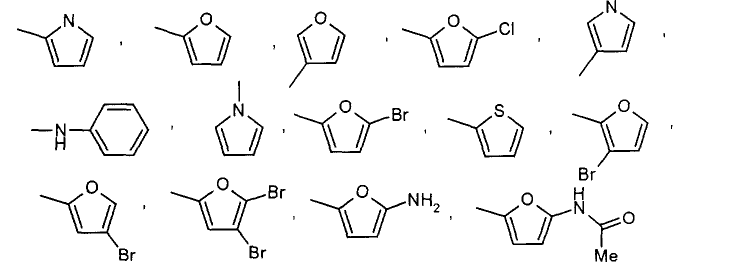 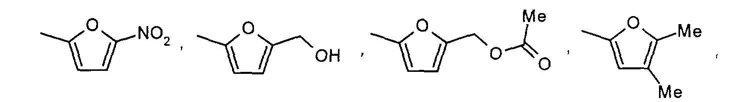 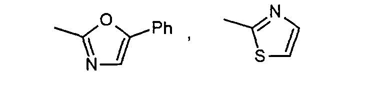
R2 est soit :
- H,
- OH,
- un halogène,
- un radical alkyle en C1 à C5,
- un atome d'oxygène substitué par un radical alkyle en C1 à C5, ou
- un radical NR5R5', où R5 et R5' sont indépendamment l'un de l'autre un atome d'hydrogène ou un atome d'oxygène,
pour l'obtention d'un médicament destiné au traitement de maladies liées à une altération de la fonction veineuse et/ou à l'oedème inflammatoire.

2. Le sulfate de 4,9-dihydro-4,9-dioxo-1,2-diméthyl-1H-napht[2,3-d]imidazole.

3. Le 4,9-dihydro-4,9-dioxo-2-(2-fluorophényl)-1H-napht[2,3-d]imidazole.

4. Le 4,9-dihydro-4,9-dioxo-2-(2-fluorophényl)-napht[2,3-d]oxazole.

5. Le 4,9-dihydro-4,9-dioxo-2-(3-fluorophényl)-napht[2,3-d]oxazole.

6. Le 4,9-dihydro-4,9-dioxo-2-(4-fluorophényl)-napht[2,3-d]oxazole.

7. Le 4,9-dihydro-4,9-dioxo-2-(2-méthylphényl)-napht[2,3-d]oxazole.

8. Le 4,9-dihydro-4,9-dioxo-2-(3-méthylphényl)-napht[2,3-d]oxazole.

9. Le 4,9-dihydro-4,9-dioxo-2-(4-méthoxyphényl)-napht[2,3-d] oxazole.

10. Le 2-(2-chlorophényl) -4,9-dihydro-4,9-dioxo-napht [2,3-d]oxazole.

11. Le 2-(4-chlorophényl)-4,9-dihydro-4,9-dioxo-napht[2,3-d]oxazole.

12. Le 4,9-dihydro-4,9-dioxo-2-(2-thiényl)-napht [2,3-d]oxazole.

13. Le 4,9-dihydro-4,9-dioxo-2-(2-fluorophényl)-naphto[2,3-d]thiazole.

14. Le 4,9-dihydro-4,9-dioxo-2-(3-fluorophényl)-naphto[2,3-d]thiazole.

15. Le 4,9-dihydro-4,9-dioxo-2-(4-fluorophényl) -naphto[2,3-d] thiazole.

16. Le 2-(2,4-difluorophényl)-4,9-dihydro-4,9-dioxo-naphto [2,3-d]thiazole.

17. Le 4,9-dihydro-4,9-dioxo-2-(3-pyridyl)-naphto[2,3-d]thiazole.

18. Le sulfate de 4,9-dihydro-4,9-dioxo-2-(4-pyridyl)-naphto[2,3-d] thiazole.

19. Le 4,9-dihydro-4,9-dioxo-2-(3-furyl)-naphto[2,3-d]thiazole.

20. Le 2-(5-chlorofuran-2-yl)-4,9-dihydro-4,9-dioxo-naphto[2,3-d]thiazole.

21. Le 4,9-dihydro-4,9-dioxo-2-(2-thiényl)-naphto[2,3-d]thiazole.

22. Le 4,9-dihydro-4,9-dioxo-2-(3-thiényl)-naphto[2,3-d]thiazole.

23. Le 4,9-dihydro-4,9-dioxo-2-phénylamino-naphto[2,3-d]thiazole.

24. Le 4,9-dihydro-4,9-dioxo-8-méthoxy-2-phényl-naphto[2,3-d]thiazole.

25. Le 4,9-dihydro-4,9-dioxo-5-méthoxy-2-phényl-naphto[2,3-d]thiazole.

26. Le 4,9-dihydro-4,9-dioxo-7-méthoxy-2-phényl-naphto[2,3-d]thiazole.

27. Le 4,9-dihydro-4,9-dioxo-6-méthoxy-2-phényl-naphto[2,3-d]thiazole.

28. Le 4,9-dihydro-4,9-dioxo-8-hydroxy-2-phényl-naphto[2,3-d]thiazole.

29. Le 4,9-dihydro-4,9-dioxo-2-(1-pyrrolyl)-naphto[2,3-d] thiazole.

30. Le 2-(5-bromo-furan-2-yl) -4,9-dihydro-4,9-dioxo-naphto[2,3-d]thiazole.

31. Le 2-(4,5-dibromofuran-2-yl) -4,9-dihydro-4,9-dioxo-naphto[2,3-d] -thiazole.

32. Le 2-(3-bromo-furan-2-yl) -4,9-dihydro-4,9-dioxonaphto[2,3-d] thiazole.

33. Le 2-(4-bromofuran-2-yl)-4,9-dihydro-4,9-dioxonaphto[2,3-d]thiazole.

34. Le 4,9-dihydro-4,9-dioxo-2-(5-nitro-furan-2-yl)naphto[2,3-d]thiazole.

35. Le 2-(5-amino-furan-2-yl)-4,9-dihydro-4,9-dioxonaphto[2,3-d]thiazole.

36. Le 2-(5-acétamidofuran-2-yl)-4,9-dihydro-4,9-dioxonaphto[2,3-d]thiazole.

37. Le 4,9-dihydro-4,9-dioxo-2-(5-hydroxyméthylfuran-2-yl)naphto[2,3-d]thiazole.

38. Le 2-(5-acétoxy-méthylfuran-2-yl)-4,9-dihydro-4,9-dioxo-naphto[2,3-d]-thiazole.

39. Le 4,9-dihydro-4,9-dioxo-2-(5-méthyl-2-furyl)naphto[2,3-d]thiazole.

40. Le 4,9-dihydro-2-(4,5-diméthyl-2-furyl)4,9-dioxonaphto[2,3-d]thiazole.

41. Le 4,9-dihydro-4,9-dioxo-2-(5-phényl-2-oxazolyl)naphto[2,3-d]thiazole.

42. Le 4,9-dihydro-4,9-dioxo-2-(2-thiazolyl)naphto[2,3-d]thiazole.

43. Le 4,9-dihydro-4,9-dioxo-6-fluoro-2-(2-furyl)naphto[2,3-d]thiazole.

44. Le 4,9-dihydro-4,9-dioxo-7-fluoro-2-(2-furyl)naphto[2,3-d]thiazole.

45. Le 4,9-dihydro-4,9-dioxo-6-fluoro-2-phénylnaphto[2,3-d]thiazole.

46. Le 4,9-dihydro-4,9-dioxo-7-fluoro-2-phénylnaphto[2,3-d]thiazole.

47. Le 4,9-dihydro-4,9-dioxo-6-fluoro-2-(5-méthyl-2-furyl)naphto[2,3-d]thiazole.

48. Le 4,9-dihydro-4,9-dioxo-7-fluoro-2-(5-méthyl-2-furyl)naphto[2,3-d]thiazole.

49. Le 4,9-dihydro-4,9-dioxo-6-fluoro-2-(4-fluorophényl)naphto[2,3-d]thiazole.

50. Le 4,9-dihydro-4,9-dioxo-7-fluoro-2-(4-fluorophényl)naphto[2,3-d]thiazole.

51. Le 4,9-dihydro-4,9-dioxo-6-fluoro-2-(4-méthylphényl)naphto[2,3-d]thiazole.

52. Le 4,9-dihydro-4,9-dioxo-7-fluoro-2-(4-méthylphényl)naphto[2,3-d]thiazole.

53. Le 4,9-dihydro-4,9-dioxo-5-fluoro-2-(2-furyl)naphto[2,3-d]thiazole.

54. Le 4,9-dihydro-4,9-dioxo-8-fluoro-2-(2-furyl)naphto [2,3-d]thiazole.

55. Le 6-chloro-4,9-dihydro-4,9-dioxo-2-(2-furyl)-naphto[2,3-d]thiazole.

56. Le 7-chloro-4,9-dihydro-4,9-dioxo-2-(2-furyl)-naphto[2,3-d]thiazole.

57. Le 4,9-dihydro-4,9-dioxo-2-(2-furyl)-5-méthoxynaphto[2,3-d]thiazole.

58. Le 4,9-dihydro-4,9-dioxo-2-(2-furyl) -8-méthoxynaphto [2,3-d]thiazole.

59. Le 4,9-dihydro-4,9-dioxo-2-(2-furyl)-5-hydroxynaphto[2,3-d]thiazole.

60. Le 4,9-dihydro-4,9-dioxo-2-(2-furyl)-8-hydroxynaphto[2,3-d]thiazole.

61. Le 4,9-dihydro-4,9-dioxo-2-(2-furyl)-6-méthoxynaphto[2,3-d]thiazole.

62. Le 4, 9-dihydro-4, 9-dioxo-2- (2-furyl)-7-méthoxynaphto[2,3-d]thiazole.

63. Le 4,9-dihydro-4,9-dioxo-2-furyl-6-méthylnaphto[2,3-d] thiazole.

64. Le 4,9-dihydro-4,9-dioxo-2-furyl-7-méthylnaphto[2,3-d]thiazole.

65. Le 4, 9-dihydro-4, 9-dioxo-6-méthyl-2-phénylnaphto [2,3-d] thiazole.

66. Le 4,9-dihydro-4,9-dioxo-7-methyl-2-phénylnaphto[2,3-d]thiazole.

67. Le 4,9-dihydro-4,9-dioxo-2-furyl-5-méthylnaphto[2,3-d]thiazole.

68. Le 4,9-dihydro-4,9-dioxo-2-furyl-8-méthylnaphto[2,3-d]thiazole.

69. Le 4,9-dihydro-4,9-dioxo-5-méthyl-2-phénylnaphto[2,3-d]thiazole.

70. Le 4,9-dihydro-4,9-dioxo-8-méthyl-2-phénylnaphto[2,3-d]thiazole.

71. Utilisation selon la revendication 1, pour l'obtention d'un médicament destiné au traitement de l'insuffisance veineuse fonctionnelle et organique.

72. Utilisation selon la revendication 1, pour l'obtention d'un médicament destiné au traitement des pathologies hémorroïdaires.

73. Utilisation selon la revendication 1, pour l'obtention d'un médicament destiné au traitement de la migraine.

74. Utilisation selon la revendication 1, pour l'obtention d'un médicament destiné au traitement des inflammations ostéoarticulaires dermatologiques et cardiovasculaires.

75. Utilisation selon la revendication 1, pour l'obtention d'un médicament destiné au traitement des états de chocs constitués par une chute importante de la pression artérielle plus particulièrement dans les états de chocs septiques.

## Patentansprüche

1. Verwendung tricyclischer Derivate und ihrer pharmazeutisch verträglichen Salze der allgemeinen Formel: 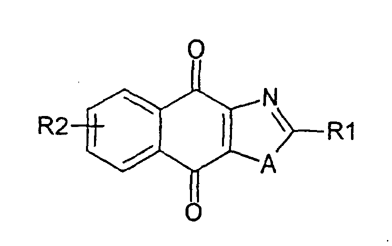 in der Me einen Methylrest und Ph einen Phenylrest bedeuten:
A
- ein Schwefel- oder Sauerstoffatom, oder
- einen Rest NR3 bedeutet, wobei R3 ein Wasserstoffatom, ein C1- bis C5-Alkylrest oder ein Phenylrest ist.
R1:
- einen C1- bis C5-Alkylrest,
- einen Phenylrest,
- einen Rest R4NH, wobei R4 ein Wasserstoffatom ist, oder
- einen Rest aus der Gruppe: 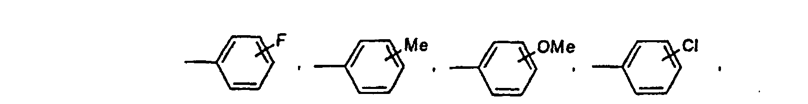 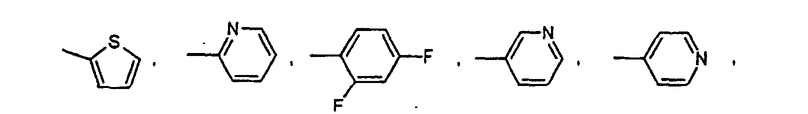 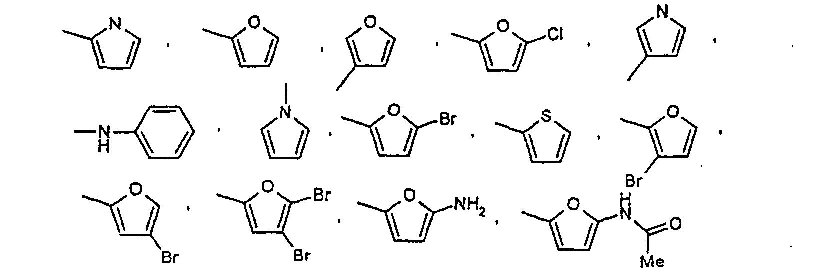 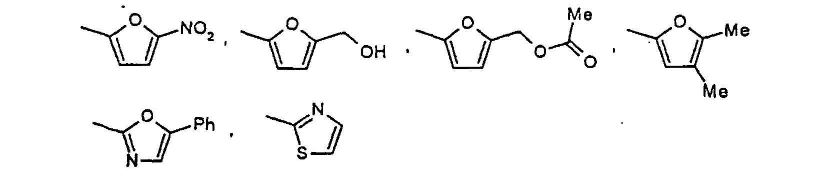 bedeutet,
R2 die Bedeutung hat:
- H,
- OH,
- Halogen,
- einen C1- bis C5-Alkylrest,
- ein Sauerstoffatom, substituiert durch einen C1- bis C5-Alkylrest, oder
- einen Rest NR5R5', wobei R5 und R5' unabhängig voneinander ein Wasserstoffatom oder ein Sauerstoffatom bedeuten,
zur Herstellung eines Medikaments zur Behandlung von Krankheiten, die mit einer Veränderung der Venenfunktion und/oder entzündlichen Ödemen einhergehen.

2. 4,9-Dihydro-4,9-dioxo-1,2-dimethyl-1H-naphth[2,3-d]imidazol-sulfat.

3. 4,9-Dihydro-4,9-dioxo-2-(2-fluorphenyl)-1H-naphth[2,3-d]imidazol.

4. 4,9-Dihydro-4,9-dioxo-2-(2-fluorphenyl)-naphth[2,3-d]oxazol.

5. 4,9-Dihydro-4,9-dioxo-2-(3-fluorphenyl)-naphth[2,3-d]oxazol.

6. 4,9-Dihydro-4,9-dioxo-2-(4-fluorphenyl)-naphth[2,3-d]oxazol.

7. 4,9-Dihydro-4,9-dioxo-2-(2-methylphenyl)-naphth[2,3-d]oxazol.

8. 4,9-Dihydro-4,9-dioxo-2-(3- methylphenyl)-naphth[2,3-d]oxazol.

9. 4,9-Dihydro-4,9-dioxo-2-(4- methoxyphenyl)-naphth[2,3-d]oxazol.

10. 2-(2-Chlorphenyl)-4,9-dihydro-4,9-dioxo-naphth[2,3-d]oxazol.

11. 2-(4-Chlorphenyl)-4,9-dihydro-4,9-dioxo-naphth[2,3-d]oxazol.

12. 4,9-Dihydro-4,9-dioxo-2-(2-thienyl)-naphth[2,3-d]oxazol.

13. 4,9-Dihydro-4,9-dioxo-2-(2-fluorphenyl)-naphtho[2,3-d]thiazol.

14. 4,9-Dihydro-4,9-dioxo-2-(3-fluorphenyl)-naphtho[2,3-d]thiazol.

15. 4,9-Dihydro-4,9-dioxo-2-(4-fluorphenyl)-naphtho[2,3-d]thiazol.

16. 2-(2,4-Difluorphenyl)-4,9-dihydro-4,9-dioxo-naphtho[2,3-d]thiazol.

17. 4,9-Dihydro-4,9-dioxo-2-(3-pyridyl)-naphtho[2,3-d]thiazol.

18. 4,9-Dihydro-4,9-dioxo-2-(4-pyridyl)-naphtho[2,3-d]thiazol-sulfat.

19. 4,9-Dihydro-4,9-dioxo-2-(3-furyl)-naphtho[2,3-d]thiazol.

20. 2-(5-Chlorfuran-2-yl)-4,9-dihydro-4,9-dioxo-naphtho[2,3-d]thiazol.

21. 4,9-Dihydro-4,9-dioxo-2-(2-thienyl)-naphtho[2,3-d]thiazol.

22. 4,9-Dihydro-4,9-dioxo-2-(3-thienyl)-naphtho[2,3-d]thiazol.

23. 4,9-Dihydro-4,9-dioxo-2-phenylamino-naphtho[2,3-d]thiazol.

24. 4,9-Dihydro-4,9-dioxo-8-methoxy-2-phenyl-naphtho[2,3-d]thiazol.

25. 4,9-Dihydro-4,9-dioxo-5-methoxy-2-phenyl,naphtho[2,3-d]thiazol.

26. 4,9-Dihydro-4,9-dioxo-7-methoxy-2-phenyl-naphtho[2,3-d]thiazol.

27. 4,9-Dihydro-4,9-dioxo-6-methoxy-2-phenyl-naphtho[2,3-d]thiazol.

28. 4,9-Dihydro-4,9-dioxo-8-hydroxy-2-phenyl-naphtho[2,3-d]thiazol.

29. 4,9-Dihydro-4,9-dioxo-2-(1-pyrrolyl)-naphtho[2,3-d]thiazol.

30. 2-(5-Bromfuran-2-yl)-4,9-dihydro-4,9-dioxo-naphtho[2,3-d]thiazol.

31. 2-(4,5-Dibromfuran-2-yl)-4,9-dihydro-4,9-dioxo-naphtho[2,3-d]thiazol

32. 2-(3-Bromfuran-2-yl)-4,9-dihydro-4,9-dioxo-naphtho[2,3-d]thiazol.

33. 2-(4-Bromfuran-2-yl)-4,9-dihydro-4,9-dioxo-naphtho[2,3-d]thiazol.

34. 4,9-Dihydro-4,9-dioxo-2-(5-nitrofuran-2-yl)naphtho[2,3-d]thiazol.

35. 2-(5-Aminofuran-2-yl)-4,9-dihydro-4,9-dioxo-naphtho[2,3-d]thiazol.

36. 2-(5-Acetamidofuran-2-yl)-4,9-dihydro-4,9-dioxo-naphtho[2,3-d]thiazol.

37. 4,9-Dihydro-4,9-dioxo-2-(5-hydroxymethylfuran-2-yl)naphtho[2,3-d]thiazol.

38. 2-(5-Acetoxy-methylfuran-2-yl)-4,9-dihydro-4,9-dioxo-naphtho[2,3-d]thiazol.

39. 4,9-Dihydro-4,9-dioxo-2-(5-methyl-2-furyl)naphtho[2,3-d]thiazol.

40. 4,9-Dihydro-2-(4,5-dimethyl-2-furyl)-4,9-dioxo-naphtho[2,3-d]thiazol.

41. 4,9-Dihydro-4,9-dioxo-2-(5-phenyl-2-oxazolyl)naphtho[2,3-d]thiazol.

42. 4,9-Dihydro-4,9-dioxo-2-(2-thiazolyl)naphtho[2,3-d]thiazol.

43. 4,9-Dihydro-4,9-dioxo-6-fluor-2-(2-furyl)naphtho[2,3-d]thiazol.

44. 4,9-Dihydro-4,9-dioxo-7-fluor-2-(2-furyl)naphtho[2,3,d]thiazol.

45. 4,9-Dihydro-4,9-dioxo-6-fluor-2-phenylnaphtho[2,3-d]thiazol.

46. 4,9-Dihydro-4,9-dioxo-7-fluor-2-phenylnaphtho[2,3-d]thiazol.

47. 4,9-Dihydro-4,9-dioxo-6-fluor-2-(5-methyl-2-fitryl)naphtho[2,3-d]thiazol.

48. 4,9-Dihydro-4,9-dioxo-7-fluor-2-(5-methyl-2-furyl)naphtho[2,3-d]thiazol.

49. 4,9-Dihydro-4,9-dioxo-6-fluor-2-(4-fluorphenyl)naphtho[2,3-d]thiazol.

50. 4,9-Dihydro-4,9-dioxo-7-fluor-2-(4-fluorphenyl)naphtho[2,3-d]thiazol.

51. 4,9-Dihydro-4,9-dioxo-6-fluor-2-(4-methylphenyl)naphtho[2,3-d]thiazol.

52. 4,9-Dihydro-4,9-dioxo-7-fluor-2-(4-methylphenyl)naphtho[2,3-d]thiazol.

53. 4,9-Dihydro-4,9-dioxo-5-fluor-2-(2-furyl)naphtho[2,3-d]thiazol.

54. 4,9-Dihydro-4,9-dioxo-8-fluor-2-(2-furyl)naphtho[2,3-d]thiazol.

55. 6-Chlor-4,9-dihydro-4,9-dioxo-2-(2-furyl)naphtho[2,3-d]thiazol.

56. 7-Chlor-4,9-dihydro-4,9-dioxo-2-(2-furyl)naphtho[2,3-d]thiazol.

57. 4,9-Dihydro-4,9-dioxo-2-(2-furyl)-5-methoxynaphtho[2,3-d]thiazol.

58. 4,9-Dihydro-4,9-dioxo-2-(2-furyl)-8-methoxynaphtho[2,3-d]thiazol.

59. 4,9-Dihydro-4,9-dioxo-2-(2-furyl)-5-hydroxynaphtho[2,3-d]thiazol.

60. 4,9-Dihydro-4,9-dioxo-2-(2-furyl)-8-hydroxynaphtho[2,3-d]thiazol.

61. 4,9-Dihydro-4,9-dioxo-2-(2-furyl)-6-methoxynaphtho[2,3-d]thiazol.

62. 4,9-Dihydro-4,9-dioxo-2-(2-furyl)-7-methoxynaphtho[2,3-d]thiazol.

63. 4,9-Dihydro-4,9-dioxo-2-furyl-6-methylnaphtho[2,3-d]thiazol.

64. 4,9-Dihydro-4,9-dioxo-2-furyl-7-methylnaphtho[2,3-d]thiazol.

65. 4,9-Dihydro-4,9-dioxo-6-methyl-2-phenylnaphtho[2,3-d]thiazol.

66. 4,9-Dihydro-4,9-dioxo-7-methyl-2-phenylnaphtho[2,3-d]thiazol.

67. 4,9-Dihydro-4,9-dioxo-2-furyl-5-methylnaphtho[2,3-d]thiazol.

68. 4,9-Dihydro-4,9-dioxo-2-furyl-8-methylnaphtho[2,3-d]thiazol.

69. 4,9-Dihydro-4,9-dioxo-5-methyl-2-phenylnaphtho[2,3-d]thiazol.

70. 4,9-Dihydro-4,9-dioxo-8-methyl-2-phenylnaphtho[2,3-d]thiazol.

71. Verwendung nach Anspruch 1 zur Herstellung eines Medikaments zur Behandlung funktioneller und organischer Veneninsuffizienz.

72. Verwendung nach Anspruch 1 zur Herstellung eines Medikaments zur Behandlung hämorrhoidaler Krankheitsbilder.

73. Verwendung nach Anspruch 1 zur Herstellung eines Medikaments zur Behandlung von Migräne.

74. Verwendung nach Anspruch 1 zur Herstellung eines Medikaments zur Behandlung osteoartikulärer dermatologischer und kardiovaskulärer Entzündungen.

75. Verwendung nach Anspruch 1 zur Herstellung eines Medikaments zur Behandlung von durch arteriellen Druckabfall hervorgerufenen Schockzuständen, insbesondere von septischen Schockzuständen.

## Claims

1. Use of tricyclic derivatives and their pharmaceutically acceptable salts having the general formula: 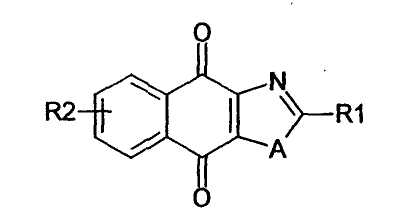 in which, Me standing for a methyl radical and Ph standing for a phenyl radical:
A is either:
- a sulfur or oxygen atom, or
- an NR3 radical where R3 is a hydrogen atom, a C1-C5 alkyl radical, or a phenyl;
R1 is either:
- a C1-C5 alkyl radical,
- a phenyl radical,
- a R4NH radical where R4 is a hydrogen atom, or
- a radical from the group comprising: 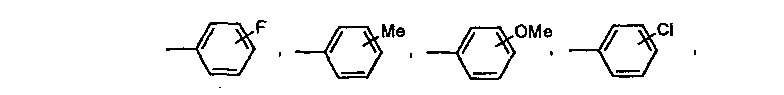 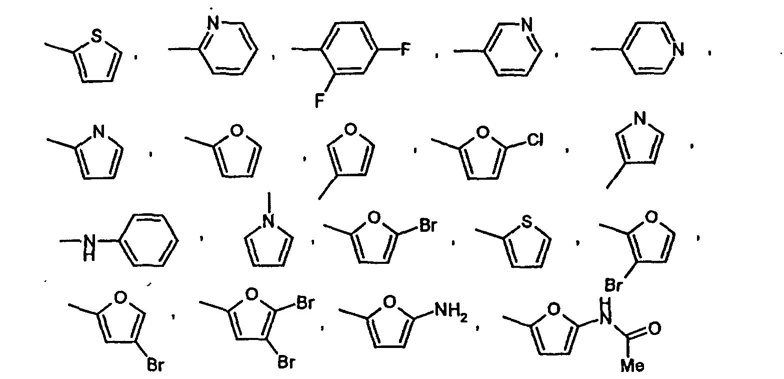 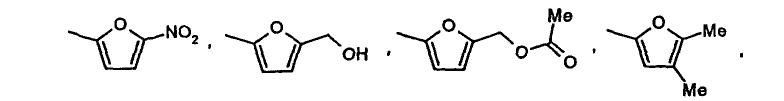 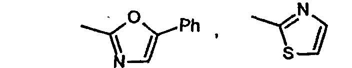
R2 is either:
- H,
- OH,
- a halogen,
- a C1-C5 alkyl radical,
- an oxygen atom substituted by a C1-C5 alkyl radical, or
- a NR5R5' radical, where R5 and R5' are, independently of each other, a hydrogen atom or an oxygen atom,
for the preparation of a drug intended for the treatment of diseases connected with an alteration of venous function and/or inflammatory edema.

2. 4,9-Dihydro-4,9-dioxo-1,2-dimethyl-1H-naphtho[2,3-d]imidazole sulfate.

3. 4,9-Dihydro-4,9-dioxo-2-(2-fluorophenyl)-1H-naphtho[2,3-d]imidazole.

4. 4,9-Dihydro-4,9-dioxo-2-(2-fluorophenyl)-naphtho[2,3-d]oxazole.

5. 4,9-Dihydro-4,9-dioxo-2-(3-fluorophenyl)-naphtho[2,3-d]oxazole.

6. 4,9-Dihydro-4,9-dioxo-2-(4-fluorophenyl)-naphtho[2,3-d]oxazole.

7. 4,9-Dihydro-4,9-dioxo-2-(2-methylphenyl)-naphtho[2,3-d]oxazole.

8. 4,9-Dihydro-4,9-dioxo-2-(3-methylphenyl)-naphtho[2,3-d]oxazole.

9. 4,9-Dihydro-4,9-dioxo-2-(4-methoxyphenyl)-naphtho[2,3-d]oxazole.

10. 2-(2-Chlorophenyl)-4,9-dihydro-4,9-dioxo-naphtho[2,3-d]oxazole.

11. 2-(4-Chlorophenyl)-4,9-dihydro-4,9-dioxo-naphtho[2,3-d]oxazole.

12. 4,9-Dihydro-4,9-dioxo-2-(2-thienyl)-naphtho[2,3-d]oxazole.

13. 4,9-Dihydro-4,9-dioxo-2-(2-fluorophenyl)-naphtho[2,3-d]thiazole.

14. 4,9-Dihydro-4,9-dioxo-2-(3-fluorophenyl)-naphtho[2,3-d]thiazole.

15. 4,9-Dihydro-4,9-dioxo-2-(4-fluorophenyl)naphtho[2,3-d]thiazole.

16. 2-(2,4-Difluorophenyl)-4,9-dihydro-4,9-dioxo-naphtho[2,3-d]thiazole.

17. 4,9-Dihydro-4,9-dioxo-2-(3-pyridyl)-naphtho[2,3-d]thiazole.

18. 4,9-Dihydro-4,9-dioxo-2-(4-pyridyl)-naphtho[2,3-d]thiazole sulfate.

19. 4,9-Dihydro-4,9-dioxo-2-(3-furyl)naphtho[2,3-d]thiazole.

20. 2-(5-Chlorofuran-2-yl)-4,9-dihydro-4,9-dioxo-naphtho[2,3-d]thiazole.

21. 4,9-Dihydro-4,9-dioxo-2-(2-thienyl)-naphtho[2,3-d]thiazole.

22. 4,9-Dihydro-4,9-dioxo-2-(3-thienyl)-naphtha[2,3-d]thiazole.

23. 4,9-Dihydro-4,9-dioxo-2-phenylamino-naphtha[2,3-d]thiazole.

24. 4,9-Dihydro-4,9-dioxo-8-methoxy-2-phenylnaphtho[2,3-d]thiazole.

25. 4,9-Dihydro-4,9-dioxo-5-methoxy-2-phenylnaphtho[2,3-d]thiazole.

26. 4,9-Dihydro-4,9-dioxo-7-methoxy-2-phenylnaphtho[2,3-d]thiazole.

27. 4,9-Dihydro-4,9-dioxo-6-methoxy-2-phenylnaphtho[2,3-d]thiazole.

28. 4,9-Dihydro-4,9-dioxo-8-hydroxy-2-phenylnaphtho[2,3-d]thiazole.

29. 4,9-Dihydro-4,9-dioxo-2-(1-pyrrolyl)-naphtho[2,3-d]thiazole.

30. 2-(5-Bromofuran-2-yl)-4,9-dihydro-4,9-dioxo-naphtho[2,3-d]thiazole.

31. 2-(4,5-Dibromofuran-2-yl)-4,9-dihydro-4,9-dioxo-naphtho[2,3-d]thiazole.

32. 2-(3-Bromofuran-2-yl)-4,9-dihydro-4,9-dioxo-naphtho[2,3-d]thiazole.

33. 2-(4-Bromofuran-2-yl)-4,9-dihydro-4,9-dioxo-naphtho[2,3-d]thiazole.

34. 4,9-Dihydro-4,9-dioxo-2-(5-nitro-furan-2-yl)-naphtho[2,3-d]thiazole.

35. 2-(5-Aminofuran-2-yl)-4,9-dihydro-4,9-dioxo-naphtho[2,3-d]thiazole.

36. 2-(5-Acetamidofuran-2-yl)-4,9-dihydro-4,9-dioxonaphtho[2,3-d]thiazole.

37. 4,9-Dihydro-4,9-dioxo-2-(5-hydroxymethylfuran-2-yl)naphtho[2,3-d]thiazole.

38. 2-(5-Acetoxymethylfuran-2-yl)-4,9-dihydro-4,9-dioxo-naphtho[2,3-d]thiazole.

39. 4,9-Dihydro-4,9-dioxo-2-(5-methyl-2-furyl)-naphtho[2,3-d]thiazole.

40. 4,9-Dihydro-2-(4,5-dimethyl-2-furyl)4,9-dioxo-naphtho[2,3-d]thiazole.

41. 4,9-Dihydro-4,9-dioxo-2-(5-phenyl-2-oxazlyl)-naphtho[2,3-d]thiazole.

42. 4,9-Dihydro-4,9-dioxo-2-(2-thiazolyl)naphtha[2,3-d]thiazole.

43. 4,9-Dihydro-4,9-dioxo-6-fluoro-2-(2-furyl)-naphtho[2,3-d]thiazole.

44. 4,9-Dihydro-4,9-dioxo-7-fluoro-2-(2-furyl)-naphtho[2,3-d]thiazole.

45. 4,9-Dihydro-4,9-dioxo-6-fluoro-2-phenylnaphtho[2,3-d]thiazole.

46. 4,9-Dihydro-4,9-dioxo-7-fluoro-2-phenylnaphtho[2,3-d]thiazole.

47. 4,9-Dihydro-4,9-dioxo-6-fluoro-2-(5-methyl-2-furyl)naphtho[2,3-d]thiazole.

48. 4,9-Dihydro-4,9-dioxo-7-fluoro-2-(5-methyl-2-furyl)naphtho[2,3-d]thiazole.

49. 4,9-Dihydro-4,9-dioxo-6-fluoro-2-(4-fluorophenyl)naphtho[2,3-d]thiazole.

50. 4,9-Dihydro-4,9-dioxo-7-fluoro-2-(4-fluorophenyl)naphtho[2,3-d]thiazole.

51. 4,9-Dihydro-4,9-dioxo-6-fluoro-2-(4-methylphenyl)naphtho[2,3-d]thiazole.

52. 4,9-Dihydro-4,9-dioxo-7-fluoro-2-(4-methylphenyl)naphtho[2,3-d]thiazole.

53. 4,9-Dihydro-4,9-dioxo-5-fluoro-2-(2-furyl)-naphtho[2,3-d]thiazole.

54. 4,9-Dihydro-4,9-dioxo-8-fluoro-2-(2-furyl)-naphtho[2,3-d]thiazole.

55. 6-Chloro-4,9-dihydro-4,9-dioxo-2-(2-furyl)naphtho[2,3-d]thiazole.

56. 7-Chloro-4,9-dihydro-4,9-dioxo-2-(2-furyl)naphtho[2,3-d]thiazole.

57. 4,9-Dihydro-4,9-dioxo-2-(2-furyl)-5-methoxynaphtho[2,3-d]thiazole.

58. 4,9-Dihydro-4,9-dioxo-2-(2-furyl)-8-methoxynaphtho[2,3-d]thiazole.

59. 4,9-Dihydro-4,9-dioxo-2-(2-furyl)-5-hydroxynaphtho[2,3-d]thiazole.

60. 4,9-Dihydro-4,9-dioxo-2-(2-furyl)-8-hydroxynaphtho[2,3-d]thiazole.

61. 4,9-Dihydro-4,9-dioxo-2-(2-furyl)-6-methoxynaphtho[2,3-d]thiazole.

62. 4,9-Dihydro-4,9-dioxo-2-(2-furyl)-7-methoxynaphtho[2, 3-d]thiazole.

63. 4,9-Dihydro-4,9-dioxo-2-furyl-6-methylnaphtho[2,3-d]thiazole.

64. 4,9-Dihydro-4,9-dioxo-2-furyl-7-methylnaphtho[2,3-d]thiazole.

65. 4,9-Dihydro-4,9-dioxo-6-methyl-2-phenylnaphtho[2,3-d]thiazole.

66. 4,9-Dihydro-4,9-dioxo-7-methyl-2-phenylnaphtho[2,3-d]thiazole.

67. 4,9-Dihydro-4,9-dioxo-2-furyl-5-methylnaphtho[2,3-d]thiazole.

68. 4,9-Dihydro-4,9-dioxo-2-furyl-8-methylnaphtho[2,3-d]thiazole.

69. 4,9-Dihydro-4,9-dioxo-5-methyl-2-phenylnaphtho[2,3-d]thiazole.

70. 4,9-Dihydro-4,9-dioxo-8-methyl-2-phenylnaphtho[2,3-d]thiazole.

71. Use according to claim 1, for the preparation of a drug intended for the treatment of functional and organic venous insufficiency.

72. Use according to claim 1, for the preparation of a drug intended for the treatment of hemorrhoid pathologies.

73. Use according to claim 1, for the preparation of a drug intended for the treatment of migraine.

74. Use according to claim 1, for the preparation of a drug intended for the treatment of dermatological and cardiovascular osteoarticular inflammations.

75. Use according to claim 1, for the preparation of a drug intended for the treatment of states of shock consisting of a large drop in arterial pressure, more particularly in states of septic shock.
